# EUROPEAN PATENT APPLICATION

(11) **EP 4 821 428 A2**
(43) Date of publication of application: **14.10.2026**
(21) Application number: 26201720.5
(22) Date of filing: 22.12.2023
(51) Int. Cl.: A61B 5/00

(54) **SYSTEM FOR ANALYTE MONITORING**

(30) Priority: 23.12.2022 US 202263435178 P
(62) Document number(s) of the earlier application(s) in accordance with Art. 76 EPC: 23853648.6 / 4 637 526
(71) Applicant: Abbott Diabetes Care, Inc., Alameda, CA 94502 (US)
(72) Inventor: KUMAR, Panganamala Ashwin, Oakland, California, 94618 (US); NAGRA, Saranpreet S., Alameda, California, 94501 (US); HEBBALE, Pooja, San Jose, California, 95134 (US)
(74) Representative: J A Kemp LLP

(57) **Abstract**

Digital and graphical user interfaces for multi-analyte monitoring systems are provided. For example, disclosed herein are various embodiments of methods, systems, and interfaces for alarm interfaces, alarm settings interfaces, alarm unavailability interfaces and features, and sensor results interfaces. Various embodiments of interfaces relating to glucose and ketone sensing are disclosed herein.

## Description

### CROSS-REFERENCE TO RELATED APPLICATIONS

This application claims priority to U.S. Application Serial No. 63/435,178, filed on December 23, 2022, which is hereby expressly incorporated by reference in its entirety for all purposes.

### FIELD

The subject matter described herein relates generally to systems and methods for monitoring a plurality of analytes in a user, as well as graphical user interfaces and devices related thereto.

### BACKGROUND

The detection and/or monitoring of analyte levels, such as glucose, ketones, lactate, oxygen, hemoglobin A1C, or the like, can be vitally important to the health of an individual having diabetes. Patients suffering from diabetes mellitus can experience complications including loss of consciousness, cardiovascular disease, retinopathy, neuropathy, nephropathy, and diabetic ketoacidosis (DKA).

Diabetics are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

As mentioned above, DKA is a complication that can result from diabetes. In particular, DKA results from insulin deficiency, in combination with increased levels of stress hormones that stimulate lipolysis, resulting in the production of ketones. Excessive production of ketones lowers the pH of the blood, and can cause severe dehydration and blood hyperosmolarity. Notably, DKA may be preventable in patients with diabetes if the presence of ketones is detected early and treatment is initiated. If left untreated, however, DKA can present life-threatening risks and result in death.

Ketones can be measured through urine dipstick tests. However, urine dipstick tests are commonly associated with false positive results. Further, many individuals diagnosed with a diabetic condition do not monitor their ketones levels as frequently as they should due to the inconvenience and unreliability associated with utilizing urine dipstick tests. Thus, a need exists for reliable ketone monitoring, wherein the user can monitor their ketone levels in a timely and routine manner, so as to prevent the need for hospital intervention.

To increase patient adherence to a plan of frequent glucose and ketone monitoring, in vivo analyte monitoring systems can be utilized, in which a sensor control device may be worn on the body of an individual who requires analyte monitoring. Further, to increase comfort and convenience for the individual, the sensor control device may have a small form-factor and can be applied by the individual with a sensor applicator. The application process includes inserting at least a portion of a sensor that senses one or more user analyte levels in a bodily fluid located in a layer of the human body, using an applicator or insertion mechanism, such that the sensor comes into contact with the bodily fluid. The analyte monitoring system may also be configured to transmit analyte data and/or alarms to another device, from which a caregiver such as, for example, a parent, a spouse, or a health care provider ("HCP"), can review the data and make therapy decisions.

Despite their advantages, however, some people are reluctant to use analyte monitoring systems for various reasons, including the complexity and volume of data presented, a learning curve associated with the software and user interfaces for analyte monitoring systems, and an overall paucity of actionable information presented.

Thus, needs exist for improved graphical user interfaces and alarms for analyte monitoring systems, as well as methods and devices relating thereto, that are robust, user-friendly, and provide for timely and actionable responses.

### SUMMARY

Provided herein are example embodiments of digital and graphical user interfaces ("GUIs") for analyte monitoring systems. In particular, described herein are systems, methods, and interfaces relating to an analyte monitoring application on a user's display device for the monitoring of analyte-related information pertaining to a plurality of analytes. Digital and graphical user interfaces for multi-analyte monitoring systems are provided. For example, disclosed herein are various embodiments of methods, systems, and interfaces for alarm interfaces, alarm settings interfaces, alarm unavailability interfaces and features, and sensor results interfaces. Various embodiments of interfaces relating to glucose and ketone sensing are disclosed herein.

According to some embodiments, provided herein is a system for monitoring a plurality of analytes in a user, the system comprising: a sensor control device comprising a sensor, wherein at least a portion of the sensor is configured to be in fluid contact with a bodily fluid of the user, and wherein the sensor control device is configured to transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte; a reader device comprising: wireless communication circuitry configured to receive the data indicative of the plurality of analyte levels of the user, one or more processors coupled with a memory, the memory storing an analyte monitoring application that, when executed by the one or more processors, causes the one or more processors to: output, based on the data indicative of the first analyte level and the data indicative of the second analyte level, a sensor results graphical user interface (GUI) comprising a first analyte section and a second analyte section, wherein the first analyte section comprises a first analyte card and a first analyte graph portion reflecting the data indicative of the first analyte level, and wherein the second analyte section comprises a second analyte card and a second analyte graph portion reflecting the data indicative of the second analyte level.

According to some embodiments, various sensor results interfaces for use with an analyte monitoring application are described. According to one example embodiment, a sensor results interface can include a first analyte section comprising data indicative of a first analyte level and a second analyte section comprising data indicative of a second analyte level. In some embodiments, the first analyte section can comprise a first analyte card and a first analyte graph portion reflecting the data indicative of the first analyte level. Further, in some embodiments, the second analyte section can comprise a second analyte card and a second analyte graph portion reflecting the data indicative of the second analyte level. In some embodiments, the data indicative of the first analyte level is data indicative of a glucose level. Further, in some embodiments, the data indicative of the second analyte level is data indicative of a ketone level. In some embodiments, the first analyte section can transition between a first collapsed view and a first expanded view. More specifically, according to some embodiments, in the first collapsed view, only the first analyte card of the first analyte section is displayed. As such, the first analyte graph portion is not displayed on the sensor results interface in the first collapsed view. More specifically, according to some aspects of the embodiments, in the first expanded view, both the first analyte card and the first analyte graph portion are displayed on the sensor results interface. In some embodiments, the section analyte section can transition between a second collapsed view and a second expanded view. In one aspect of the embodiments, in the second collapsed view, only the second analyte card of the second analyte section is displayed. In this regard, the second analyte graph portion is not displayed on the sensor results interface in the second collapsed view. In some aspects of the embodiments, in the second expanded view, both the second analyte card and the second analyte graph portion are displayed on the sensor results interface.

According to some embodiments, provided herein is a system for monitoring a plurality of analytes in a user, the system comprising: a sensor control device comprising an analyte sensor, wherein at least a portion of the analyte sensor is configured to be in fluid contact with a bodily fluid of the user, and wherein the sensor control device is configured to transmit data indicative of a plurality of analyte levels of the user, wherein the data indicative of the plurality of analyte levels comprises a data indicative of a first analyte level and a data indicative of a second analyte level, wherein the first analyte level is indicative of a first analyte, and wherein the second analyte level is indicative of a second analyte different from the first analyte; a reader device comprising: wireless communication circuitry configured to receive the data indicative of the plurality of analyte levels of the user, and one or more processors coupled with a memory, the memory storing an analyte monitoring application that, when executed by the one or more processors, causes the one or more processors to: determine whether the data indicative of the first analyte level or the data indicative of the second analyte level meets one or more alarm conditions, wherein the one or more alarm conditions comprise a first alarm condition associated with a first set of alarm settings that are configurable by the user and a second alarm condition associated with a second set of alarm settings that are not configurable by the user, in response to a determination that at least one of the one or more alarm conditions is met, display an alarm notification user interface (GUI) comprising an alarm associated with the at least one of the one or more alarm conditions.

In some embodiments, systems, methods, and interfaces for urgent low glucose alarms and high ketone alarms in an analyte monitoring system are provided, wherein the analyte monitoring system comprises a sensor control device configured to transmit data indicative of a plurality of analyte levels in a user. The analyte monitoring system further comprises a reader device (e.g., smart phone) having wireless communication circuitry, and one or more processors coupled with a memory storing instructions that, when executed by the one or more processors, cause the processors to determine whether the data indicative of the plurality of analyte levels meets one or more alarm conditions. The one or more alarm conditions comprise a first alarm condition associated with a first set of alarm settings that are configurable by a user, and a second alarm condition associated with a second set of alarm settings that are not configurable by the user, wherein the second alarm condition is an urgent low glucose alarm condition and/or a high ketone alarm condition. In some embodiments, the second set of alarm settings can include, for example, a non-configurable on-off setting, a non-configurable urgent low glucose threshold setting or non-configurable high ketone threshold setting, a non-configurable alarm tone setting, and/or a non-configurable setting to override a "Do Not Disturb" feature.

According to another embodiment, various alarm settings interfaces for an analyte monitoring application are provided. According to one aspect of the embodiments, the analyte monitoring application can be configured to display one or more alarm settings interfaces, wherein the one or more alarm settings interfaces comprise a plurality of selectable glucose alarms options, a plurality of selectable ketones alarms options, and one or more selectable other options, such as a signal loss alarm option. In some embodiments, for example, the plurality of selectable glucose alarm options comprises an urgent low glucose alarm option, a low glucose alarm option, and a high glucose alarm option. In some exemplary embodiments, the plurality of selectable ketones alarm options comprises an elevated ketone alarm option and a high ketone alarm option. Further, in some embodiments, one or more of the alarm settings interfaces can be configurable by the user. In some exemplary embodiments, alarm settings interfaces relating to low glucose alarm conditions, high glucose alarm conditions, elevated ketone alarm conditions, and signal loss alarm conditions can comprise configurable settings. In some embodiments, one or more of the alarm settings interfaces can be non-configurable by the user. For example, alarm settings interfaces relating to urgent low glucose alarm conditions and high ketone alarm conditions can comprise non-configurable settings or features.

According to some embodiments, systems and methods for detecting alarm unavailability conditions are also described. In particular, a reader device (e.g., a smart phone) comprises one or more processors coupled with a memory, the memory storing instructions that, when executed by the one or more processors, cause the one or more processors to detect one or more alarm unavailability conditions while at least one alarm of the analyte monitoring system is enabled, and present a notification associated with the detected one or more alarm unavailability conditions. In some embodiments, the one or more alarm unavailability conditions can include one or more of: a wireless communication circuitry being disabled or malfunctioning, one or more systemwide notifications being disabled, one or more application-specific notifications being disabled, one or more critical alerts being disabled, an override "Do Not Disturb" feature being disabled, one or more alarm tones being set to silent, no active sensor detected, or a sensor fault condition.

According to some embodiments, provided herein is an analyte monitoring system, comprising: a sensor control device comprising an analyte sensor, wherein the sensor control device is configured to be worn on a user's body and transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte; and a reader device comprising: a display; wireless communication circuitry configured to receive the data indicative of the one or more analyte levels of the user; one or more processors coupled with a memory, the memory storing an analyte monitoring application that, when executed by the one or more processors, causes the one or more processors to output to the display a graphical user interface (GUI) comprising: a graph portion comprising a first graph comprising a first trend line representing data indicative of the first analyte level over a predetermined time period and a second graph comprising a second trend line representing data indicative of the second analyte level over the predetermined time period; a first plurality of summary metrics associated with the data indicative of the first analyte level for the predetermined time period, wherein the first plurality of summary metrics comprises a plurality of first minimum analyte levels and first maximum analyte levels associated with a plurality of time increments within the predetermined time period, and a second plurality of summary metrics associated with the data indicative of the second analyte level for the predetermined time period, wherein the second plurality of summary metrics comprises one or more alarm metrics indicative of an alarm condition associated with the data indicative of the second analyte level, and wherein the graph portion further comprises an x-axis comprising units of time, and wherein the one or more alarm metrics, the first plurality of first minimum analyte levels and first maximum analyte levels are aligned with the x-axis of the graph portion.

According to some embodiments, provided herein is an analyte monitoring system, comprising: a sensor control device comprising an analyte sensor, wherein the sensor control device is configured to be worn on a user's body and transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte; and a reader device comprising: a display; wireless communication circuitry configured to receive the data indicative of the plurality of analyte levels of the user; one or more processors coupled with a memory, the memory storing an analyte monitoring application that, when executed by the one or more processors, causes the one or more processors to output to the display a graphical user interface (GUI) comprising: a first view and a second view, wherein the first view comprises a first tab configured to output data indicative of the first analyte level for a particular period of time, and wherein the first view further comprises a second tab configured to output data indicative of the second analyte level for the particular period of time, wherein the first tab is further configured to output a first analyte graph summary section and a logbook section, wherein the logbook section comprises information on one or more activity events related to the data indicative of the first analyte level, and wherein the second tab is further configured to output a second analyte graph section and an alarms section, wherein the alarms section comprises a list of one or more alarm events related to the data indicative of the second analyte level.

Many of the embodiments provided herein are improved GUIs or GUI features for an analyte monitoring application, that are highly intuitive, user-friendly, and provide for rapid access to important physiological information of the monitored user. More specifically, these embodiments allow the user to easily navigate through and between different user interfaces that can quickly indicate to the user various physiological conditions of the monitored user, without requiring the user to go through the arduous task of examining large volumes of data. Furthermore, some of the GUIs and GUI features and interfaces provide for versatility in that they allow for the user to simultaneously monitor multiple analytes. Other improvements and advantages are provided as well. The various configurations of these devices are described in detail by way of the embodiments which are only examples.

Other systems, devices, methods, features and advantages of the subject matter described herein will be or will become apparent to one with skill in the art upon examination of the following figures and detailed description. It is intended that all such additional systems, devices, methods, features, and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be depicted schematically rather than literally or precisely.
FIG. 1 is a system overview of an analyte monitoring system comprising a sensor applicator, a sensor control device, a reader device, a network, a trusted computer system, and a local computer system.
FIG. 2A is a block diagram depicting an example embodiment of a reader device.
FIGS. 3A and 3B are block diagrams depicting example embodiments of sensor control devices.
FIG. 4A is a flow diagram depicting an example embodiment of a method for displaying a sensor results GUI.
FIGS. 4B-1 to 4B-7 are example embodiments of sensor results GUIs.
FIGS. 4C-1 to 4C-14 are example embodiments of sensor results GUIs.
FIGS. 4D-1 to 4D-3 are example embodiments of sensor results GUIs.
FIGS. 4E-1 to 4E-6 are example embodiments of sensor results GUIs.
FIGS. 4F-1 to 4F-4 are example embodiments of sensor results GUIs
FIGS. 4G-1 to 4G-5 are example embodiments of sensor results GUIs.
4H-1 to 4H-3 are example embodiments of sensor results GUIs.

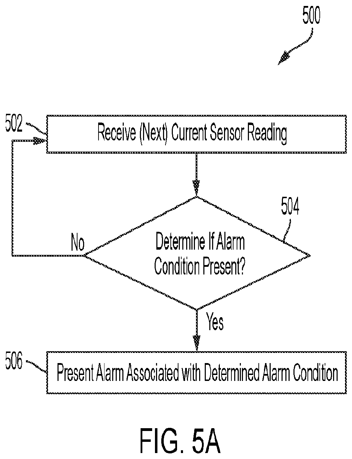

FIG. 5A is a flow diagram depicting an example embodiment of a method for determining and presenting alarms in an analyte monitoring system.

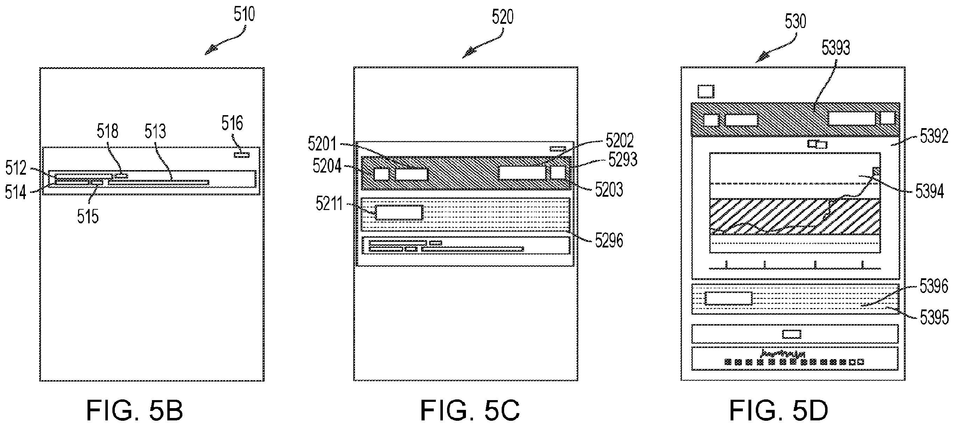

FIGS. 5B to 5L are example embodiments of GUIs relating to various alarms in an analyte monitoring system.

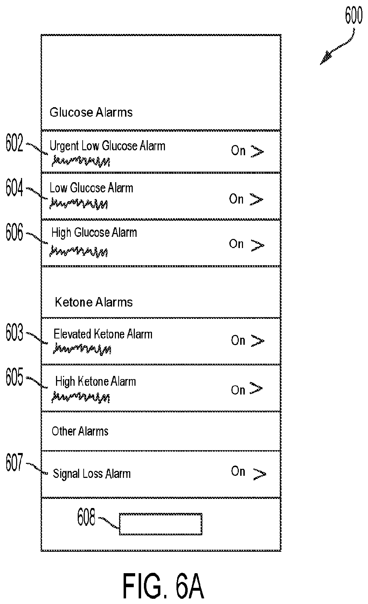

FIGS. 6A to 6R are example embodiments of GUIs relating to various alarm settings in an analyte monitoring system.

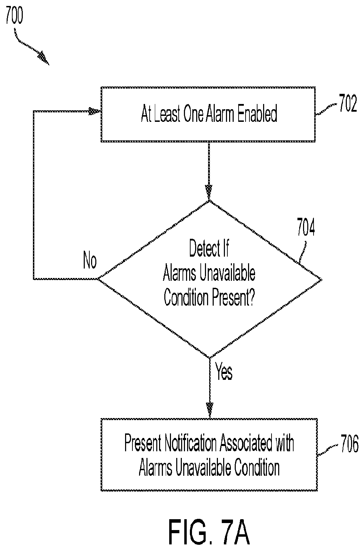

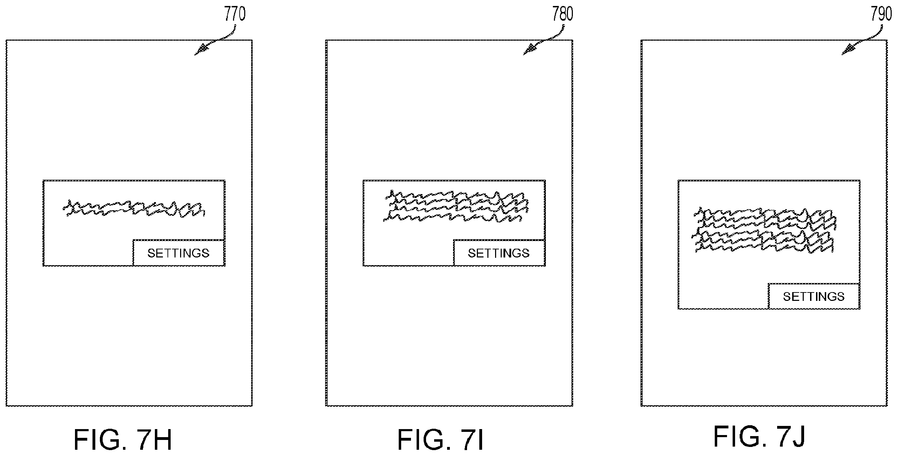

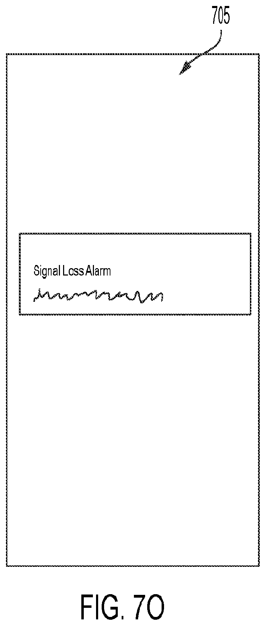

FIG. 7A is a flow diagram depicting an example embodiment of a method for the determination and notification of various alarm unavailable conditions.

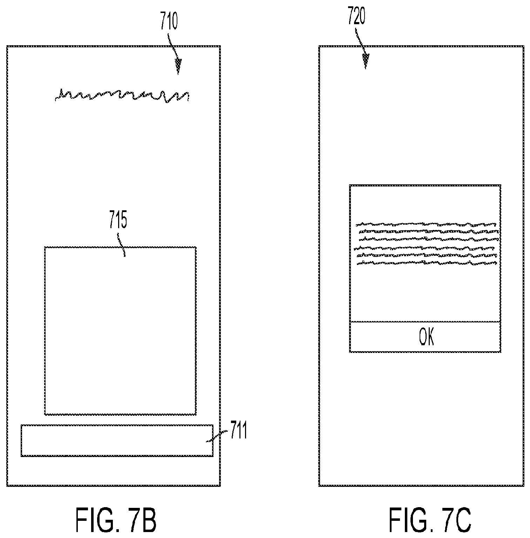

FIG. 7B is an example embodiment of a GUI relating to various alarm unavailability condition in an analyte monitoring system.
FIGS. 7C to 7L are example embodiments of modals relating to various alarm unavailability condition in an analyte monitoring system.

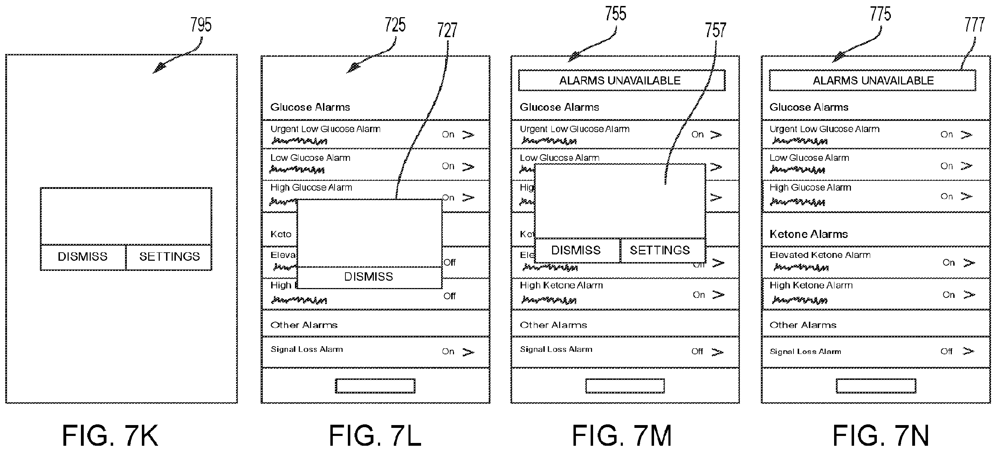

FIGS. 7M to 7O are example embodiments of GUIs relating to various alarm unavailability conditions in an analyte monitoring system.

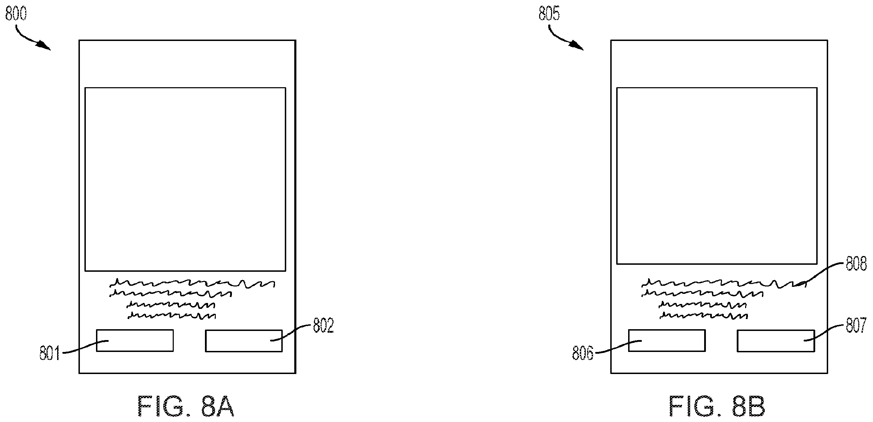

FIGS. 8A to 8I are block diagrams depicting example embodiments of onboarding GUIs and features relating thereto in an analyte monitoring system.

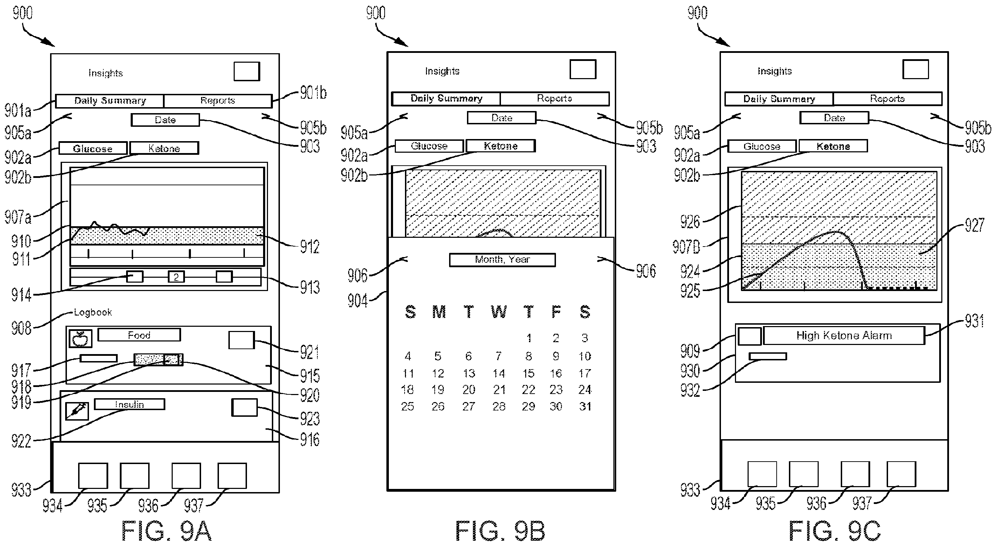

FIGS. 9A to 9F are block diagrams depicting example embodiments of insights GUIs and features relating thereto in an analyte monitoring system.

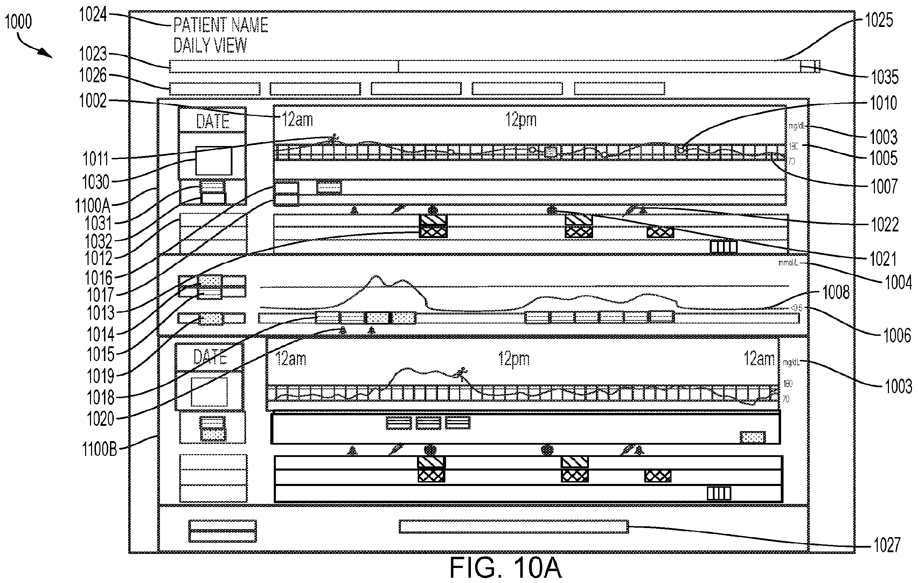

FIGS. 10A to 10D are block diagrams depicting example embodiments of report GUIs and features relating thereto in an analyte monitoring system.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include the graphical user interfaces (GUIs), alarms, and digital interfaces for analyte monitoring systems, and methods and devices relating thereto. Accordingly, many embodiments include in vivo analyte sensors structurally configured so that at least a portion of the sensor is, or can be, positioned in the body of a user to obtain information about at least one or more analytes of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices, reader devices, local computer systems, and trusted computer systems are disclosed, and these devices and systems can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps.

As previously described, a number of embodiments described herein provide for improved GUIs for analyte monitoring systems, wherein the GUIs are highly intuitive, user-friendly, and provide for rapid access to physiological information of a user. According to some embodiments, a sensor results GUI of an analyte monitoring system is provided, wherein the sensor results GUI comprises data indicative of one or more analyte levels. For example, the sensor results GUI can comprise data indicative of a first analyte level and data indicative of a second analyte level, wherein the sensor results GUI can display the data indicative of the first analyte level in a first analyte section and the data indicative of the second analyte level in the second analyte section so as to provide for a more user-friendly and intuitive user interaction with the analyte monitoring system, and provide for timely and actionable responses by the user, to name a few advantages.

According to another embodiment, an alarm GUI of an analyte monitoring system is provided, wherein the alarms and associated GUIs are actionable, user-friendly, and provide for rapid access to physiological information of a user. According to some embodiments, for example, methods and interfaces are provided for determining an alarm unavailability condition in an analyte monitoring system. According to other embodiments, methods and systems are provided for an alarm settings GUI of an analyte monitoring application. Additional improved digital and user interfaces for an analyte monitoring application are described. According to some embodiments, the alarm GUIs provided herein can be utilized to for one or more measured analytes.

Collectively and individually, these methods, systems, and digital and user interfaces improve upon the accuracy and integrity of analyte data being collected by an analyte monitoring system, and the alarming capabilities of the analyte monitoring system. Other improvements and advantages are provided as well. The various configurations of these devices are described in detail by way of the embodiments which are only examples.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

### Embodiments of In Vivo Analyte Monitoring Systems

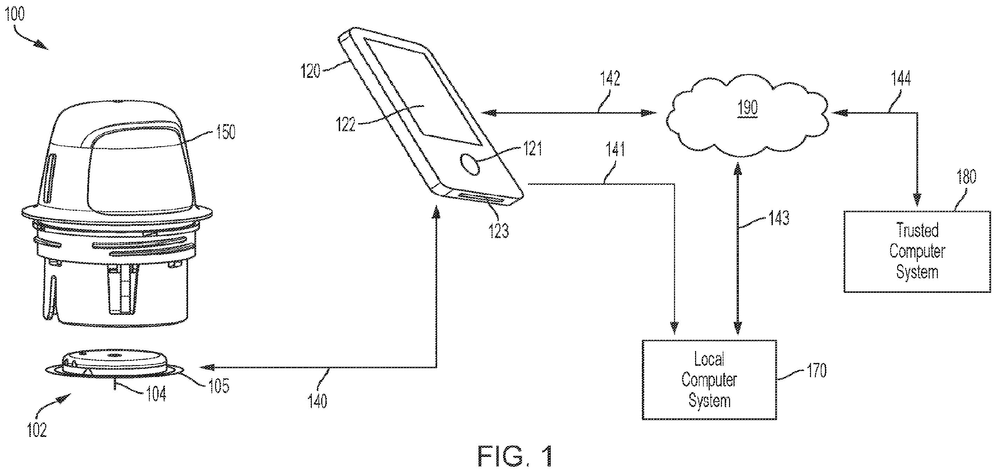

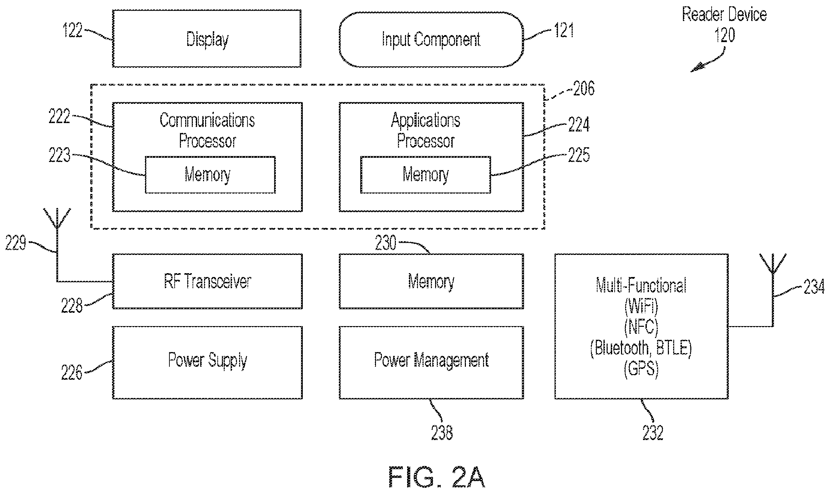

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can view and use applications installed in memory on reader device 120 using screen 122 (which, in many embodiments, can comprise a touchscreen), and input 121. A device battery of reader device 120 can be recharged using power port 123. While only one reader device 120 is shown, sensor control device 102 can communicate with multiple reader devices 120. Each of the reader devices 120 can communicate and share data with one another. More details about reader device 120 is set forth with respect to FIG. 2A below. Reader device 120 can communicate with local computer system 170 via a communication path 141 using a wired or wireless communication protocol. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by a wired or wireless communication protocol as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

### Example Embodiment of Reader Device

FIG. 2A is a block diagram depicting an example embodiment of a reader device 120, which, in some embodiments, can comprise a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further, reader device 120 can also include a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

### Example Embodiments of Sensor Control Devices

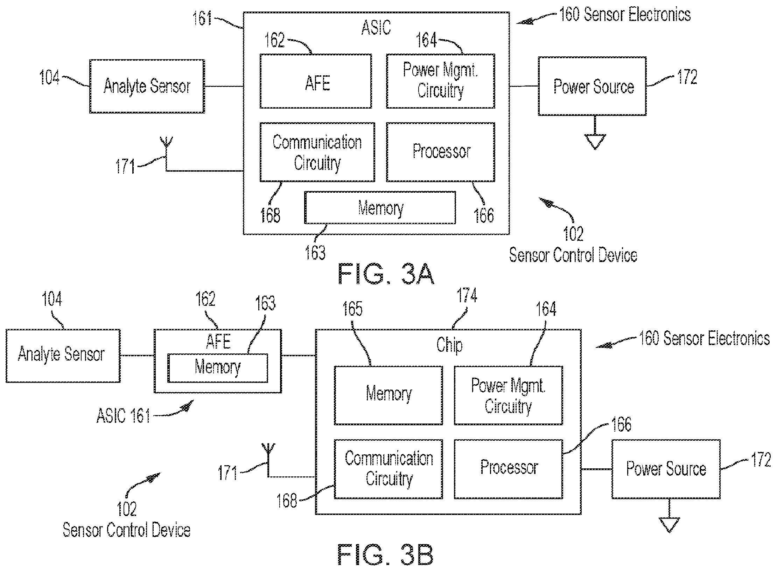

FIGS. 3A and 3B are block diagrams depicting example embodiments of sensor control devices 102 having analyte sensors 104 and sensor electronics 160 (including analyte monitoring circuitry) that can have the majority of the processing capability for rendering end-result data suitable for display to the user. In FIG. 3A, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 172, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 3B is similar to FIG. 3A but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Example Embodiments of Sensor Results GUIs

Example embodiments of sensor results interfaces for analyte monitoring systems, and other related features will now be described. As an initial matter, it will be understood by those of skill in the art that a "sensor results interface" or "sensor results GUI" can refer to an interface for presenting data indicative of one or more analyte levels of a user. These interfaces can be stored as instructions in the memory of a reader device 120 (e.g., a smart phone), local computer system 170 (e.g., desktop computer), trusted computer system 180 (e.g., cloud-based server), or any other computing device or system that can be implemented with analyte monitoring system 100. These stored instructions, when executed by the one or more processors of the corresponding computing device or system, can cause the one or more processors to display any of the interfaces described herein. By way of illustration, it will be understood by those of skill in the art that, in some embodiments, the stored instructions can comprise an analyte monitoring software program installed on a reader device 120 or local computer system 170. In other embodiments, the stored instructions can comprise software residing on a cloud-based server of trusted computer system 180, wherein the interfaces are subsequently rendered and/or displayed on a separate computing device or system (e.g., via a web browser on a user's smart phone or desktop computer). As such, it will be understood by those of skill in the art that the interfaces can be implemented on a single centralized device or, in the alternative, can be distributed across multiple discrete devices in geographically dispersed locations. Likewise, those of skill in the art will recognize that the presentations of various computer systems in the embodiments disclosed herein, as shown in FIG. 1, are intended to cover both physical computing devices and virtual computing devices (e.g., virtual servers or virtual machines). It will be further understood by those of skill in the art that any one or more of the example embodiments of the methods, interfaces, and systems described herein can either be implemented independently, or in combination with any of the other embodiments described in the present application.

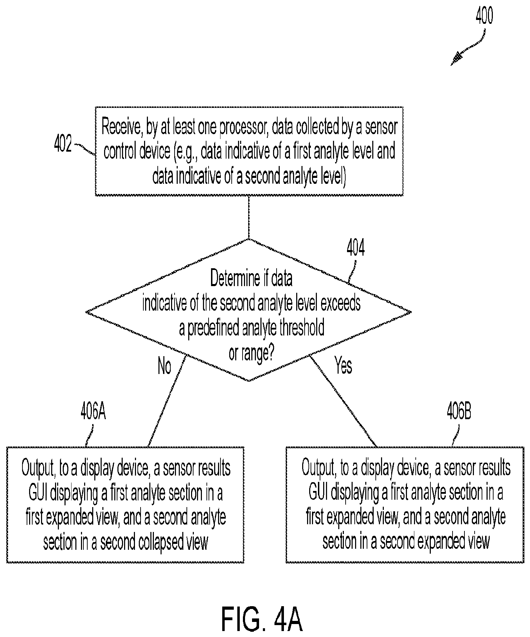

FIG. 4A shows operations of a method for performing the process for a sensor results GUI that displays data indicative of a first analyte level and data indicative of a second analyte level from a sensor control device 102. Specifically, the sensor results GUI can display a first analyte section, comprising the data indicative of the first analyte level, in a first collapsed view or a first expanded view. More specifically, the sensor results GUI can display a second analyte section, comprising the data indicative of the second analyte level, in a second collapsed view or a second expanded view. In some embodiments, in the first expanded view, the first analyte section (e.g., the glucose section) comprises a first analyte card (e.g., glucose card) and a first analyte graph portion (e.g., glucose graph portion). In some embodiments, in the first collapsed view, the first analyte section (e.g., the glucose section) comprises only the first analyte card (e.g., glucose card) and not the first analyte graph portion (e.g., glucose graph portion). In some embodiments, in the second expanded view, the second analyte section (e.g., the ketone section) comprises a second analyte card (e.g., ketone card) and a second analyte graph portion (e.g., ketone graph portion). In some embodiments, in the second collapsed view, the second analyte section (e.g., the ketone section) comprises only the second analyte card (e.g., ketone card) and not the second analyte graph portion (e.g., ketone graph portion).

At Step 402, the method for performing the process for a sensor results GUI that displays data indicative of a first analyte level and data indicative of a second analyte level from a sensor control device further includes: receiving, by at least one processor, data collected by a sensor control device 102. In some embodiments, the data can include data indicative of a ketone level and data indicative of a glucose level received by reader device 120. In some embodiments, the data can comprise data from a glucose-ketone sensor 104 disposed in the sensor control device 102, wherein at least a portion of the glucose-ketone sensor 104 is configured to be positioned under the subject's skin and in contact with a bodily fluid. In some embodiments, data is received from a single sensor control device 102 with a sensor 104 capable of sensing both glucose and ketone. In some embodiments, data is received from a single sensor control device 102 comprising two or more discrete sensors 104A, 104B, wherein a first sensor 104A is capable of sensing glucose and a second sensor 104B is capable of sensing ketone. In some embodiments, data is received from two or more sensor control devices 102, wherein a first sensor control device 102 comprises a sensor 104 capable of sensing ketone and a second sensor control device 102 comprises a sensor 104 capable of sensing glucose. Those of skill in the art will recognize various other sensor control device, sensor, and reader device configurations and combinations providing the aforementioned capabilities can be implemented, all of which are fully within the scope of the present disclosure.

At Step 404, the method 400 further includes determining whether the data indicative of the second analyte level (e.g., data indicative of a ketone level) exceeds a predefined analyte threshold or range. For example, in some embodiments, data indicative of a ketone level exceeds a predefined "normal" threshold or range (e.g., normal ketone range is below 0.6 mmol/L, anything above normal ketone range would be considered to exceed predefined normal threshold or range).

At Step 406A, if it is determined at Step 404 that the data indicative of the second analyte level does not exceed a predefined analyte threshold or range (e.g., ketone levels are below 0.6 mmol/L), method 400 further includes, outputting, to a display device, a sensor results GUI displaying the first analyte section in the first expanded view, and the second analyte section in the second collapsed view. For example, the sensor results GUI can display a glucose section in the first expanded view, wherein in the first expanded view, the glucose card and the glucose graph portion are outputted, and a ketone section in the second collapsed view, wherein in the second collapsed view, the ketone card is outputted and not the ketone graph portion. Alternatively, at Step 406B, if it is determined at Step 404 that the data indicative of the second analyte level exceeds a predefined analyte threshold or range, method 400 further includes, outputting, to a display device, a sensor results GUI displaying the first analyte section in the first expanded view, and the second analyte section in the second expanded view. For example, the sensor results GUI can display a glucose section in the first expanded view, wherein in the first expanded view, the glucose card and the glucose graph portion are outputted, and a ketone section in the second expanded view, wherein in the second expanded view, the ketone card and the ketone graph portion are outputted. It will be appreciated by those of skill in the art that the method steps described herein can be performed either by a single device or by multiple devices. For example, in some embodiments, the receiving of data can be performed on the sensor results control device 102 and the outputting of the sensor results GUIs can be performed by reader device 120. In other embodiments, both the receiving of data and the outputting of the sensor results GUI can be performed by the sensor control device 102 or, alternatively, the reader device 120.

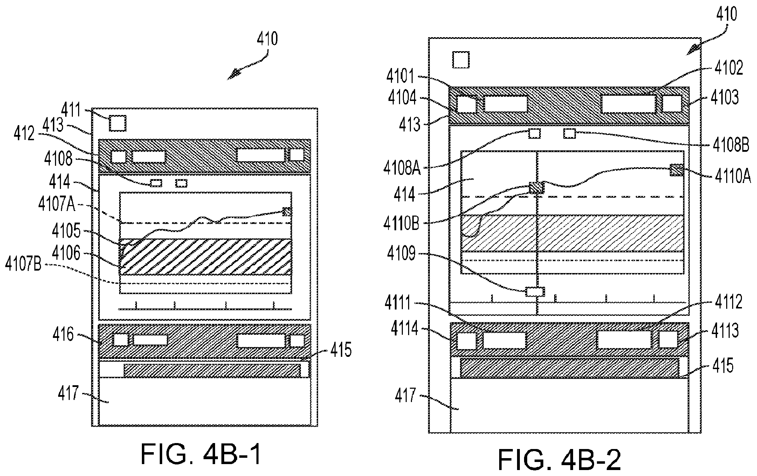

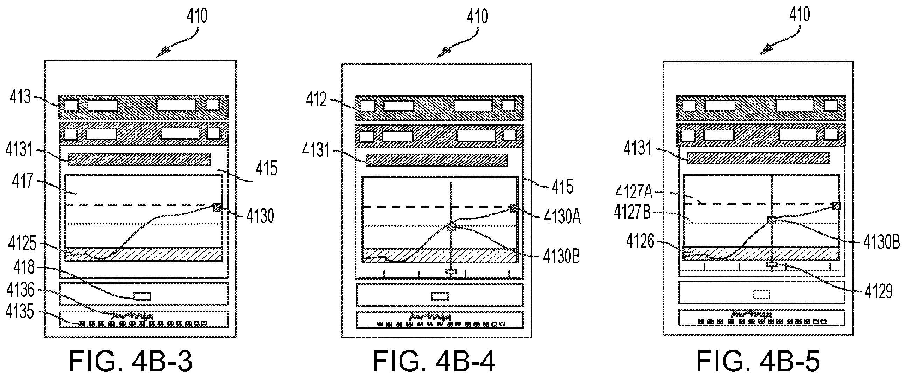

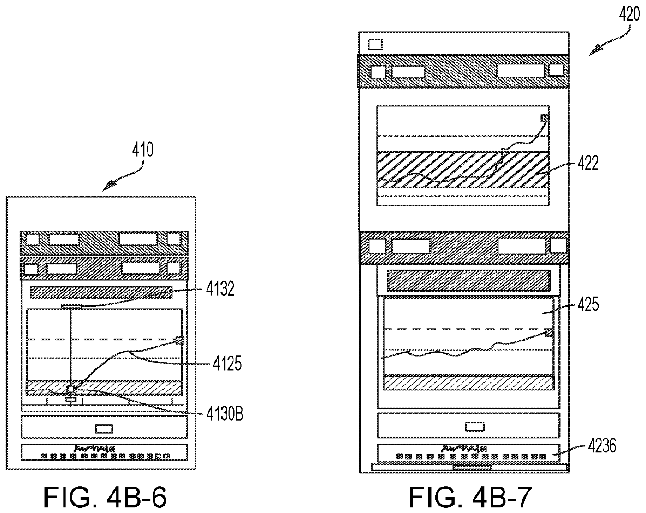

FIGS. 4B-1 to 4G-6 are example embodiments of sensor results GUIs for use with an analyte monitoring system. FIGS. 4B-1 to 4B-6 are example embodiments of a sensor results GUI 410, wherein the sensor results GUI 410 comprises: (1) a menu icon 411; (2) a first analyte section 412 comprising a first analyte card 413 and/or a first analyte graph portion 414 reflecting data indicative of a first analyte level (e.g., a glucose card and/or a glucose graph portion reflecting data indicative of a glucose level); (3) a second analyte section 415 comprising a second analyte card 416 and/or a second analyte graph portion 417 reflecting data indicative of a second analyte level (e.g., a ketone card and/or a ketone graph portion reflecting data indicative of a ketone level); (4) a selectable "Add Note" link 418 that, when selected by the user, outputs a note interface (not shown) wherein the user can input notes; and (5) a graphical indication 4135 comprising a progress indicator that visually illustrates the remaining lifetime of the sensor 104 (e.g., a glucose-ketone sensor) (not shown in the sensor results GUI figures described herein). In some exemplary embodiments, the first analyte section 412 is located proximal and directly adjacent to the second analyte section 415 on sensor results GUI 410. In some exemplary embodiments, the second analyte section 415 is located proximal and directly adjacent to the "Add Note" link 418 on sensor results GUI 410. Moreover, in some embodiments, the "Add Note" link 418 is located proximal and directly adjacent to the graphical indication 4135 comprising a progress indicator on sensor results GUI 410.

In one aspect of the embodiments, and as illustrated in FIGS. 4B-1 to 4B-6, the first analyte card 413 (e.g., glucose card) can include: a text description 4101 of a first analyte condition (e.g., "High Glucose"), a first current analyte level value 4102 (e.g., a current glucose level of 320 mg/dL), and a first trend indicator 4103 (e.g., directional trend arrow indicating the glucose level is rising). In other exemplary embodiments, the first trend indicator 4103 can include a directional trend arrow indicating the glucose level is falling or changing slowly. In some embodiments, the first current analyte level value 4102 can comprise a numeric value (which can be displayed in a unit of measure determined by the third party's region or country) (e.g., 320 mg/dL). Further, in some embodiments, and as best depicted in FIGS. 4B-1 to 4B-6, the first analyte card 413 can further include an alarm icon 4104 adjacent to the text description 4101 of the first analyte condition.

In another aspect of the embodiments, and as illustrated in FIGS. 4B-1 to 4B-6, the second analyte card 416 (e.g., ketone card) can include a text description 4111 of a second analyte condition (e.g., "High Ketones"), a second current analyte level value 4112 (e.g., a current ketone level of 2.1 mmol/L), and a second trend indicator 4113 (e.g., a directional trend arrow indicating the ketone level is rising). In other exemplary embodiments, the second trend indicator 4113 can include a directional trend arrow indicating the ketone level is falling quickly, falling, changing slowly, or rising quickly. In some embodiments, the second current analyte level value 4112 can comprise a numeric value (which can be displayed in a unit of measure determined by the third party's region or country) (e.g., 2.1 mmol/L). Further, and as depicted in FIGS. 4B-1 to 4B-6, the second analyte card 416 can further include an alarm icon 4114 adjacent to the text description 4111 of the second analyte condition.

In some embodiments, the first analyte card 413 includes a background color indicative of a condition or an analyte range associated with the data indicative of the first analyte level. Similarly, in some embodiments, the second analyte card 416 includes a background color indicative of a condition or an analyte range associated with the data indicative of the second analyte level.

In some embodiments, the colors corresponding to the data indicative of the first analyte level are determined by the first current analyte level value 4102 of the user and the target analyte ranges that are set. For example, if the user's first current analyte level value 4102 is below a target analyte range (e.g., glucose level below 70 mg/dL), the background color of the first analyte card 413 can be red. If the user's first current analyte level value 4102 is within a target range, the background color of the first analyte card 413 can be green. If the user's first current analyte level value 4102 is between a target range (e.g., glucose level between 70mg/dL and 250 mg/dL), the background color of the first analyte card 413 can be yellow. If the user's first current analyte level value 4102 is above a target range (e.g., glucose level above 250 mg/dL), the background color of the first analyte card 413 can be orange. According to some embodiments, the target range can be a predetermined analyte range configured by the user. In some exemplary embodiments, sensor results GUI 410 comprises a first analyte card 413 (e.g., a glucose card) having an orange color indicating a first current analyte level value 4102 above a target range (e.g., high glucose).

In some embodiments, the colors corresponding to the data indicative of the second analyte level are determined by the second current analyte level value 4112 of the user and the predefined ranges of the sensor 104. For example, if the user's second current analyte level value 4112 is within a predefined "normal" range (e.g., ketone level below 0.6 mmol/L), the background color of the second analyte card 416 can be green. If the user's second current analyte level value 4112 is within a predefined "elevated" analyte range (e.g., ketone level between 0.6 to 1.4 mmol/L), the background color of the second analyte card 416 can be yellow. If the user's second current analyte level value 4112 is within a predefined "high" analyte range (e.g., ketone level above 1.5 mmol/L), the background color of the second analyte card 416 can be red. According to some embodiments, the predefined ranges can be configured by the user. In some exemplary embodiments, sensor results GUI 410 comprises a second analyte card 416 (e.g., a ketone card) having a red color indicating a second current analyte level value 4112 is within a predefined high analyte range (e.g., high ketones). Those of skill in the art will recognize that in some embodiments illustrated herein, the high ketone range is depicted as 1.5 mmol/L or 2.0 mmol/L and that these examples are meant to be illustrative only, and that other predefined ranges associated with the data indicative of the first analyte level and data indicative of the second analyte level can be implemented and are fully within the scope of the present disclosure. Those of skill in the art will further recognize that various additional colors can be implemented to indicate a particular analyte range and can be implemented, and are fully within the scope of the present disclosure.

In another aspect of the embodiments, and with reference to FIGS. 4B-1 to 4B-2, the first analyte graph portion 414 is located adjacent to the first analyte card 412, and can include a first analyte trend line 4105 to reflect the user's analyte level, based on the data indicative of the first analyte level, over a predetermined amount of time. For example, the x-axis of the first analyte graph portion 414 can comprise units of time (e.g., in five minute increments, fifteen-minute increments, three hour increments, etc.) covering a twelve-hour period, whereas the y-axis can comprise a measured first analyte concentration of the user. Those of skill in the art will further recognize that other predetermined periods of time (e.g., three hours, four hours, twenty-four hours, forty-eight hours, etc.) can be reflected on the x-axis, and are fully within the scope of the present disclosure.

Further, the first analyte graph portion 414 can further include: a shaded area to indicate the user's target analyte range (e.g., target glucose threshold) associated with the data indicative of the first analyte level, as indicated by shaded area 4106, as well as one or more alarm thresholds, depicted in FIGS. 4B-1 to 4B-2as dashed lines 4107A (e.g., high glucose threshold) and 4107B (e.g., low glucose threshold). In some embodiments, the alarm thresholds can be configured by the user. In some embodiments, the shaded area 4106 and the dashed lines 4107A, 4107B can each comprise a color indicative of a condition associated with the range or threshold the shaded area 4106 and dashed lines 4107A, 4107B respectively represent. In still other embodiments, the first analyte graph portion 414 can have no alarm thresholds (e.g., if the user's sensor control device 102 does not support alarms).

In some embodiments, the first analyte graph portion 414 can further include one or more icons 4108 associated with the data indicative of the first analyte level, which correspond to one or more points on the first analyte trend line 4105. In some embodiments, the one or more icons 4108 are positioned on the first analyte trend line 4105 so as to lie along the corresponding one or more points along the x-axis. Moreover, the one or more icons 4108 can include: a food icon, a rapid-acting insulin icon, a long-acting insulin icon, and an exercise icon. In the exemplary embodiments depicted in FIGS. 4B-1 to 4B-2, a food icon 4108A, and rapid-acting insulin icon or a long-acting insulin icon 4108B are displayed. Further, the one or more icons 4108 can be selectable by the user. In this regard, and in response to a received input by the user (e.g., by selecting a corresponding area on the touchscreen), a note card section (not illustrated) is further displayed on sensor results GUI 410, wherein the note card section includes a text description associated with the selected one or more icons 4108. Those of skill in the art will recognize that these aforementioned icons are meant to be illustrating only, and do not represent an exhaustive list of all icons which can be displayed on the sensor results GUIs described herein.

According to another aspect of many embodiments, the sensor results GUI 410 can be real-time, or near real-time, and interactive. In some embodiments, for example, the information reflected in the first analyte card 413 (e.g., first current analyte level value 4102) and first analyte graph portion 414 (e.g., first analyte trend line 4105) can be automatically updated and/or refreshed at a predetermined frequency (e.g., every thirty seconds, every minute, every five minutes, etc.). In still other embodiments, the first analyte card 413 and first analyte graph portion 414 can be updated in response to a predetermined input by the user or by some other predetermined gesture.

In addition, according to another aspect of the embodiments, the first analyte section 412 can be configured to display a first historical analyte data based on the user's interaction with the first analyte graph portion 414. In some embodiments, for example, when the user interacts with a point on the first analyte trend line 4105 (e.g., by touching a point on the first analyte trend line 4105), the first analyte section 412 can be updated to display corresponding first historical analyte data (if any) and a corresponding time stamp 4109 associated with the point of the first analyte trend line 4105 being interacted with by the user. In some embodiments (not shown), and for example, upon a first use of the sensor 104, the first analyte graph portion 414 does not display any first historical analyte data and only displays the first current analyte data.

As shown FIGS. 4B-1 to 4B-2, a first current analyte data being displayed in the first analyte section 412 can be indicated by a colored circle 4110 on the first analyte trend line 4105, wherein the color of the colored circle 4110 indicates a condition associated with the first current analyte data. In some embodiments, the user can touch colored circle 4110 and drag it along first analyte trend line 4105, and the information displayed in the first analyte section 412 can be immediately updated with first historical analyte data based on the point or portion of the first analyte trend line 4105 being touched or selected by the user. In this embodiment, the color of the colored circle 4110 can update so as to correspond to a condition associated with the selected historical analyte data. Additionally, in some embodiments, and as illustrated in FIG. 4B-2, one or more colored circles 4110 can be displayed on the first analyte trend line 4105, wherein a first colored circle 4110A corresponds to the first current analyte data, and a second colored circle 4110B corresponds to the area on the first analyte trend line 4105 being touched or selected by the user. In this regard, the first colored circle 4110A comprises a color reflecting a condition associated with the first current analyte data (e.g., orange first colored circle indicating high glucose condition), and the second colored circle 4110B comprises a color reflecting a condition associated with the first historical analyte data corresponding to the selected area (e.g., orange second colored circle indicating high glucose condition). Though not illustrated, those of skill in the art will recognize that the first analyte trend line 4105 can include more than two colored circles 4110, as depicted here, and is fully within the scope of the present disclosure.

In yet another aspect of the embodiments, and as shown in FIGS. 4B-1 to 4B-6, the second analyte graph portion 417 is located adjacent to the second analyte card 416, and can include a second analyte trend line 4125 to reflect the user's analyte level, based on the data indicative of the second analyte level, over a predetermined amount of time. For example, the x-axis of the second analyte graph portion 417 can comprise units of time (e.g., in five minute increments, fifteen-minute increments, three hour increments, etc.) covering a twelve-hour period, whereas the y-axis can comprise a measured second analyte concentration of the user. Those of skill in the art will further recognize that other predetermined periods of time (e.g., three hours, four hours, twenty-four hours, forty-eight hours, etc.) can be reflected on the x-axis, and are fully within the scope of the present disclosure.

Additionally, the second analyte graph portion 417 can further include: a shaded area to indicate the user's predefined or predetermined normal analyte range (e.g., normal ketone threshold) associated with the data indicative of the second analyte level, as indicated by shaded area 4126, as well as one or more alarm thresholds, depicted in in FIGS. 4B-3 to 4B-6 as dashed lines 4127A (e.g., high ketone threshold) and 4127B (e.g., elevated ketone threshold). In some embodiments, the alarm thresholds can be configured by the user. In some embodiments, the shaded area 4126 and the dashed lines 4127A, 4127B can each comprise a color indicative of a condition associated with the respective range or threshold each represents. In still other embodiments, the second analyte graph portion 417 can have no alarm thresholds (e.g., if the user's sensor control device 102 does not support alarms).

In some embodiments, though not depicted in FIGS. 4B-1 to 4B-6, the second analyte graph portion 417 can further include one or more icons 4128 (not shown) associated with the data indicative of the second analyte level, which correspond to one or more points on the second analyte trend line 4125. For example, the one or more icons 4128 can include: a food icon, a rapid-acting insulin icon, a long-acting insulin icon, and an exercise icon. Further, the one or more icons 4128 can be selectable by the user. In this regard, and in response to a received input by the user (e.g., by selecting a corresponding area on the touchscreen), a note card section (not illustrated) is further displayed on sensor results GUI 410, wherein the note card section includes a text description associated with the selected one or more icons 4128. Those of skill in the art will recognize that these aforementioned icons are meant to be illustrating only, and do not represent an exhaustive list of all icons which can be displayed on the sensor results GUIs described herein.

In some embodiments, and as depicted in FIGS. 4B-1 to 4B-6, the second analyte graph portion 417 can further include a banner notification 4131 comprising instructions, wherein the instructions relate to a high ketone level condition or an elevated ketone level condition that is present. In these embodiments, the banner notification 4131 can include a background color indicative of the high ketone level condition (e.g., red background color) or the elevated ketone level condition (e.g., yellow background color) that is present. For example, in the embodiments depicted in FIGS. 4B-1 to 4B-6, the second analyte graph portion 417 comprises a banner notification 4131 ("Seek Medical Attention") comprising instructions relating to the high ketone level condition. Further, banner notification 413 further comprises a red background color to indicate the high ketone level condition.

Further, and according to another aspect of many embodiments, the sensor results GUI 410 can be real-time, or near real-time, and interactive. In some embodiments, for example, the information reflected in the second analyte card 416 (e.g., second current analyte level value 4112) and second analyte graph portion 417 (e.g., second analyte trend line 4125) can be automatically updated and/or refreshed at a predetermined frequency (e.g., every thirty seconds, every minute, every five minutes, etc.). In still other embodiments, the second analyte card 416 and second analyte graph portion 417 can be updated in response to a predetermined input by the user or by some other predetermined gesture.

Similar to first analyte section 412 of sensor results GUI 410, the second analyte section 415 can be configured to display a second historical analyte data based on the user's interaction with the second analyte graph portion 417. In some exemplary embodiments, when the user interacts with a point on the second analyte trend line 4125 (e.g., by touching a point on the second analyte trend line 4125), the second analyte section 415 can be updated to display corresponding historical analyte data (if any) and a corresponding time stamp 4129 associated with the point of the second analyte trend line 4125 being interacted with by the user. In some embodiments (not shown), and for example, upon a first use of the sensor 104, the second analyte graph portion 417 does not display any second historical analyte data and only displays the second current analyte data. In some embodiments, and as illustrated in FIG. 4B-6, instead of a second historical analyte data (numerical value), the second analyte trend line 4125 displays a condition descriptor 4132 ("Normal") to indicate the condition of the second historical analyte data being displayed.

As shown FIGS. 4B-3 to 4B-6, a second current analyte data being displayed in the second analyte section 415 can be indicated by a colored circle 4130 on the second analyte trend line 4125, wherein the color of the colored circle 4130 indicates a condition associated with the second current analyte data. In some embodiments, the user can touch colored circle 4130 and drag it along second analyte trend line 4125, and the information displayed in the second analyte section 415 can be immediately updated with second historical analyte data based on the point or portion of the second analyte trend line 4125 being touched or selected by the user. In this embodiment, the color of the colored circle 4130 can update so as to correspond to a condition associated with the selected historical analyte data. Additionally, in some embodiments, and as illustrated in FIGS. 4B-4 to 4B-6, one or more colored circles 4130 can be displayed on the second analyte trend line 4125, wherein a first colored circle 4130A corresponds to the second current analyte data (e.g, red first colored circle 4130A indicating a high ketone condition in FIGS. 4B-4 to 4B-6), and a second colored circle 4130B corresponds to the area on the second analyte trend line being touched or selected by the user (e.g., orange second colored circle 4130B indicating an elevated ketone condition in FIG. 4B-4, a yellow second colored circle 4130B indicating an elevated ketone condition in FIG. 4B-5, and a green second colored circle 4130B indicating a normal ketone condition in FIG. 4B-6). In this regard, the first colored circle 4130A comprises a color reflecting a condition associated with the second current analyte data, and the second colored circle 4130B comprises a color reflecting a condition associated with the second historical analyte data corresponding to the selected area. Though not illustrated, those of skill in the art will recognize that the second analyte trend line 4125 can include more than two colored circles 4130, as depicted here, and is fully within the scope of the present disclosure.

According to another aspect of the embodiments, the first analyte section 412 of sensor results GUI 410 is configured to transition between a first collapsed view and a first expanded view. Specifically, in the first collapsed view (FIGS. 4B-3 to 4B-6), only the first analyte card 413 is displayed in the first analyte section 412. As such, in the first collapsed view, the first analyte graph portion 414 is not displayed in the first analyte section 412 of sensor results GUI 410. In the first expanded view (FIGS. 4B-1 and 4B-2), however, both the first analyte card 413 and the first analyte graph portion 414 are displayed on sensor results GUI 410.

In like manner, the second analyte section of sensor results GUI 410 is configured to transition between a second collapsed view and a second expanded view. Specifically, in the second collapsed view (not illustrated), only the second analyte card 416 is displayed in the second analyte section 415. In this regard, the second analyte graph portion 417 is not displayed when the second analyte section 415 of sensor results GUI 410 is in the second collapsed view. However, in the second expanded view FIGS. 4B-1 to 4B-6), both the second analyte card 416 and the second analyte graph portion 417 are displayed on sensor results GUI 410.

According to one aspect of the embodiments, the first analyte card 413 and the second analyte card 416 are displayed on the sensor results GUI 410 at all times ( FIGS. 4B-1 to 4B-6). In some embodiments, and as shown in FIGS. 4B-1 to 4B-6, the first expanded view and the second expanded view are displayed on the sensor results GUI 410 at the same time. In some embodiments, though not illustrated, the first expanded view and the second collapsed view are displayed on the sensor results GUI 410 at the same time. In some embodiments, as illustrated in FIGS. 4B-3 to 4B-6, the first collapsed view and the second expanded view are displayed on the sensor results GUI 410 at the same time.

In some embodiments, and as illustrated in FIGS. 4B-1 to 4B-2, the sensor results GUI 410 is configured to display the first expanded view of the first analyte section 412 by default. Further, in some embodiments, the second analyte section 415 is only configured to display the second expanded view by default when the data indicative of the second analyte level exceeds a predefined normal analyte range, or reaches an elevated or high analyte range (FIGS. 4B-1 to 4B-3 illustrating default view of sensor results GUI 410). FIGS. 4B-1 and 4B-2 depict sensor results GUI 410 in its default view, wherein the first analyte section 412 is displayed in the first expanded view and the second analyte section 415 is displayed in second expanded view.

In some embodiments, the first analyte section 412 and the second analyte section 415 can be updated in response to a predetermined input by the user, such as when the user taps, drags, scrolls, pulls the screen down with a finger, pulls the screen up with a finger, or by some other predetermined gesture. For example, in exemplary embodiments, the sensor results GUI 410 can transition from displaying the first expanded view (e.g., FIGS. 4B-1 and 4B-2 ) to the first collapsed view (FIGS. 4B-3 to 4B-6) in response to a received input by the user (e.g., by a scroll gesture, a tap gesture, a pull up gesture, or by selecting or pressing a corresponding area of the touchscreen). In this regard, the first analyte section 412 can transition from displaying the first analyte card 413 and the first analyte graph portion 414, to displaying only the first analyte card 413 on the sensor results GUI 410. Moreover, in some embodiments, the sensor results GUI 410 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 412 can transition from displaying only the first analyte card 413, to displaying both the first analyte card 413 and the first analyte graph portion 414 on the sensor results GUI 410. Further, in some embodiments, the first analyte section 412 is configured to transition from displaying the first collapsed view to the first expanded view in response to a drag gesture anywhere on the sensor results GUI 410.

Further, though not illustrated, the sensor results GUI 410 can transition from displaying the second collapsed view to the second expanded view in response to a received input by the user (e.g., by a scroll gesture, a tap gesture, a pull up gesture, or by selecting or pressing a corresponding area of the touchscreen). In this manner, the second analyte section 415 can transition from displaying only the second analyte card 416, to displaying both the second analyte card 416 and the second analyte graph portion 417 on the sensor results GUI 410. Moreover, though not illustrated, the sensor results GUI 410 can transition from displaying the second expanded view to the second collapsed view in response to a received input by the user (e.g., by a scroll gesture, a tap gesture, a pull down gesture, or by selecting or pressing a corresponding area of the touchscreen). In this manner, the second analyte section 415 can transition from displaying the second analyte card 416 and the second analyte graph portion 417, to displaying only the second analyte card 416 on the sensor results GUI 410.

In some embodiments, the status of the sensor 104 (e.g., glucose ketone sensor 104) comprises an indication of a remaining lifetime of the sensor 104. In some embodiments, and as illustrated in FIGS. 4B-3 to 4B-6, the indication of the remaining lifetime of the sensor 104 comprises a graphical indication 4135, wherein the graphical indication comprises a progress indicator that is visually illustrates the remaining lifetime of the sensor 104. In some embodiments, the graphical indication 4135 is a plurality of circles (or boxes, or the like) that comprise a colored portion, and the progress indicator is each circle of the plurality of circles (or boxes, or the like) that comprises a colored portion, wherein a ratio of each circle that comprises a colored portion and a total plurality of circles is proportional to a ratio of the remaining lifetime of the sensor 104 and a total lifetime of the sensor 104. In some embodiments, the total lifetime of the sensor 104 is about 14 days. AlthoughFIGS. 4B-3 to 4B-6show 14 days as the total lifetime of the sensor 104, those of skill in the art will recognize that the progress indicator can be used for a sensor 104 having a 15-day lifetime, 20-day lifetime, 25-day lifetime, 30-day lifetime, etc.

In some embodiments, when the remaining lifetime of the sensor 104 is greater than about 1 day, the graphical indication 4135 is a first plurality of circles and the progress indicator is each circles of the first plurality of circles that comprises a first colored portion. Further, when the remaining lifetime of the sensor 104 is less than about one day (not illustrated), the graphical indication 4135 is a second plurality of circles and the progress indicator is each circles of the second plurality of circles that comprises a second colored portion. In this regard, the ratio of each circle of the second plurality of circles that comprises the second colored portion and a total second plurality of circles is proportional to a ratio of the remaining lifetime of the sensor 104 relative to the less than about one day of remaining lifetime of the sensor 104. Additionally, in some embodiments, when the remaining lifetime of the sensor 104 is less than about one hour, the graphical indication 4135 is a graphical bar and the progress indicator is a portion of the graphical bar comprising a third colored portion. In this manner, the ratio of the portion of the graphical bar that comprises the third colored portion and a total of the graphical bar is proportional to a ratio of the remaining lifetime of the sensor 104 relative to the less than about one hour of remaining lifetime of the sensor 104.

In some embodiments, the indication of the remaining lifetime of the sensor 104 comprises a text description 4136 comprising a numerical value (see, e.g., FIG. 4B-3). In some embodiments, if the remaining lifetime of the sensor 104 is greater than about one day, the numerical value of the text description 4136 comprises a number of days of the remaining lifetime of the sensor 104. In some embodiments (not shown), if the remaining lifetime of the sensor 104 is less than about one day but greater than about 1 hour, the numerical value of the text description 4136 comprises a number of hours of the remaining lifetime of the sensor 104. In some embodiments (not shown), if the remaining lifetime of the sensor 104 is less than about 1 hour, the numerical value of the text description 4136 comprises a number of minutes of the remaining lifetime of the sensor 104.

FIG. 4B-7 depicts an additional example embodiment of a sensor results GUI for use with an analyte monitoring system. Specifically,FIG. 4B-7 depicts a sensor results GUI 420, which is similar to the sensor results GUI embodiment depicted in FIGS. 4B-1 to 4B-6, except that sensor results GUI 420 is presented through a different mobile operating system (e.g., Android) than the embodiment shown in FIG. 4B-1 and 4B-2 (e.g., iOS). More specifically, and most similar to FIGS. 4B-1 to 4B-2, FIG. 4B-7 depicts the default view of the sensor results GUI 420, wherein the first analyte section 422 is displayed in the first expanded view, and the second analyte section 425 is displayed in the second expanded view. Further, in the embodiment depicted in FIG. 4B-7, the graphical indication 4236 comprises a progress indicator used for a sensor 104 having a 15-day lifetime.

FIGS. 4C-1 to 4C-6 are additional example embodiments of a sensor results GUI 430 for use with an analyte monitoring system, wherein a first analyte section 432 depicts a first current analyte level value 4302 above a target analyte range (e.g., glucose card depicting a high glucose level), and second analyte section 435 depicting a second current analyte level value 4312 exceeding a predefined normal analyte range (e.g., ketone card depicting an elevated ketone level). Generally, the sensor results GUI 430 in FIGS. 4C-1 to 4C-6 is similar to sensor results GUI 410, as described with respect to FIGS. 4B-1 to 4B-6. For example, sensor results GUI 430 comprises: (1) a menu icon 431; (2) a first analyte section 432 comprising a first analyte card 433 and/or a first analyte graph portion 434 reflecting data indicative of a first analyte level (e.g., a glucose card and/or a glucose graph portion reflecting data indicative of a glucose level); a (3) a second analyte section 435 comprising a second analyte card 436 and/or a second analyte graph portion 437 reflecting data indicative of a second analyte level (e.g., a ketone card and/or a ketone graph portion reflecting data indicative of a ketone level); (4) a selectable "Add Note" link 438; and (5) a graphical indication 4335 comprising a progress indicator that visually illustrates the sensor's 104 remaining lifetime.

As described earlier, the first analyte card 433 (e.g., glucose card) of sensor results GUI 430 can include: a text description 4301 of a first analyte condition (e.g., "High Glucose") having an alarm icon 4304 adjacent thereto, a first current analyte level value 4302 (e.g., a current glucose level of 298 mg/dL), and a first trend indicator 4303 (e.g., a directional trend arrow indicating the glucose level is rising). In addition, the second analyte card 436 (e.g., ketone card) can include a text description 4311 of a second analyte condition (e.g., "Elevated Ketones") having an alarm icon 4314 adjacent thereto, a second current analyte level value 4312 (e.g., a current ketone level of 1.1 mmol/L), and a second trend indicator 4313 (e.g., a directional trend arrow indicating the ketone level is rising).

Further, in the exemplary embodiments shown inFIGS. 4C-1 to 4C-6 , sensor results GUI 430 comprises a first analyte card 433 (e.g., a glucose card) having an orange color indicating a first current analyte level above a target analyte range (e.g., high glucose), and second analyte card 436 (e.g., a ketone card) having a yellow color indicating a second current analyte level within a predefined elevated analyte range (e.g., elevated ketones).

As described earlier, the first analyte graph portion 434 can include a first analyte trend line 4305, and the second analyte graph portion 437 can include a second analyte trend line 4325. In the exemplary embodiments depicted in FIGS. 4C-1 to 4C-3, two food icons 4308A, 4308B, and a rapid-acting insulin icon or a long-lasting insulin icon 4308C are displayed on the first analyte section 432. In addition, in the embodiments depicted in FIGS. 4C-1 to 4C-6 , the second analyte graph portion 437 further include a banner notification 4331 ("Treat as recommended by your healthcare provider") comprising instructions relating to the elevated ketone level condition. Further, banner notification 4331 further comprises a yellow background color to indicate the elevated ketone level condition. In some embodiments, the banner notification 4331 is displayed only when the user's ketone level is between a particular range of the predefined "elevated" analyte range (e.g., if the predefined "elevated" analyte range is when the user's current ketone levels are between 0.6 mmol/L and 1.4 mmol/L, then the banner notification 4331 only appears when the user's current ketone levels are between 1.0 mmol/L and 1.4 mmol/L).

As previously described, the user can interact with sensor results GUI 430 by selecting or dragging the colored circle 4310 along the first analyte trend line 4305 and causing the first analyte section 432 to display corresponding first historical analyte data and a corresponding time stamp 4309 associated with the point of the first analyte trend line 4305 where colored circle 4310 is located FIGS. 4C-2 to 4C-3).

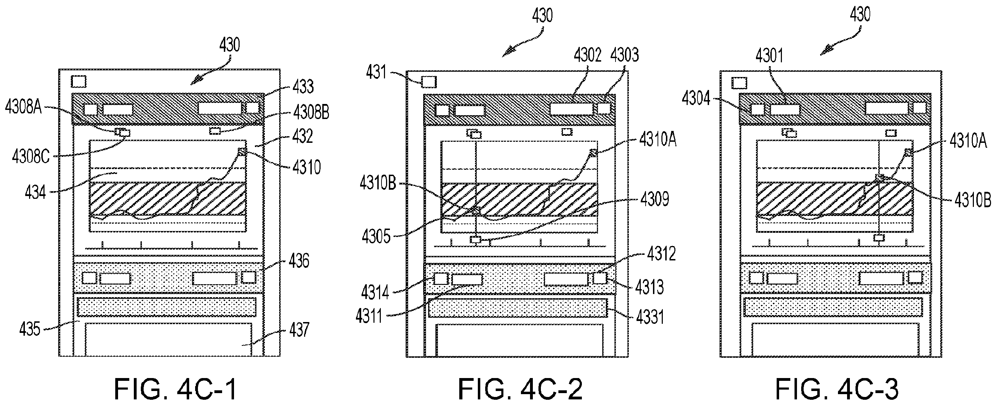

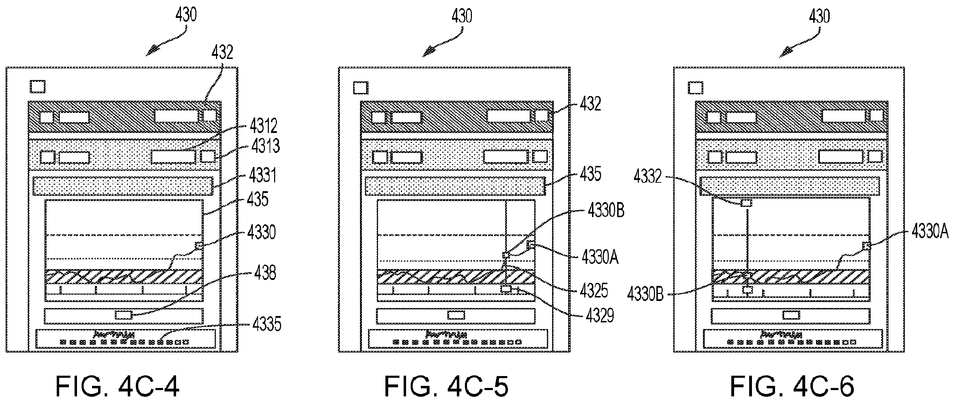

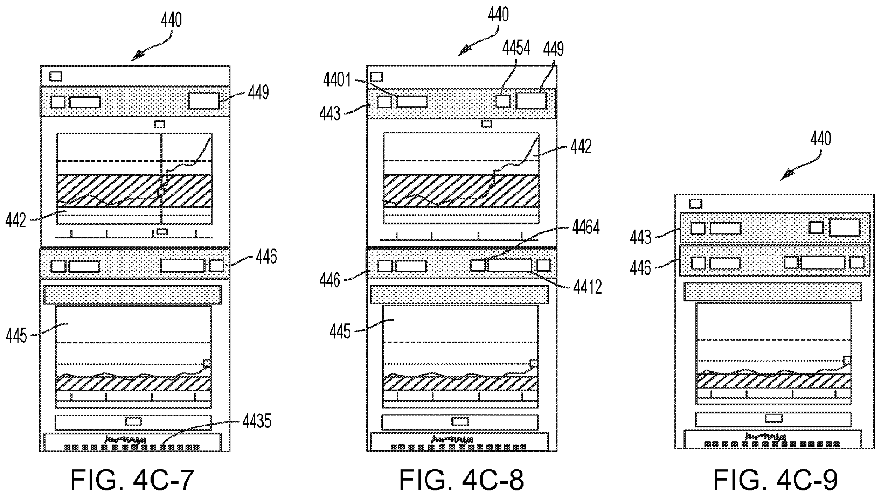

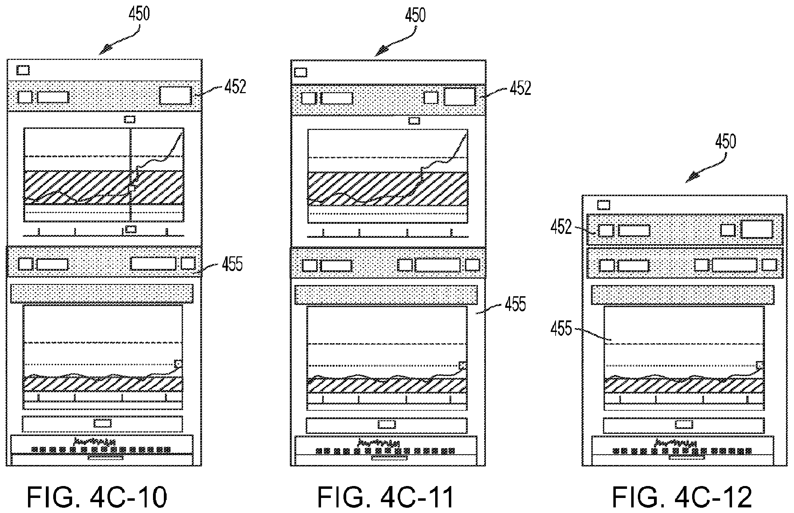

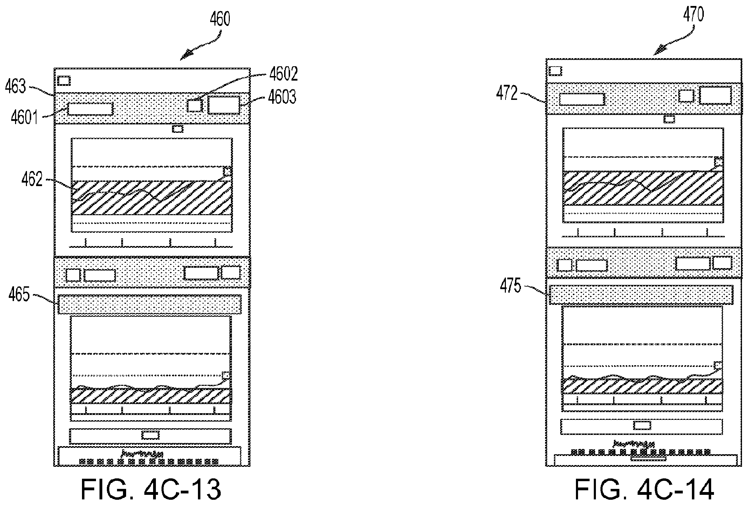

In like manner, and as described earlier, the user can interact with sensor results GUI 430 by selecting or dragging the colored circle 4330 along the second analyte trend line 4325 and causing the second analyte section 435 to display corresponding second historical analyte data and a corresponding time stamp 4329 associated with the point of the second analyte trend line 4325 where colored circle 4330 is located (best illustrated in FIGS. 4C-5 to 4C-6). In some embodiments, and similar to the embodiment depicted in FIG. 4B-6, instead of a second historical analyte level value (numerical value), the second analyte trend line 4325 displays a condition descriptor 4332 ("Normal") to indicate the condition of the second historical analyte level value being displayed (FIG. 4C-6).

In the exemplary embodiment shown in FIGS. 4C-2 to 4C-3, one or more colored circles 4310 can be displayed on the first analyte trend line , wherein a first colored circle 4310A indicates a current condition associated with the data indicative of the first analyte level (e.g., orange first colored circle 4310A indicating high glucose condition), and a second colored circle 4310B can indicate a condition associated with the data indicative of the first analyte level that is corresponding to areas on the first analyte trend line 4325 being touched or selected by the user (e.g., green second colored circle 4310B indicating a normal glucose condition in FIG. 4C-2, and a yellow second colored circle 4310B indicating glucose between a target range in FIG. 4C-3).

In a similar manner, in the exemplary embodiment shown in FIGS. 4C-5 to 4C-6, one or more colored circles 4330 can be displayed on the second analyte trend line 4325, wherein a first colored circle 4330A indicates a current condition associated with the data indicative of the second analyte level (e.g., yellow first colored circle indicating an elevated ketone condition), and a second colored circle 4330B can indicate a condition associated with the data indicative of the second analyte level that is corresponding to areas on the second analyte trend line 4325 being touched or selected by the user (e.g., orange second colored circle 4330B indicating an elevated ketone condition in FIG. 4C-5, and a green second colored circle 4330B indicating normal ketone condition in 4C-6).

As described earlier with respect to FIGS. 4B-1 to 4B-6, the first analyte section 432 of sensor results GUI 430 is configured to transition between a first collapsed view and a first expanded view, and the second analyte section 435 of sensor results GUI 430 is configured to transition between a second collapsed view and a second expanded view. In some embodiments, and as illustrated in the default view of sensor results GUI 430, which is depicted in FIGS. 4C-1 to 4C-3, the sensor results GUI 430 is configured to display the first expanded view of the first analyte section 432 by default. Further, in some embodiments, the second analyte section 435 is configured to display the second expanded view by default (FIGS. 4C-1 to 4C-3) when the data indicative of the second analyte level exceeds a predefined normal analyte range, or reaches an elevated or high analyte range. In the embodiment depicted in FIGS. 4C-1 to 4C-6, the data indicative of the second analyte level exceeds a predefined normal analyte range (elevated ketones). As such, and similar to sensor results GUI 430, the default view (FIGS. 4C-1 to 4C-3) of the sensor results GUI 430 is configured to display the first expanded view of the first analyte section 432, and the second expanded view of the second analyte section 435.

In the exemplary embodiment depicted inFIGS. 4C-1 to 4C-6, the first analyte section 432 and the second analyte section 435 can be updated in response to a predetermined input by the user, such as when the user taps, drags, scrolls, pulls the screen down with a finger, pulls the screen up with a finger, or by some other predetermined gesture. For example, the sensor results GUI 430 can transition from displaying the first expanded view (e.g., FIGS. 4C-1 to 4C-3) to the first collapsed view (FIG. 4C-4 to 4C-6) in response to a received input by the user (e.g., by a scroll gesture, a tap gesture, a pull up gesture, or by selecting or pressing a corresponding area of the touchscreen). In this regard, the first analyte section 432 can transition from displaying the first analyte card 433 and the first analyte graph portion 434, to displaying only the first analyte card 433 on the sensor results GUI 430.

In some embodiments, the sensor results GUI 430 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 432 can transition from displaying only the first analyte card 433, to displaying both the first analyte card 433 and the first analyte graph portion 434 on the sensor results GUI 430. Further, in some embodiments, the first analyte section 432 is configured to transition from displaying the first collapsed view to the first expanded view in response to a drag gesture anywhere on the sensor results GUI 430.

Although not illustrated, the sensor results GUI 430 can transition from displaying the second collapsed view to the second expanded view in response to a received input by the user (e.g., by a scroll gesture, a tap gesture, a pull up gesture, or by selecting or pressing a corresponding area of the touchscreen). As such, the second analyte section 435 can transition from displaying only the second analyte card 436, to displaying both the second analyte card 436 and the second analyte graph portion 437 on the sensor results GUI 430. Moreover (though not shown), the sensor results GUI 430 can transition from displaying the second expanded view to the second collapsed view in response to a received input by the user (e.g., by a scroll gesture, a tap gesture, a pull down gesture, or by selecting or pressing a corresponding area of the touchscreen). In this manner, the second analyte section 435 can transition from displaying the second analyte card 436 and the second analyte graph portion 437, to displaying only the second analyte card 436 on the sensor results GUI 430.

As described earlier, sensor results GUI 430 further comprises a graphical indication 4335 comprising a progress indicator that visually illustrates the remaining lifetime of the sensor 104 (e.g., a glucose-ketone sensor), wherein the progress indicator is used for a sensor 104 having a 14-day lifetime (best shown in FIGS. 4C-4 to 4C-6).

FIGS. 4C-7 to 4C-9 depict an additional example embodiment of a sensor results GUI for use with an analyte monitoring system. The sensor results GUI 440 depicted inFIGS. 4C-7 to 4C-9 is similar to the GUI embodiment depicted in FIGS. 4C-1 to 4C-6, except that the first analyte section 442 of the sensor results GUI 440 displays an out-of-range text indicator 449 (e.g., "HI") instead of a first current analyte level value and a first trend indicator. In some embodiments, and as depicted in FIGS. 4C-7 to 4C-9 , if the first current analyte level value is considered to be out-of-range with respect to a predetermined analyte threshold range, or an adverse state of condition is detected, the first current analyte level value and the first trend indicator will not be displayed on the first analyte card 443. Rather, in these embodiments, and as depicted in FIGS. 4C-7 to 4C-9 , an out-of-range text indicator 449 (e.g., "HI" or "LO") is displayed so as to indicate the out-of-range condition that is present. Though not illustrated, those of skill in the art will recognize that out-of-range text indicators can be utilized for the second analyte section 445 when the data indicative of the second analyte level exceeds a predefined analyte range, and is fully within the scope of the present disclosure.

Further, in these embodiments, and as depicted in FIGS. 4C-7 to 4C-9 , the text description 4401 of the first analyte condition (e.g., "High Glucose") further describes the trend condition (e.g., "(Out of Range)"). In addition, and unlike previous embodiments described herein, a yellow background color (instead of an orange background color) is used to indicate a high glucose level. As such, in the exemplary embodiment shown in FIGS. 4C-7 to 4C-9 , sensor results GUI 440 comprises a glucose card 443 having a yellow background color indicating a high glucose level, and a ketone card 446 having a yellow background color indicating an elevated ketones level.

Moreover, in some embodiments, and as depicted in FIG. 4C-8, the first analyte card 443 can further include a calibration icon 4454 adjacent to the out-of-range text indicator 449 or, alternatively, when depicted, adjacent to the first current analyte level value. In some embodiments, and as shown in FIG. 4C-8, the second analyte card 446 can further include a calibration icon 4464 adjacent to the second current analyte level value 4412 or, alternatively, when depicted, adjacent to the second out-of-range text indicator.

The default view of sensor results GUI 440, which is depicted in FIGS. 4C-7 and 4C-8, displays the first expanded view of the first analyte section 442, and the second expanded view of the second analyte section 445. FIG. 4C-9 depicts the sensor results GUI 440 in an updated configuration (e.g., in response to a predetermined input by the user, such as, by a scroll gesture, a tap gesture, a pull up gesture, or by selecting or pressing a corresponding area of the touchscreen), wherein it transitions from displaying the first expanded view (as shown inFIGS. 4C-7 and 4C-8) to the first collapsed view (FIG. 4C-9). In FIG. 4C-9, the second expanded view is displayed in the updated configuration of the sensor results GUI 440.

In the exemplary embodiment depicted in FIGS. 4C-7 to 4C-9, the sensor results GUI 440 further comprises a graphical indication 4435 comprising a progress indicator that visually illustrates the remaining lifetime of the sensor 104 (e.g., a glucose-ketone sensor), wherein the progress indicator is used for a sensor 104 having a 15-day lifetime.

FIGS. 4C-10 to 4C-12 depict an additional example embodiment of a sensor results GUI 450 for use with an analyte monitoring system. FIGS. 4C-10 to 4C-12 depict a sensor results GUI 450, which is similar to the GUI embodiment depicted in FIGS. 4C-7 to 4C-9, except that sensor results GUI 450 is presented through a different mobile operating system (e.g., Android) than the embodiment shown in FIGS. 4C-7 to 4C-9 (e.g., iOS). The default view of sensor results GUI 450, as depicted in FIGS. 4C-10 and 4C-11, displays the first expanded view of the first analyte section 452, and the second expanded view of the second analyte section 455. FIG. 4C-12 depicts the sensor results GUI 450 in an updated configuration (e.g., in response to a predetermined input by the user, such as, by a scroll gesture, a tap gesture, a pull up gesture, or by selecting or pressing a corresponding area of the touchscreen), wherein it transitions from displaying the first expanded view (as shown in FIGS 4C-10 and 4C-11) in the default view, to the first collapsed view (FIG. 4C-12). In FIG. 4C-12 the second expanded view is displayed in the updated configuration of the sensor results GUI 450.

FIG. 4C-13 depicts another additional example embodiment of a sensor results GUI for use with an analyte monitoring system. The sensor results GUI 460 depicted in FIG. 4C-13 is similar to the GUI embodiment depicted in FIGS. 4C-7 to 4C-9, except that the first analyte section of the sensor results GUI 460 displays a first current analyte level value 4602 and a first trend indicator 4603 instead of an out-of-range text indicator. Further, in some embodiments, and as depicted in FIG. 4C-13, the first analyte card 463 does not display an alarm icon adjacent to the text description. In some embodiments, and as illustrated in FIG. 4C-13,, the text description 4601 of the first analyte condition further describes the trend condition (e.g., "Glucose Going High"). Though not illustrated, those of skill in the art will recognize that the text description 4611 of the second analyte condition can further describe the trend condition associated therewith, and is fully within the scope of this present disclosure. FIG. 4C-13, displays a default view of sensor results GUI 460, wherein the first analyte section 462 is displayed in the first expanded view, and the second analyte section 465 is displayed in the second expanded view.

FIGS. 4C-14 depicts an additional example embodiment of a sensor results GUI for use with an analyte monitoring system. 4C-14 depicts a sensor results GUI 470, which is similar to the GUI embodiment depicted in FIG. 4C-13,, except that sensor results GUI 470 is presented through a different mobile operating system (e.g., Android) than the embodiment shown in 4C-13 (e.g., iOS). As such, FIG. 4C-14 displays a default view of sensor results GUI 470, wherein the first analyte section 472 is displayed in the first expanded view, and the second analyte section 475 is displayed in the second expanded view.

FIGS. 4D-1 and 4D-2 are an additional example embodiment of a sensor results GUI for use with an analyte monitoring system, wherein a first analyte section 482 depicts a first current analyte level value 4802 within a target analyte range (e.g., glucose card depicting a normal glucose level, or glucose in range), and wherein the second analyte section 485 depicts a second current analyte level value 4812 exceeding a predefined normal analyte range (e.g., ketone card depicting a high ketone level). Generally, sensor results GUI 480 in FIGS. 4B-1 and 4B-2 is similar to sensor results GUI 410, as described with respect to FIGS. 4B-1 to 4B-6. For example, sensor results GUI 480 comprises: (1) a menu icon 481; (2) a first analyte section 482 comprising a first analyte card 483 and/or a first analyte graph portion 484 reflecting data indicative of a first analyte level (e.g., a glucose card and/or a glucose graph portion reflecting data indicative of a glucose level); a (3) a second analyte section 485 comprising a second analyte card 486 and/or a second analyte graph portion 487 reflecting data indicative of a second analyte level (e.g., a ketone card and/or a ketone graph portion reflecting data indicative of a ketone level); (4) a selectable "Add Note" link 488; and (5) a graphical indication 4835 comprising a progress indicator that visually illustrates the sensor's 104 remaining lifetime.

As previously described, the first analyte card 483 (e.g., glucose card) of sensor results GUI 480 can include: a text description 4801 of a first analyte condition (e.g., "Glucose In Range"), a first current analyte level value 4802 (e.g., a current glucose level of 142 mg/dL), and a first trend indicator 4803 (e.g., a directional trend arrow indicating the glucose level is falling). Similarly, the second analyte card 486 (e.g., ketone card) can include a text description 4811 of a second analyte condition (e.g., "High Ketones") having an alarm icon 4814 adjacent thereto, a second current analyte level value 4812 (e.g., a current ketone level of 2.2 mmol/L), and a second trend indicator 4813 (e.g., a directional trend arrow indicating the ketone level is rising).

In the exemplary embodiment shown in FIGS. 4D-1 and 4D-2, sensor results GUI 480 comprises a first analyte card 483 (e.g., a glucose card) having a green color indicating a first current analyte level within a target analyte range (e.g., normal glucose or glucose in range), and second analyte card 486 (e.g., a ketone card) having a red color indicating a second current analyte level within a predefined high analyte range (e.g., high ketones).

Similar to sensor results GUI embodiments previously described, the first analyte graph portion 484 can include a first analyte trend line 4805, and the second analyte graph portion 487 can include a second analyte trend line 4815. In the exemplary embodiment shown in FIGS. 4D-1 and 4D-2, a rapid-acting insulin icon or a long-lasting insulin icon 4808 is displayed on the first analyte section 482. Those of skill in art will recognize that various icons 4808 can be displayed on the first analyte section 482 and/or the second analyte section 485, and are fully within the present disclosure. In addition, in the embodiments depicted in FIGS. 4D-1 and 4D-2, the second analyte graph portion 487 further include a banner notification 4831 ("Seek Medical Attention") comprising instructions relating to the high ketone level condition. Further, banner notification 4831 further comprises a red background color to indicate the high ketone level condition.

As previously mentioned, the user can interact with sensor results GUI 480 by selecting or dragging a colored circle 4810 along the first analyte trend line 4805 and causing the first analyte section 482 to display corresponding first historical analyte data and a corresponding time stamp 4809 (not shown) associated with the point of the first analyte trend line 4805 where colored circle 4810 is located.

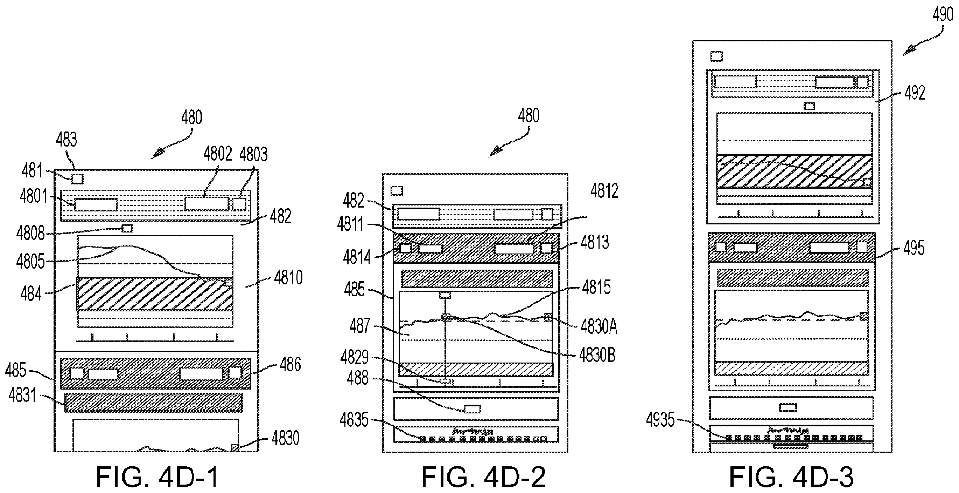

In a similar manner, the user can interact with sensor results GUI 480 by selecting or dragging a colored circle 4830 along the second analyte trend line 4825 and causing the second analyte section 485 to display corresponding second historical analyte data and a corresponding time stamp 4829 associated with the point of the second analyte trend line 4825 where colored circle 4830 is located (best illustrated in FIG. 4D-2).

In the exemplary embodiment shown in FIG. 4D-1, only one colored circle 4810 is displayed on the first analyte trend line 4805, wherein the colored circle 4810 indicates a current condition associated with the data indicative of the first analyte level (e.g., green first colored circle indicating normal glucose condition). Though not illustrated, those of skill in the art will recognize that additional colored circles 4810, as previously detailed, can be displayed on the first analyte trend line 4805, and are fully within the scope of the present disclosure. In the exemplary embodiment shown in 4D-2, two colored circles 4830A, 4830B are displayed on the second analyte trend line 4815, wherein a first colored circle 4830A indicates a current condition associated with the data indicative of the second analyte level (e.g., red first colored circle 4830A indicating high ketone condition), and a second colored circle 4830B indicates a condition associated with the data indicative of the second analyte level that is corresponding to areas on the second analyte trend line 4825 being touched or selected by the user (e.g., red second colored circle 4830B indicating a high ketone condition in FIG. 4D-2).

As described in detail earlier with respect to FIGS 4B-1 to 4B-6, the first analyte section 482 of sensor results GUI 480 is configured to transition between a first collapsed view and a first expanded view, and the second analyte section 485 of sensor results GUI 480 is configured to transition between a second collapsed view and a second expanded view. In some embodiments, and as illustrated in the default view of sensor results GUI 480, as depicted in FIG. 4D-1, the sensor results GUI 480 is configured to display the first expanded view of the first analyte section 482 by default. Further, in some embodiments, and as depicted in the default view of sensor results GUI 480 in FIG. 4D-1, the second analyte section 485 is configured to display the second expanded view by default when the data indicative of the second analyte level exceeds a predefined normal analyte range, or reaches an elevated or high analyte range In the embodiment depicted in FIGS. 4D-1 and 4D-2, the data indicative of the second analyte level exceeds a predefined normal analyte range (high ketones). As such, the default view (FIG. 4D-1) of the sensor results GUI 480 is configured to display the first expanded view of the first analyte section 482, and the second expanded view of the second analyte section 485. In some embodiments, and as shown inFIG. 4D-1, the first expanded view and the second expanded view are displayed on the sensor results GUI 480 contemporaneously.

Further, in the exemplary embodiment depicted in FIGS. 4D-1 and 4D-2, the first analyte section 482 and the second analyte section 485 can be updated in response to a predetermined input by the user, such as when the user taps, drags, scrolls, pulls the screen, or by some other predetermined gesture. For example, the sensor results GUI 480 can transition from displaying the first expanded view (e.g., FIG. 4D-1) to the first collapsed view (FIG. 4D-2) in response to a received input by the user (e.g., by a scroll gesture, a tap gesture, a pull up gesture, or by selecting or pressing a corresponding area of the touchscreen). In this regard, the first analyte section 482 can transition from displaying the first analyte card 483 and the first analyte graph portion 484, to displaying only the first analyte card 483 on the sensor results GUI 480. In some embodiments, the sensor results GUI 480 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 482 can transition from displaying only the first analyte card 483, to displaying both the first analyte card 483 and the first analyte graph portion 484 on the sensor results GUI 480. Additionally, in some embodiments, the first analyte section 482 is configured to transition from displaying the first collapsed view to the first expanded view in response to a drag gesture anywhere on the sensor results GUI 480.

Though not depicted in FIGS. 4D-1 and 4D-2, the sensor results GUI 480 can transition from displaying the second collapsed view to the second expanded view in response to a received input by the user (e.g., by a scroll gesture, a tap gesture, a pull up gesture, or by selecting or pressing a corresponding area of the touchscreen). As such, the second analyte section 485 can transition from displaying only the second analyte card 486, to displaying both the second analyte card 486 and the second analyte graph portion 487 on the sensor results GUI 480. In addition (not shown), the sensor results GUI 480 can transition from displaying the second expanded view to the second collapsed view in response to a received input by the user (e.g., by a scroll gesture, a tap gesture, a pull down gesture, or by selecting or pressing a corresponding area of the touchscreen). In this manner, the second analyte section 485 can transition from displaying the second analyte card 486 and the second analyte graph portion 487, to displaying only the second analyte card 486 on the sensor results GUI 480.

In the exemplary embodiment depicted in FIGS. 4D-1 and 4D-2, sensor results GUI 480 further comprises a graphical indication 4835 comprising a progress indicator that visually illustrates the remaining lifetime of the sensor 104 (e.g., a glucose-ketone sensor), wherein the progress indicator is used for a sensor 104 having a 14-day lifetime (best shown in FIG. 4D-2).

FIG. 4D-3 depicts an additional example embodiment of a sensor results GUI for use with an analyte monitoring system. FIG. 4D-3 depicts a sensor results GUI 490, which is similar to the GUI embodiments depicted inFIGS. 4D-1 and 4D-2, except that sensor results GUI 490 is presented through a different mobile operating system (e.g., Android) than the embodiment shown in FIGS. 4D-1 and 4D-2 (e.g., iOS). Specifically, FIG. 4D-3 depicts a default view of the sensor results GUI 490, wherein the first analyte section 492 is displayed in the first expanded view and the second analyte section 495 is displayed in the second expanded view (similar to FIG. 4D-1). Unlike the embodiment described in FIGS. 4D-1 and 4D-2, however, the sensor results GUI 490 of FIG. 4D-3 comprises a graphical indication 4935 comprising a progress indicator that visually illustrates the remaining lifetime of the sensor 104 (e.g., a glucose-ketone sensor), wherein the progress indicator is used for a sensor 104 having a 15-day lifetime.

FIGS. 4E-1 to 4E-4 depict an additional example embodiment of a sensor results GUI for use with an analyte monitoring system, wherein a first analyte section 812 depicts a first current analyte level value 8102 above a target analyte range (e.g., glucose card depicting a high glucose level), and second analyte section 815 having a second current analyte level value 8112 (not illustrated) within a predefined normal analyte range (e.g., ketone card depicting a normal ketone level). Generally, sensor results GUI 810 in FIGS. 4E-1 to 4E-4 is similar to sensor results GUI 410, as described with respect to FIGS. 4B-1 to 4B-6.

For example, sensor results GUI 810 comprises: (1) a menu icon 811; (2) a first analyte section 812 comprising a first analyte card 813 and/or a first analyte graph portion 814 reflecting data indicative of a first analyte level (e.g., a glucose card and/or a glucose graph portion reflecting data indicative of a glucose level); a (3) a second analyte section 815 comprising a second analyte card 816 and/or a second analyte graph portion reflecting data indicative of a second analyte level (e.g., a ketone card and/or a ketone graph portion reflecting data indicative of a ketone level); (4) a selectable "Add Note" link 818; and (5) a graphical indication 8135 comprising a progress indicator that visually illustrates the sensor's 104 remaining lifetime.

As mentioned earlier, the first analyte card 813 (e.g., glucose card) of sensor results GUI 810 can include: a text description 8101 of a first analyte condition (e.g., "High Glucose"), a first current analyte level value 8102 (e.g., a current glucose level of 256 mg/dL), and a first trend indicator 8103 (e.g., a directional trend arrow indicating the glucose level is rising). Further, in some embodiments, and as depicted in FIGS. 4E-1 to 4E-4, the first analyte card can further include an alarm icon 8104 adjacent to the text description 8101 of the first analyte condition.

Further, and as illustrated in FIGS. 4E-1 to 4E-4, the second analyte card 815 (e.g., ketone card) can include a text description 8111 of a second analyte condition (e.g., "Normal Ketones"). In another aspect of the embodiments, however, and as depicted in FIGS. 4E-1 to 4E-4, if an adverse state of condition is not detected, or if a second current analyte level value 8112 (not illustrated) is considered to be in a "normal" or a predefined analyte threshold range, the second current analyte level value 8112 (not illustrated) and a second trend indicator 8113 (not shown) will not be displayed on the second analyte card.

In the exemplary embodiments shown in FIGS. 4E-1 to 4E-4, sensor results GUI 810 comprises a first analyte card 813 (e.g., a glucose card) having an orange color indicating a first current analyte level above a target analyte range (e.g., high glucose), and second analyte card 815 (e.g., a ketone card) having a green color indicating a second current analyte level within a predefined normal or threshold analyte range (e.g., normal ketones).

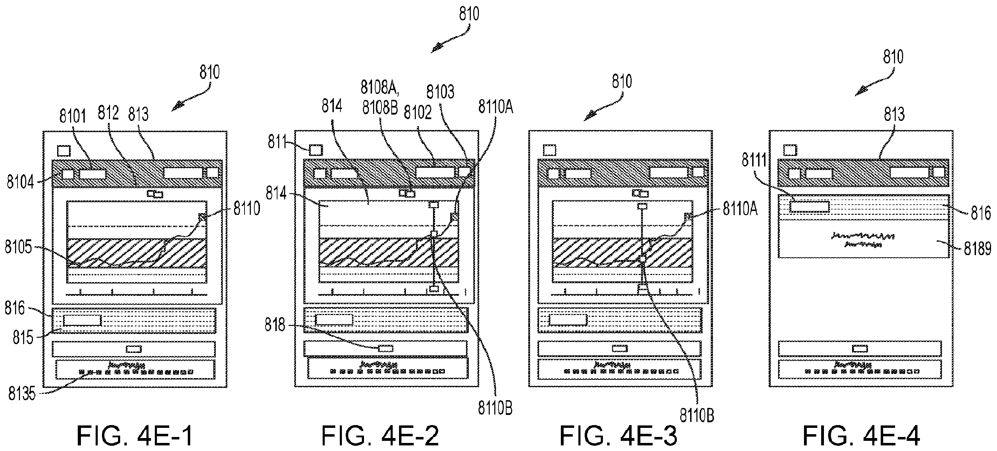

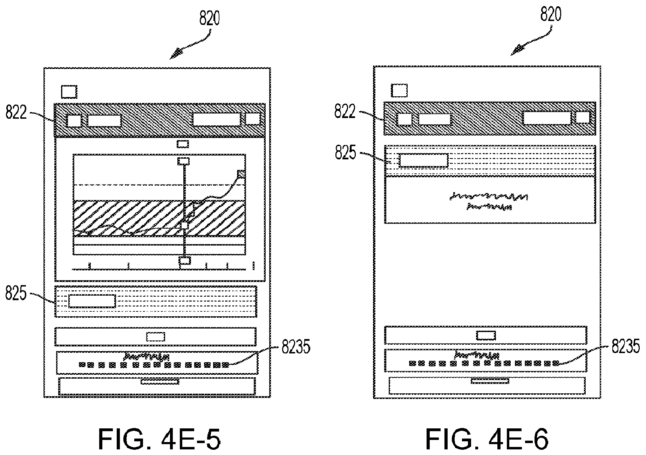

As described earlier, the first analyte graph portion 814 can include a first analyte trend line 8105. Further, and as depicted in FIGS. 4E-1 to 4E-3, a food icon 8108A, and a rapid-acting insulin icon or a long-lasting insulin icon 8108B are displayed on the first analyte section 812. As previously described, the user can interact with sensor results GUI 810 by selecting or dragging the colored circle 8110 along the first analyte trend line 8105 and causing the first analyte section 812 to display corresponding first historical analyte data and a corresponding time stamp 8109 associated with the point of the first analyte trend line 8105 where colored circle 8110 is located (FIGS. 4E-2 and 4E-3). Moreover, one or more colored circles 8110 can be displayed on the first analyte trend line 8105, wherein a first colored circle 8110A indicates a current condition associated with the data indicative of the first analyte level (e.g., orange first colored circle indicating high glucose condition), and a second colored circle 8110B can indicate a condition associated with the data indicative of the first analyte level that is corresponding to areas on the first analyte trend line 8105 being touched or selected by the user (e.g., yellow second colored circle 8110B indicating a glucose condition between a target range, as seen in FIG. 4E-2, and a green second colored circle 8110B indicating glucose within a target range, as shown in FIG. 4E-3).

In one aspect of the embodiments, however, a second analyte trend line 8125 (not shown) only appears if an adverse state of condition is detected, or if a second current analyte level value 8112 (not shown) is considered to exceed a "normal" or a predefined analyte threshold range. As such, in the exemplary embodiment illustrated in FIGS. 4E-1 to 4E-4, the second analyte section 812 does not display a second analyte trend line 8105. Instead, and as illustrated in 4E-4, when the data indicative of the second analyte level is at a predefined normal analyte range (e.g., normal ketones), the second analyte section 815 is configured to display the second analyte card 816 (e.g., ketone card) and a message 8189 relating to the predefined or normal threshold range (e.g., "You have normal ketones; 0-0.5mmol/L").

With reference to FIGS. 4E-1 to 4E-4, the first analyte section 812 of sensor results GUI 810 is configured to transition between a first collapsed view and a first expanded view, and the second analyte section 815 of sensor results GUI 810 is configured to transition between a second collapsed view and a second expanded view. In some embodiments, and as illustrated in the default view of sensor results GUI 810, depicted in FIGS. 4E-1 to 4E-3, the sensor results GUI 810 is configured to display the first expanded view of the first analyte section 812 by default. Further, in some embodiments, and as illustrated in the default view of sensor results GUI 810, depicted in FIGS. 4E-1 to 4E-3, the sensor results GUI 810 is configured to display the second collapsed view of the second analyte section 815 by default when the data indicative of the second analyte level is at a predefined normal analyte range (e.g., normal ketone threshold). In the embodiment depicted in FIGS. 4E-1 to 4E-4, the data indicative of the second analyte level is at a predefined normal analyte range (normal ketones). As such, the default view (FIGS. 4E-1 to 4E-3) of the sensor results GUI 810 is configured to display the first expanded view of the first analyte section 812, and the second collapsed view of the second analyte section 815.

As previously described, and in the exemplary embodiment depicted in FIGS. 4E-1 to 4E-4, the first analyte section 812 and the second analyte section 815 can be updated in response to a predetermined input by the user, such as when the user taps, drags, scrolls, pulls the screen down with a finger, pulls the screen up with a finger, or by some other predetermined gesture. For example, the sensor results GUI 810 can transition from displaying the first expanded view (e.g., FIGS. 4E-1 to 4E-3) to the first collapsed view (FIG. 4E-4) in response to a received input by the user (e.g., by a scroll gesture, a tap gesture, a pull up gesture, or by selecting or pressing a corresponding area of the touchscreen). In this regard, the first analyte section 812 can transition from displaying the first analyte card 813 and the first analyte graph portion 814, to displaying only the first analyte card 813 on the sensor results GUI 810. Further, in some embodiments, the sensor results GUI 810 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 812 can transition from displaying only the first analyte card 813, to displaying both the first analyte card 813 and the first analyte graph portion 814 on the sensor results GUI 810. In some embodiments, the first analyte section 812 is configured to transition from displaying the first collapsed view to the first expanded view in response to a drag gesture anywhere on the sensor results GUI 810.

In addition, the sensor results GUI 810 can transition from displaying the second collapsed view (FIG. 4E-1 to 4E-3) to the second expanded view (FIG. 4E-4) in response to a received input by the user (e.g., by a scroll gesture, a tap gesture, a pull up gesture, or by selecting or pressing a corresponding area of the touchscreen). In some embodiments, and as illustrated in FIG. 4E-4, when the data indicative of the second analyte level is at a predefined normal analyte range (e.g., normal ketones), the second expanded view will display the second analyte card 816 (e.g., ketone card) and the message 8189 relating to the predefined or normal threshold range (e.g., "You have normal ketones; 0-0.5mmol/L"). As such, if the second analyte section 815 transitions from displaying the second collapsed view to the second expanded view, the second analyte section 815 will transition from displaying only the second analyte card 816, to displaying both the second analyte card 816 and message 8189 relating to the predefined normal threshold range (FIG. 4E-4). Moreover, the sensor results GUI 810 can transition from displaying the second expanded view 8148 to the second collapsed view 8146 in response to a received input by the user (e.g., by a scroll gesture, a tap gesture, a pull down gesture, or by selecting or pressing a corresponding area of the touchscreen). In this manner, the second analyte section 815 can transition from displaying the second analyte card 816 and the message 8189 relating to the predefined normal threshold range (or a second analyte graph portion, if displayed), to displaying only the second analyte card 816 on the sensor results GUI 810.

The sensor results GUI 810 embodiment depicted in FIGS. 4E-1 to 4E-4 further comprises a graphical indication 8135 comprising a progress indicator that visually illustrates the remaining lifetime of the sensor 104 (e.g., a glucose-ketone sensor), wherein the progress indicator is used for a sensor 104 having a 14-day lifetime.

FIGS. 4E-5 and 4E-6 depict an additional example embodiment of a sensor results GUIs for use with an analyte monitoring system. FIGS. 4E-5 and 4E-6 depict a sensor results GUI 820, which is similar to the GUI embodiment depicted in FIGS. 4E-1 to 4E-4, except that sensor results GUI 820 is presented through a different mobile operating system (e.g., Android) than the embodiment shown in FIGS. 4E-1 to 4E-4 (e.g., iOS). Specifically, FIG. 4E-5 depicts sensor results GUI 820 in its default view, wherein the first analyte section 822 is displayed in the first expanded view, and the second analyte section 825 is displayed in its second collapsed view, similar to FIGS. 4E-1 to 4E-3. Further, FIG. 4E-6 depicts sensor results GUI 820 in an updated configuration (e.g., in response to a predetermined input by the user, such as, by a scroll gesture, a tap gesture, a pull up gesture, or by selecting or pressing a corresponding area of the touchscreen), wherein the first analyte section 822 transitions from displaying the first expanded view (as shown in FIGS 4E-5), to the first collapsed view (FIG. 4E-6). In addition, FIG. 4E-6 depicts the second expanded view in the updated configuration of the sensor results GUI 820. Further, in the embodiment depicted in FIGS. 4E-5 and 4E-6, the progress indicator 8235 of the graphical indication is used for a sensor 104 having a 15-day lifetime.

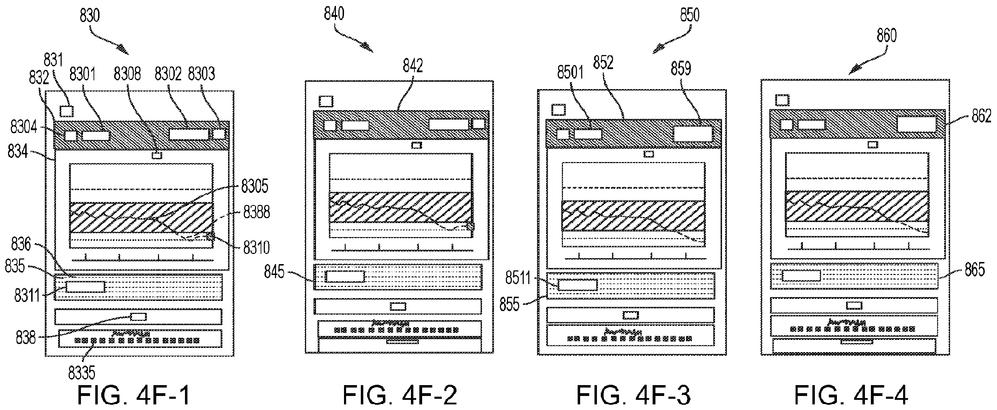

FIG. 4F-1 is an additional example embodiment of a sensor results GUI for use with an analyte monitoring system, wherein a first analyte section 832 depicts a first current analyte level value 8302 below a target analyte range (e.g., glucose card depicting a low glucose level), and second analyte section 835 having a second current analyte level value 8312 (not shown) at a predefined normal analyte range (e.g., ketone card depicting a normal ketone level). Generally, sensor results GUI 830 in FIG. 4F-1 is similar to sensor results GUI 410, as described with respect to FIGS. 4B-1 to 4B-6. For example, sensor results GUI 830 comprises: (1) a menu icon 831; (2) a first analyte section 832 comprising a first analyte card 833 and/or a first analyte graph portion 834 reflecting data indicative of a first analyte level (e.g., a glucose card and/or a glucose graph portion reflecting data indicative of a glucose level); a (3) a second analyte section 835 comprising a second analyte card 836 and/or a second analyte graph portion reflecting data indicative of a second analyte level (e.g., a ketone card and/or a ketone graph portion reflecting data indicative of a ketone level); (4) a selectable "Add Note" link 838; and (5) a graphical indication 8335 comprising a progress indicator that visually illustrates the sensor's 104 remaining lifetime.

Specifically, the first analyte card 833 (e.g., glucose card) of sensor results GUI 830 can include: a text description 8301 of a first analyte condition (e.g., "Low Glucose") having an alarm icon 8304 adjacent thereto, a first current analyte level value 8302 (e.g., a current glucose level of 69 mg/dL), and a first trend indicator 8303 (e.g., a directional trend arrow indicating the glucose level is falling).

More specifically, and as illustrated in FIG. 4F-1, the second analyte card 836 (e.g., ketone card) can include a text description 8311 of a second analyte condition (e.g., "Normal Ketones"). In another aspect of the embodiments, however, and as depicted in FIG. 4F-1, if an adverse state of condition is not detected, or if a second current analyte level value 8312 (not shown) is considered to be in a "normal" or a predefined analyte threshold range, the second current analyte level value 8312 (not shown) and a second trend indicator 8313 (not shown) will not be displayed on the second analyte card 836.

Still referring to FIG. 4F-1, sensor results GUI 830 comprises a first analyte card 833 (e.g., a glucose card) having a red color indicating a first current analyte level below a target analyte range (e.g., low glucose), and second analyte card 836 (e.g., a ketone card) having a green color indicating a second current analyte level within a predefined normal or threshold analyte range (e.g., normal ketones).

As described earlier, the first analyte graph portion 834 can include a first analyte trend line 8305 ( FIG. 4F-1). Further, and as depicted in FIG. 4F-1, a rapid-acting insulin icon or a long-lasting insulin icon 8308 are displayed on the first analyte section 832. As previously described, the user can interact with sensor results GUI 830 by selecting or dragging the colored circle 8310 along the first analyte trend line 8305 and causing the first analyte section 832 to display corresponding first historical analyte data and a corresponding time stamp 8309 (not shown) associated with the point of the first analyte trend line 8305 where colored circle 8310 is located. Moreover, though not shown, one or more colored circles 8310 can be displayed on the first analyte trend line 8305.

In some embodiments, and as illustrated in FIG. 4F-1, a portion or an area of the first analyte trend line 8305 includes a colored line portion 8388, wherein the colored line portion 8388 indicates a condition corresponding to the data indicative of the first analyte level associated with said area or portion of the first analyte trend line 8305 (e.g., an area of the first analyte trend line 8305 is colored red so as to indicate a low glucose level corresponding to the area of the first analyte trend line 8305 that comprises the colored line portion 8388). Though not shown, those of skill in the art will recognize that a colored line portion on a second analyte trend line 8315 with any of the embodiments described herein can be implemented, and is fully within the scope of the present disclosure.

In another aspect of the embodiments, a second analyte trend line 8315 (not shown) only appears if an adverse state of condition is detected, or if a second current analyte level value 8312 (not shown) is considered to exceed a "normal" or a predefined analyte threshold range. As such, in the exemplary embodiment illustrated in FIG. 4F-1, the second analyte section 835 does not display a second analyte trend line 8315 because the second current analyte level value 8312 (not shown) is in a normal range. Instead, when the data indicative of the second analyte level is at a predefined normal analyte range (e.g., normal ketones), the second analyte section 835 is configured to display the second analyte card 836 (e.g., ketone card) and a message 8389 (not shown) relating to the predefined or normal threshold range (e.g., "You have normal ketones; 0-0.5mmol/L").

With reference to FIG. 4F-1, the first analyte section 832 of sensor results GUI 830 is configured to transition between a first collapsed view and a first expanded view, and the second analyte section 835 of sensor results GUI 830 is configured to transition between a second collapsed view and a second expanded view. In some embodiments, and as illustrated in the default view of sensor results GUI 830, depicted in 4F-1, the sensor results GUI 830 is configured to display the first expanded view of the first analyte section 832 by default. Further, in some embodiments, and as illustrated in the default view of sensor results GUI 830, depicted in FIG. 4F-1, the sensor results GUI 830 is configured to display the second collapsed view of the second analyte section 835 by default when the data indicative of the second analyte level is at a predefined normal analyte range (e.g., normal ketone threshold). In the embodiment depicted in FIG. 4F-1, the data indicative of the second analyte level is at a predefined normal analyte range (normal ketones). As such, the default view (FIG. 4F-1) of the sensor results GUI 830 is configured to display the first expanded view of the first analyte section 832, and the second collapsed view of the second analyte section 835.

As previously described, and in the exemplary embodiment depicted in FIG. 4F-1, the first analyte section 832 and the second analyte section 835 can be updated in response to a predetermined input by the user, such as when the user taps, drags, scrolls, pulls the screen down with a finger, pulls the screen up with a finger, or by some other predetermined gesture. For example, the sensor results GUI 830 can transition from displaying the first expanded view (e.g., FIG. 4F-1) to the first collapsed view (not illustrated) in response to a received input by the user (e.g., by a scroll gesture, a tap gesture, a pull up gesture, or by selecting or pressing a corresponding area of the touchscreen). In this regard, the first analyte section 832 can transition from displaying the first analyte card 833 and the first analyte graph portion, to displaying only the first analyte card 833 on the sensor results GUI 830. Further, in some embodiments, the sensor results GUI 830 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 832 can transition from displaying only the first analyte card 833, to displaying both the first analyte card 833 and the first analyte graph portion on the sensor results GUI 830. In some embodiments, the first analyte section 832 is configured to transition from displaying the first collapsed view to the first expanded view in response to a drag gesture anywhere on the sensor results GUI 830.

In addition, the sensor results GUI 830 can transition from displaying the second collapsed view FIG. 4F-1) to the second expanded view (not depicted) in response to a received input by the user (e.g., by a scroll gesture, a tap gesture, a pull up gesture, or by selecting or pressing a corresponding area of the touchscreen). In some embodiments, when the data indicative of the second analyte level is at a predefined normal analyte range (e.g., normal ketones), the second expanded view will display the second analyte card 836 (e.g., ketone card) and the message 8389 (not shown) relating to the predefined or normal threshold range (e.g., "You have normal ketones; 0-0.5mmol/L"). As such, if the second analyte section 835 transitions from displaying the second collapsed view to the second expanded view, the second analyte section 835 will transition from displaying only the second analyte card 836, to displaying both the second analyte card 836 and message 8389 (not shown) relating to the predefined normal threshold range (not shown). Moreover, the sensor results GUI can transition from displaying the second expanded view to the second collapsed view in response to a received input by the user (e.g., by a scroll gesture, a tap gesture, a pull down gesture, or by selecting or pressing a corresponding area of the touchscreen). In this manner, the second analyte section 835 can transition from displaying the second analyte card 836 and the message 8389 (not shown) relating to the predefined normal threshold range (or a second analyte graph portion, if displayed), to displaying only the second analyte card 836 on the sensor results GUI 830.

The sensor results GUI 830 embodiment depicted inFIG. 4F-1 further comprises a graphical indication 8335 comprising a progress indicator that visually illustrates the remaining lifetime of the sensor 104 (e.g., a glucose-ketone sensor), wherein the progress indicator is used for a sensor 104 having a 15-day lifetime.

FIG. 4F-2 depicts an additional example embodiment of a sensor results GUIs for use with an analyte monitoring system. FIG. 4F-2 depicts a sensor results GUI 840, which is similar to the GUI embodiment depicted inFIG. 4F-1, except that sensor results GUI 840 is presented through a different mobile operating system (e.g., Android) than the embodiment shown inFIG. 4F-1 (e.g., iOS). Specifically, FIG. 4F-1depicts sensor results GUI 840 in its default view, wherein the first analyte section 842 is displayed in the first expanded view, and the second analyte section 845 is displayed in its second collapsed view, similar to FIG. 4F-1.

FIG. 4F-3 depicts an additional example embodiment of a sensor results GUIs for use with an analyte monitoring system. FIG. 4F-3 depicts a sensor results GUI 850, which is similar to the GUI embodiment depicted inFIG. 4F-1, except that the first analyte section 852 of sensor results GUI 850 displays an out-of-range text indicator 859 (e.g., "LO") instead of a first current analyte level value and a first trend indicator. Specifically, in some embodiments, if the first current analyte level value is considered to be out-of-range with respect to a predetermined analyte threshold range, or an adverse state of condition is detected, the first current analyte level value and the first trend indicator will not be displayed on the first analyte card 852. Rather, in these embodiments, and as depicted in FIG. 4F-3, an out-of-range text indicator 859 (e.g., "HI" or "LO") is displayed so as to indicate the out-of-range condition that is present. In addition, and as depicted in FIG. 4F-3, the text description 8501 of the first analyte condition further describes the trend condition (e.g., "(Out of Range)"). Though not illustrated, those of skill in the art will recognize that the text description 8511 of the second analyte condition can further describe the trend condition, and is fully within the scope of this present disclosure. Specifically, FIG. 4F-3 depicts sensor results GUI 850 in its default view, wherein the first analyte section 852 is displayed in the first expanded view, and the second analyte section 855 is displayed in its second collapsed view, similar toFIG. 4F-1.

FIG. 4F-4 depicts an additional example embodiment of a sensor results GUIs for use with an analyte monitoring system. FIG. 4F-4 depicts a sensor results GUI 860, which is similar to the GUI embodiment depicted in FIG. 4F-3, except that sensor results GUI 860 is presented through a different mobile operating system (e.g., Android) than the embodiment shown in FIG. 4F-3 (e.g., iOS). Specifically, FIG. 4F-4depicts sensor results GUI 860 in its default view, wherein the first analyte section 862 is displayed in the first expanded view, and the second analyte section 865 is displayed in its second collapsed view, similar to FIGS. 4F-3.

FIGS. 4G-1 to 4G-4 depict another example embodiment of a sensor results GUI for use with an analyte monitoring system, wherein a first analyte section 872 depicts a first current analyte level value 8702 with a target analyte range (e.g., glucose card depicting a normal glucose level), and second analyte section 875 depicting a second current analyte level value 8712 within a predefined normal analyte range (e.g., ketone card depicting a normal ketone level). Generally, sensor results GUI 870 in FIGS. 4G-1 to 4G-4 is similar to sensor results GUI 410, as described with respect to FIGS. 4B-1 to 4B-6. For example, sensor results GUI 870 comprises: (1) a menu icon 871; (2) a first analyte section 872 comprising a first analyte card 873 and/or a first analyte graph portion 874 reflecting data indicative of a first analyte level (e.g., a glucose card and/or a glucose graph portion reflecting data indicative of a glucose level); a (3) a second analyte section 875 comprising a second analyte card 876 and/or a second analyte graph portion reflecting data indicative of a second analyte level (e.g., a ketone card and/or a ketone graph portion reflecting data indicative of a ketone level); (4) a selectable "Add Note" link 878; and (5) a graphical indication 8735 comprising a progress indicator that visually illustrates the sensor's 104 remaining lifetime.

Specifically, the first analyte card 873 (e.g., glucose card) can include: a text description 8701 of a first analyte condition (e.g., "Glucose In Range"), a first current analyte level value 8702 (e.g., a current glucose level of 126 mg/dL), and a first trend indicator 8703 (e.g., a directional trend arrow indicating the glucose level is rising). More specifically, and as illustrated in FIGS. 4G-1 to 4G-4, the second analyte card 876 (e.g., ketone card) can include a text description 8711 of a second analyte condition (e.g., "Normal Ketones"). In another aspect of the embodiments, however, and as depicted in FIGS. 4G-1 to 4G-4, if an adverse state of condition is not detected, or if a second current analyte level value 8712 (not shown) is considered to be in a "normal" or a predefined analyte threshold range, the second current analyte level value 8712 (not shown) and a second trend indicator 8713 (not shown) will not be displayed on the second analyte card 876.

Moreover, in the exemplary embodiments shown in FIGS. 4G-1 to 4G-4, sensor results GUI 870 comprises a first analyte card 873 (e.g., a glucose card) having an green color indicating a first current analyte level within a target analyte range (e.g., glucose in range or normal glucose), and second analyte card 876 (e.g., a ketone card) having a green color indicating a second current analyte level within a predefined normal or threshold analyte range (e.g., normal ketones).

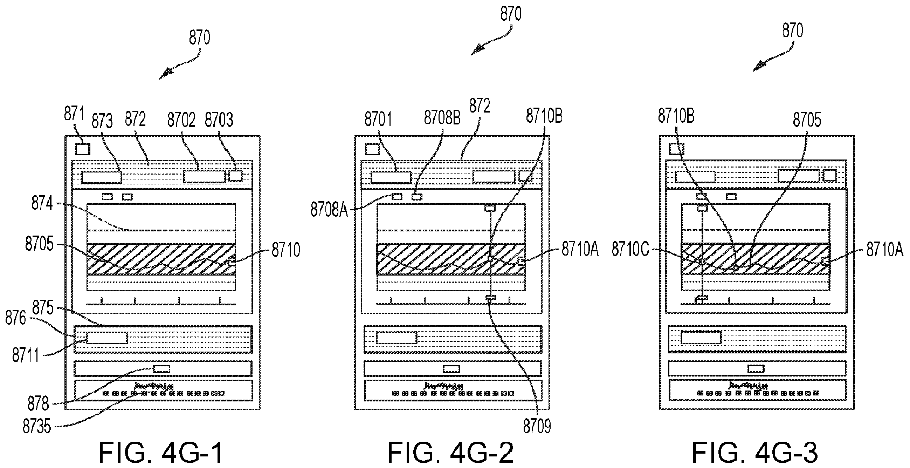

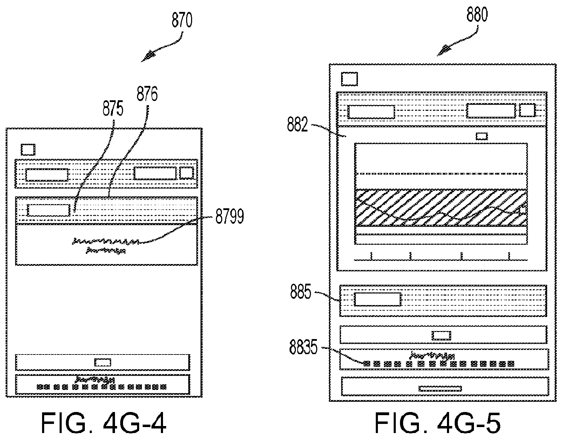

As previously described, the first analyte graph portion 874 can include a first analyte trend line 8705. Further, and as depicted in FIGS. 4G-1 to 4G-3, a food icon 8708A, and a rapid-acting insulin icon or a long-lasting insulin icon 8708B are displayed on the first analyte section 872. As previously described, the user can interact with sensor results GUI 870 by selecting or dragging the colored circle 8710 along the first analyte trend line 8705 and causing the first analyte section 872 to display corresponding first historical analyte data and a corresponding time stamp 8709 associated with the point of the first analyte trend line 8705 where colored circle 8710 is located (FIGS. 4G-2 and 4G-3). Moreover, one or more colored circles 8710 can be displayed on the first analyte trend line 8705, wherein a first colored circle 8710A indicates a current condition associated with the data indicative of the first analyte level (e.g., green first colored circle 8710A indicating normal glucose condition), and a second colored circle 8710B can indicate a condition associated with the data indicative of the first analyte level that is corresponding to areas on the first analyte trend line 8705 being touched or selected by the user (e.g., green second colored circle 8710B indicating a normal glucose condition, as seen in FIGS. 4G-2 and 4G-3). In addition, the first analyte trend line 8705 can further include a third colored circle 8710C to indicate a condition associated with the data indicative of the first analyte level that is corresponding to a second area the user selects or touches on the first analyte trend line 8705 (e.g., green third colored circle 8710C indicating a normal glucose condition, as seen in FIGS. 4G-3).

In another aspect of the embodiments, a second analyte trend line 8725 only appears if an adverse state of condition is detected, or if a second current analyte level value 8712 (not shown) is considered to exceed a "normal" or a predefined analyte threshold range. As such, in the exemplary embodiment illustrated in FIGS. 4G-1 to 4G-4, the second analyte section 865 does not display a second analyte trend line 8715 (not shown). Instead, and as illustrated in FIG. 4G-4, when the data indicative of the second analyte level is at a predefined normal analyte range (e.g., normal ketones), the second analyte section 875 is configured to display the second analyte card 876 (e.g., ketone card) and a message 8799 relating to the predefined or normal threshold range (e.g., "You have normal ketones; 0-0.5mmol/L").

Still referring to FIGS. 4G-1 to 4G-4, the first analyte section 872 of sensor results GUI 870 is configured to transition between a first collapsed view and a first expanded view, and the second analyte section 875 of sensor results GUI 870 is configured to transition between a second collapsed view and a second expanded view. In some embodiments, and as illustrated in the default view of sensor results GUI 870, depicted in FIGS. 4G-1 to 4G-3, the sensor results GUI 870 is configured to display the first expanded view of the first analyte section 872 by default. Further, in some embodiments, and as illustrated in the default view of sensor results GUI 870, depicted in FIGS. 4G-1 to 4G-3, the sensor results GUI 870 is configured to display the second collapsed view of the second analyte section 875 by default when the data indicative of the second analyte level is at a predefined normal analyte range (e.g., normal ketone threshold). In the embodiment depicted in FIGS. 4G-1 to 4G-4, the data indicative of the second analyte level is at a predefined normal analyte range (normal ketones). As such, the default view (FIGS. 4G-1 to 4G-3) of the sensor results GUI 870 is configured to display the first expanded view of the first analyte section 872, and the second collapsed view of the second analyte section 875.

As previously described, and in the exemplary embodiment depicted in FIGS. 4G-1 to 4G-4, the first analyte section 872 and the second analyte section 875 can be updated in response to a predetermined input by the user, such as when the user taps, drags, scrolls, pulls the screen down with a finger, pulls the screen up with a finger, or by some other predetermined gesture. For example, the sensor results GUI 870 can transition from displaying the first expanded view (e.g., FIGS. 4G-1 to 4G-3) to the first collapsed view (FIG. 4G-4) in response to a received input by the user (e.g., by a scroll gesture, a tap gesture, a pull up gesture, or by selecting or pressing a corresponding area of the touchscreen). In this regard, the first analyte section 872 can transition from displaying the first analyte card 873 and the first analyte graph portion 874, to displaying only the first analyte card 873 on the sensor results GUI 870. Further, in some embodiments, the sensor results GUI 870 can transition from displaying the first collapsed view to the first expanded view in response to a received input by the user (e.g., by a scroll gesture, a tap gesture, a pull down gesture, or by selecting or pressing a corresponding area of the touchscreen). In this regard, the first analyte section 872 can transition from displaying only the first analyte card 873, to displaying both the first analyte card 873 and the first analyte graph portion 874 on the sensor results GUI 870. In some embodiments, the first analyte section 872 is configured to transition from displaying the first collapsed view to the first expanded view in response to a drag gesture anywhere on the sensor results GUI 870.

Further, the sensor results GUI 870 can transition from displaying the second collapsed view (FIG. 4G-1 to 4G-3) to the second expanded view (FIG. 4G-4) in response to a received input by the user (e.g., by a scroll gesture, a tap gesture, a pull up gesture, or by selecting or pressing a corresponding area of the touchscreen). In some embodiments, and as illustrated in FIG. 4G-4, when the data indicative of the second analyte level is at a predefined normal analyte range (e.g., normal ketones), the second expanded view will display the second analyte card 876 (e.g., ketone card) and the message 8789 relating to the predefined or normal threshold range (e.g., "You have normal ketones; 0-0.5mmol/L"). As such, if the second analyte section 875 transitions from displaying the second collapsed view to the second expanded view, the second analyte section 875 will transition from displaying only the second analyte card 876, to displaying both the second analyte card 876 and message 8789 relating to the predefined normal threshold range (FIG. 4G-4). Moreover, the sensor results GUI 870 can transition from displaying the second expanded view to the second collapsed view in response to a received input by the user (e.g., by a scroll gesture, a tap gesture, a pull down gesture, or by selecting or pressing a corresponding area of the touchscreen). In this manner, the second analyte section 875 can transition from displaying the second analyte card 876 and the message 8789 relating to the predefined normal threshold range (or a second analyte graph portion, if displayed), to displaying only the second analyte card 876 on the sensor results GUI 870. As such, the first collapsed view and the second expanded view are displayed on the sensor results GUI 870 at the same time.

The sensor results GUI 870 embodiment depicted in FIGS. 4G-1 to 4G-4 further comprises a graphical indication 8735 comprising a progress indicator that visually illustrates the remaining lifetime of the sensor 104 (e.g., a glucose-ketone sensor), wherein the progress indicator is used for a sensor 104 having a 14-day lifetime.

FIG. 4G-5 depicts an additional example embodiment of a sensor results GUIs for use with an analyte monitoring system. FIG. 4G-5 depicts a sensor results GUI 880, which is similar to the GUI embodiment depicted in FIGS. 4G-1 to 4G-4, except that sensor results GUI 880 is presented through a different mobile operating system (e.g., Android) than the embodiment shown inFIGS. 4G-1 to 4G-4(e.g., iOS). Specifically, FIG. 4G-5 depicts sensor results GUI 880 in its default view, wherein the first analyte section 882 is displayed in the first expanded view, and the second analyte section 885 is displayed in its second collapsed view, similar to FIGS. 4G-1 to 4G-3. Further, FIG. 4G-6 depicts a graphical indication 8835 comprising a progress indicator that visually illustrates the remaining lifetime of the sensor 104 (e.g., a glucose-ketone sensor), wherein the progress indicator is used for a sensor 104 having a 15-day lifetime.

FIGS. 4H-1 to 4H-3 depict an additional example embodiment of a sensor results GUI for use with an analyte monitoring system. Specifically, FIGS. 4H-1 to 4H-3 depict an exemplary embodiment of a sensor results GUI 4100 comprising a first analyte section 41022 reflecting data indicative of a high glucose level, and a second analyte section 41015 depicting data indicative of a normal ketone level. However, those of skill in the art will appreciate that the first analyte section 41022 reflecting data indicative of a high glucose level and the second analyte section 41015 depicting data indicative of a normal ketone level depicted in sensor results GUI 4100 is meant to be illustrative and non-exhaustive, and that sensor results GUI 4100 can be utilized to reflect data indicative of a first analyte level in various other conditions (e.g., normal glucose levels, low glucose levels, urgent low glucose level), and data indicative of a second analyte level in various other conditions (e.g., elevated ketone level, high ketone level), in various different combinations, as shown in exemplary embodiments depicted in FIGS. 4B-1 to 4G-5, without departing from the scope of the present disclosure.

With particular reference to FIGS. 4H-1 and 4H-2, a sensor results GUI 41000 is depicted which is similar to the sensor results GUI embodiment depicted in FIGS. 4B-1 to 4B-6, except that sensor results GUI 41000 comprises a second analyte section 41015 with a second analyte graph portion 41007 which can include a shaded area 41008 indicative of a high ketone threshold and a shaded area 41009 indicative of an elevated ketone threshold. More specifically, in some embodiments, the shaded area 41008 indicative of a high ketone threshold is a first color (e.g., red color) and the shaded area 41009 indicative of an elevated ketone threshold is a second color (e.g., yellow color) different from the first color. More specifically, in some embodiments, the shaded area 41008 indicative of a high ketone threshold covers an area of the second analyte graph portion 41007 corresponding to a high ketone level (e.g., 1.5 mmol/L and above), and the shaded area 41009 indicative of an elevated ketone threshold covers an area of the second analyte graph portion 41007 corresponding to an elevated ketone level (e.g., expanding across an area of the y-axis which covers between 0.5 mmol/L up to 1.5 mmol/L).

Further, in some embodiments, a first current analyte level value 41302 and a message 41189 relating to the predefined or normal threshold range can be positioned directly adjacent to and below a text description 41301 of a first analyte condition, as best depicted in FIGS. 4H-1 and 4H-3 (e.g., "You have Normal Ketones below 0.6 mmol/L").

Still with reference to FIGS. 4H-1 and 4H-2, the sensor analyte graph portion 41007 can further include a second analyte trend line 41025 which comprises a colored portion 41026 (e.g., green colored portion) configured to indicate a condition associated with the second current analyte data. For example, the colored portion 41026 can reflect when the user's analyte level is in a predetermined normal analyte range (e.g., normal ketone range). Specifically, according to some embodiments, when the user's analyte level is in the predetermined normal ketone range, the colored portion 41026 is displayed along the area of the second analyte trend line 41025 which corresponds to the period of time in which the user's analyte level is in the predetermined normal ketone range. In some embodiments, when the user's analyte level is in a predetermined normal analyte range (e.g., normal ketone range), the second analyte trend line 41025 comprises a colored portion 41026 and is configured as a flat line for the period of time corresponding to when the user's analyte level is within the predetermined normal ketone range. For example, if the user's analyte level is in the predetermined normal ketone range between 6PM and 9PM, then the second analyte trend line 41025 comprises the colored portion (e.g., green colored portion) 41026 and appears as a flat line across the x-axis from the 6PM to 9PM range.

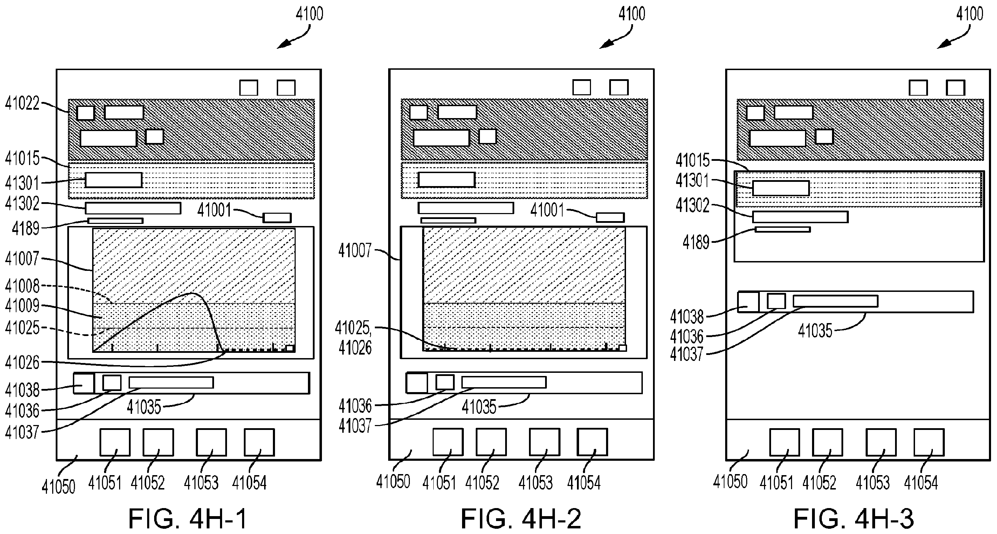

According to an aspect of the embodiments, the second analyte trend line 41025 can display the flat line with the colored portion 41026 to indicate the user's analyte level is in the predetermined normal analyte range only if an adverse state of condition is detected in association with the second historical analyte data and/or second current analyte data for a predefined period of time (e.g., within last 24-hours). For example, if within the last 24-hours, the user's ketone level exceeds a predetermined normal ketone range, then the second analyte trend line 41025 can appear with the flat line comprising the colored portion 41026 to indicate when the user's analyte level was within the predetermined normal analyte range for the predefined period of time. According to another aspect of the embodiment, and as depicted in FIG. 4H-3, if an adverse state of condition is not detected in association with the second historical analyte data and/or second current analyte data for a predefined period of time (e.g., within last 24-hours), then the second analyte section 41015 is displayed without the second analyte graph portion, similar to the sensor results interfaces depicted in FIGS. 4E-1 to 4G-5.

Referring to FIGS. 4H-1 and 4H-2, the second analyte graph portion 41007 comprises a selectable icon 41001 (e.g., switch, toggle, slider, check box, radio button, etc.) that allows the user to select or change the range of time over which the user's analyte data will be shown on the second analyte graph portion 41007. For example, the selectable icon 41001 can be used to select or change the range of time of three hours, six hours, or twelve hours. Those of skill in the art will appreciate that the selectable icon can be used to select various other ranges of predefined periods of time without departing from the scope of the present disclosure.

In exemplary embodiments, and as best depicted in FIG. 4H-2, if the user's ketone levels exceed a predetermined normal ketone range within a predefined period of time (e.g., within a 24-hour time period), but are still within the normal ketone range for the entire range of time over which the user's analyte data is shown on the second analyte graph portion 41007 (e.g., the second analyte graph portion 41007 is configured to display six hours of data), then the second analyte trend line 41025 can appear as a flat line comprising the colored portion 41026 to indicate when the user's analyte level was within the predetermined normal ketone range for the entire range of time (e.g., the second analyte graph portion 41007 can display the second analyte trend line 41025 as a flat line for the entire six hour period of time shown by the second analyte graph portion 41007).

With reference to FIGS. 4H-1 to 4H-3, the sensor results GUI 41000 can further comprise a graphical indication 41035 comprising a progress indicator that visually illustrates the remaining lifetime of the sensor 104 (e.g., a glucose-ketone sensor) (not shown in the sensor results GUI 41000 described herein). According to an aspect of the embodiments, the graphical indication 41035 can include numerical value 41036 corresponding to the number of days, hours, or minutes remaining in the lifetime of the sensor 104 (e.g., "14") and a textual message 41037 that indicates the units of measure (e.g., days, hours, minutes, or seconds) used to calculate the remaining lifetime of the sensor 104 (e.g., "Days until Sensor ends," "Hours until Sensor ends," "Minutes until Sensor ends," or "Seconds until Sensor ends"). For example, if the remaining lifetime of the sensor 104 is 13 days, then the numerical value 41036 (e.g., "13") and the textual message 41037 (e.g., "Days until Sensor ends") of the graphical indication 41035 can collectively indicate that there is "13 Days until Sensor ends." Additionally, in some embodiments, the graphical indication can further comprise a tab 41038, wherein the tab 41038 comprises a colored portion configured to reflect a status of the remaining lifetime of the sensor. For example, in some embodiments, if the remaining lifetime of the sensor exceeds one day, then the tab 41038 can include a green colored portion. Further, in some embodiments, if the remaining lifetime of the sensor is less than one day (e.g., if the remaining lifetime of the sensor is calculated in hours, minutes, or seconds), then the tab 41038 can include a red colored portion. Those of skill in the art will recognize that various other graphical indications, colors and textual messages can be utilized to visually illustrate the remaining lifetime of the sensor without departing from the scope of the present disclosure.

According to yet another aspect of the embodiments, and with reference to FIGS. 4H-1 to 4H-3, the sensor results GUI 41000 can further include a bottom bar navigation menu 41050. In some embodiments, the bottom bar navigation menu 41050 comprises a plurality of selectable icons. In some embodiments (though not illustrated), the bottom bar navigation menu 41050 comprises three selectable icons. In other embodiments, and as depicted inFIGS. 4H-1 to 4H-3 the bottom bar navigation menu 41050 comprises four selectable icons: (1) a selectable home icon 41051 which, upon being selected, outputs a home or sensor results interface, as described herein (e.g., FIGS. 4B-1 to 4H-3); (2) a selectable insights icon 41052 which, upon being selected, outputs interfaces comprising insights, reports, logbooks, daily summary data (e.g., FIGS. 9A-9F); (3) a selectable alarms icon 41053 which, upon being selected, outputs interfaces relating to alarms (e.g., FIG. 6A); and (4) a selectable profile icon 41054 which, upon being selected, outputs an interface related to the user's profile or account.

It will be understood by those of skill in the art that any of the sensor results GUIs (or portions thereof) described herein, are meant to be illustrative only, and that the individual elements, or any combination of elements, depicted and/or described for a particular embodiment or figure are freely combinable with any other element, or any combination of other elements, depicted and/or described with respect to any of the other embodiments.

### Example Embodiments of Alarm GUIs

Various example embodiments relating to alarming, alarm interfaces, alarm settings interfaces for analyte monitoring systems, and other related features will now be described. It will be understood by those of skill in the art that any one or more of the example embodiments of the methods, interfaces, and systems described herein can either be implemented independently, or in combination with any of the other embodiments described in the present application.

FIG. 5A depicts an example embodiment of a method 500 for determining one or more alarm conditions and presenting an alarm associated with the determined one or more alarm conditions. At Step 502, a current sensor reading is received. In some embodiments, the current sensor reading can comprise one or more signals from a sensor (e.g., glucose-ketone sensor) disposed in the sensor control device 102, wherein at least a portion of the sensor (e.g., glucose-ketone sensor) is configured to be positioned under the user's skin and in contact with a bodily fluid. In other embodiments, the sensor reading can be a glucose level measurement and a ketone level measurement received by reader device 120. In some embodiments, data is received from a single sensor control device 102 with a sensor capable of sensing both glucose and ketone. In some embodiments, data is received from a single sensor control device 102 comprising two or more discrete sensors 104, wherein a first sensor 104 is capable of sensing glucose and a second sensor 104 is capable of sensing ketone. In some embodiments, data is received from two or more sensor control devices 102, wherein a first sensor control device 102 comprises a sensor 104 capable of sensing ketone and a second sensor 104 control device 102 comprises a sensor 104 capable of sensing glucose. In some embodiments, the reader device 120 can be in direct communication with sensor control device 102. In other embodiments, reader device 120 can receive a glucose level measurement and a ketone level measurement via another computing device, such as, for example, a cloud-based server. Those of skill in the art will recognize various other sensor control device, sensor, and reader device configurations and combinations providing the aforementioned capabilities can be implemented, all of which are fully within the scope of the present disclosure.

At Step 504, a determination is made as to whether one or more alarm conditions are present. According to some embodiments, for example, the one or more alarm conditions can comprise at least one of: a low glucose condition, an urgent low glucose condition, a high glucose condition, an elevated ketone condition, a high ketone condition, amongst other alarm conditions. In some embodiments, the alarm condition can also comprise a signal loss condition, wherein a valid current glucose reading and/or current ketone reading has not been received within a predetermined amount of time (e.g., one minute, five minutes, ten minutes, twenty minutes, etc.). In some embodiments, the signal loss condition can be a result of a loss of wireless connectivity (e.g., Bluetooth connectivity) between reader device 120 and sensor control device 102. As stated earlier, the determination step can be performed by reader device 120, sensor control device 102, or any another computing device described with respect to analyte monitoring system 100.

Referring still to FIG. 5A, at Step 506, if it is determined that the one or more alarm conditions are present, an alarm associated with the determined alarm condition is presented. In some embodiments, the presentation of the alarm can comprise a visual notification (e.g., pop-up window, banner notification, full screen notification, etc.). In other embodiments, the presentation of the alarm can comprise a visual notification accompanied by an audible and/or vibratory indicator. In still other embodiments, the presentation of the alarm can comprise an audible and/or vibratory indicator, without a visual notification.

As stated earlier, it will be appreciated by those of skill in the art that the method steps described herein can be performed either by a single device or by multiple devices. For example, in some embodiments, the determination of the one or more alarm conditions can be performed by sensor control device 102 and the presentation of alarms can be performed by reader device 120. In other embodiments, both the determination of the alarm condition and the presentation of the alarm can be performed by reader device 120.

FIGS. 5B and 5C are example embodiments of GUIs comprising alarms for use with an analyte monitoring system. FIG. 5B, for example, is a GUI 510 depicting an alarm for an analyte monitoring system, wherein the alarm comprises an alarm condition text 512 (e.g., "High Glucose Alarm"), an analyte alarm message 513 (e.g., "Your glucose is high and rising"), an analyte level measurement 514 (e.g., a current glucose level of 256 mg/dL) associated with the alarm condition, and a trend indicator 515 (e.g., a trend arrow or directional arrow) associated with the alarm condition. Additionally, a time-of-alarm indicator 516 is also shown. In some embodiments, the time-of-alarm indicator 516 can indicate the amount of time elapsed since the alarm condition was triggered (e.g., now, 5 min ago, 10 min ago). In some embodiments, an alarm icon 518 can also be adjacent to the alarm condition text 512.

FIGS. 5C is a GUI 520 depicting another embodiment of a GUI comprising a high glucose alarm for an analyte monitoring system. According to one aspect of the embodiments, GUI 520 can be displayed when the user selects the high glucose alarm in previous GUI 510. In some embodiments, GUI 510 expands into GUI 520 in response to a received input by the user (e.g., by swiping or long-pressing a corresponding area of the touchscreen). Specifically, GUI 510 can expand into GUI 520 in response to a first predetermined input by the user, such as when the user, drags, long-press for some predetermined press time, or swipes with a finger, or by some other first predetermined gesture.

In one aspect of the embodiments, expanded GUI 520, for example, is similar to GUI 510 of FIG. 5B, except that expanded GUI 520 further includes: a first analyte card 5293 reflecting data indicative of a first analyte level (e.g., a glucose card reflecting data indicative of a glucose level), and a second analyte card 5296 reflecting data indicative of a second analyte level (e.g., a ketone card reflecting data indicative of a ketone level).

In another aspect of the embodiments, and in expanded GUI 520, for example, the first analyte card 5293 (e.g., the glucose card) can include: a text description 5201 of a first analyte condition (e.g., "High Glucose"), a first current analyte level value 5202 (e.g., a current glucose level of 256 mg/dL), and a first trend indicator 5203 (e.g., a directional trend arrow indicating the glucose level is rising). In yet another aspect of the embodiments, and in expanded GUI 520, for example, the second analyte card 5296 (e.g., the ketone card) can include: a text description 5211 of a second analyte condition (e.g., "Normal Ketones"), a second current analyte level value 5212 (not shown), and a second trend indicator 5213 (not shown) based on the data indicative of the second analyte condition.

In one aspect of the embodiments, though not illustrated, if an adverse state of condition is not detected, or if the first current analyte level value 5202 is considered to be in a "normal" or a predetermined analyte threshold range, the first current analyte level value 5202 and the first directional trend arrow 5203 will not be displayed. Similarly, if an adverse state of condition is not detected, or if the second current analyte level value 5212 (not shown) is considered to be in a "normal" or predetermined analyte range, the second current analyte level value 5212 (not shown) and a second directional trend arrow 5213 (not shown) will not be displayed. In expanded GUI 520, for example, the second analyte card 5296 is a ketone card, and the current ketone level is considered to be in a normal or predetermined threshold range. Thus, and as shown in FIG. 5C, the current ketone level value 5212 and the corresponding ketone directional trend arrow 5213 are not displayed on expanded GUI 520.

In another aspect of the embodiments, an alarm icon 5204 can also be adjacent to the text description 5201 of the first analyte card 5293 and/or the second analyte card 5296. For example, an alarm icon 5204 is depicted on the glucose card 5293 of expanded GUI 520. In some embodiments, the first analyte card 5293 includes a background color indicative of a condition or an analyte range of the first monitored analyte and the second analyte card 5296 includes a background color indicative of a condition or an analyte range of the second monitored analyte. In some embodiments, the colors corresponding to glucose are determined by the current glucose level of the user and the target glucose ranges that are set. In some embodiments, the colors corresponding to ketones are determined by the current ketone level of the used and the predefined ranges of the sensor 104 (not depicted in the alarm GUIs described herein). In some exemplary embodiments, expanded GUI 520 comprises a glucose card 5293 having an orange color indicating a high glucose level, and a ketone card 5296 having a green color indicating a ketone level within a normal or target range.

FIG. 5D is an example embodiment of a sensor results GUI comprising the data indicative of the first analyte level and the data indicative of the second analyte level associated with the alarm GUIs depicted in FIGS. 5B and 5C. Specifically, and according to one aspect of the embodiments, FIG. 5D depicts GUI 530, which can be displayed when the user selects the High Glucose Alarm in previous GUI 510 or expanded GUI 520. In some embodiments, GUI 530 is outputted in response to a second received input by the user (e.g., by selecting, tapping, or pressing a corresponding area of the touchscreen) on GUI 510 and/or expanded GUI 520. Specifically, in some embodiments, GUI 530 is outputted in response to a second received input or a second predetermined gesture. More specifically, in some embodiments, GUI 510 expands into GUI 530 in response to a second received input or a second predetermined gesture. Even more specifically, in some embodiments, the first received input is different than the second received input. Similarly, in some embodiments, the first predetermined gesture is different than the second predetermined gesture.

According to one aspect of the embodiments, GUI 530 includes: (1) a first analyte section 5392 comprising a first analyte card 5393 and/or a first analyte graph portion 5394 reflecting data indicative of a first analyte level (e.g., a glucose card and/or a glucose graph portion reflecting data indicative of a glucose level); and (2) a second analyte section 5395 comprising a second analyte card 5396 and/or a second analyte graph portion reflecting data indicative of a second analyte level (e.g., a ketone card and/or a ketone graph portion reflecting data indicative of a ketone level). Those of skill in the art will recognize that the sensor results GUI 530 depicted in FIG. 5D is similar to the sensor results GUIs described in FIGS. 4B-1 to 4G-6, and is fully within the scope of the present disclosure. For example, the first analyte section 5392 of GUI 530 is configured to transition between a first collapsed view and a first expanded view, and the second analyte section of GUI 530 is configured to transition between a second collapsed view and a second expanded view.

Specifically, in the first collapsed view, only the first analyte card 5393 is displayed in the first analyte section 5392 of GUI 530. As such, in the first collapsed view, the first analyte graph portion 5394 is not displayed in the first analyte section 5392 of GUI 530. In the first expanded view, however, the first analyte card 5393 and the first analyte graph portion 5394 are displayed on GUI 530. Similarly, in the second collapsed view 5356, only the second analyte card 5396 and not the second analyte graph portion is displayed in the second analyte section 5395 of GUI 530. In the second expanded view, however, the second analyte card 5396 and the second analyte graph portion are displayed on GUI 530.

In one aspect of the embodiments, the sensor results GUI 530 is configured to display the expanded view of the analyte section associated with the alarm triggered and selected by the user. Further, the sensor results GUI 530 can be further configured to display the collapsed view of the analyte section not associated with the alarm triggered and selected by the user. In the exemplary embodiment depicted in FIG. 5D, and in response to the user selecting the high glucose alarm in previous GUI 510 or expanded GUI 520, GUI 530 is configured to display the first expanded view of the glucose section 5392 (e.g., GUI 530 displays the glucose card 5392 and the glucose graph portion 5394). In some embodiments, and as depicted in FIG. 5D, expanded GUI 530 is further configured to display the second collapsed view of the ketone section 5395 (e.g., GUI 530 displays the ketone card of the ketone section). In this manner, GUI 530 predominantly displays information specific to the alarm triggered and previously selected by the user.

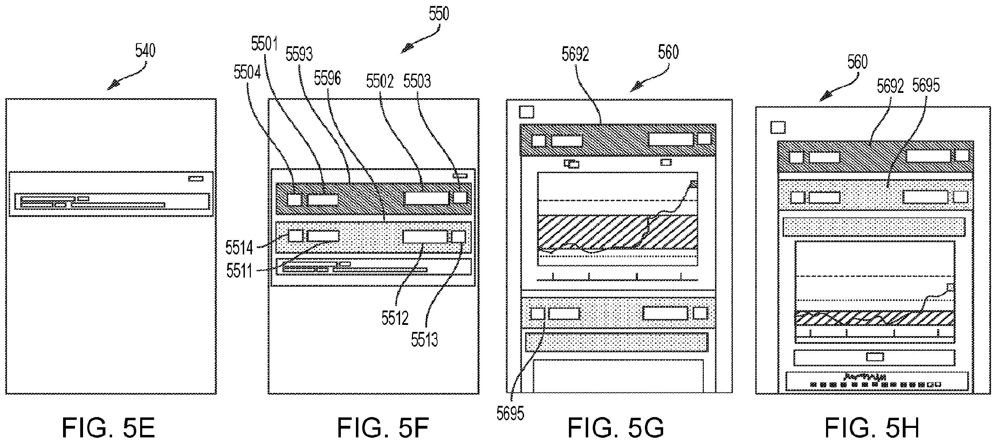

FIG. 5E and 5F are additional example embodiments of GUIs comprising alarms for use with an analyte monitoring system. FIG. 5E, for example, depicts GUI 540, which is similar to the GUI embodiment depicted in FIG. 5B, except that GUI 540 depicts an alarm comprising an elevated ketone alarm for an elevated ketone alarm condition. In some embodiments, though not illustrated, GUI 540 can further comprise a condition tag, wherein the condition tag comprises a textual description indicating that the alarm reflected in GUI 540 is critical (e.g., the condition tag can recite "critical"). Moreover, FIG. 5F depicts a GUI 550, which is similar to GUI 520 depicted in FIG. 5C, except that the alarm in GUI 550 comprises an elevated ketone alarm for an elevated ketone alarm condition. According to one aspect of the embodiments, and similarly to GUI 520, GUI 550 can be displayed when the user selects the elevated ketone alarm in previous GUI 540. In some embodiments, GUI 540 expands into GUI 550 in response to a first received input by the user (e.g., by swiping or long-pressing a corresponding area of the touchscreen). Specifically, GUI 540 can expand into GUI 550 in response to a first predetermined input by the user, such as when the user drags, long-presses for a predetermined press time, or swipes with a finger, or by some other first predetermined gesture.

In one aspect, expanded GUI 550 comprises the same alarm displayed in GUI 540 of FIG. 5D. However, expanded GUI 550 further includes: a first analyte card 5593 reflecting data indicative of a first analyte level (e.g., a glucose card reflecting data indicative of a glucose level), and a second analyte card 5596 reflecting data indicative of a second analyte level (e.g., a ketone card reflecting data indicative of a ketone level). Further, in expanded GUI 550, the first analyte card 5593 (e.g., the glucose card) includes: a text description 5501 of a first analyte condition (e.g., "High Glucose") having an alarm icon 5504 adjacent thereto, a first current analyte level value 5502 (e.g., a current glucose level of 298 mg/dL), and a first trend indicator 5503 (e.g., a directional trend arrow indicating the glucose level is rising). The second analyte card 5596 (e.g., the ketone card) of GUI 550 further includes: a text description 5511 of a second analyte condition (e.g., "Elevated Ketones") having an alarm icon 5514 adjacent thereto, a second current analyte level value 5512 (e.g., a current ketone level of 1.1 mmol/L), and a second trend indicator 5513 (e.g., a directional trend arrow indicating the ketone level is rising). Further, in some exemplary embodiments, expanded GUI 550 comprises a glucose card 5593 having an orange color indicating a high glucose level, and a ketone card 5596 having a yellow color indicating an elevated ketone level.

FIGS. 5G and 5H are additional exemplary embodiments of sensor results GUI comprising data indicative of the first analyte level and data indicative of the second analyte level associated with GUIs comprising alarms. Specifically, FIGS. 5G and 5F depict sensor results GUI 560 comprising data associated with the alarm GUIs depicted in FIGS. 5E and 5F. More specifically, FIGS. 5G and 5H depict a GUI 560 similar to the GUI embodiment depicted in FIG. 5D, except that FIGS. 5G and 5H depict GUI 560 which is outputted in response to a second received input by the user corresponding to the elevated ketone alarm depicted in FIGS. 5E or 5F. Similar to GUI 530, GUI 560 can be configured to display the expanded view of the respective analyte section associated with the selected alarm from previous GUI 540 or expanded GUI 550, and the collapsed view of the respective analyte section not associated with the selected alarm from previous GUI 540 or expanded GUI 550.

In some embodiments, the sensor results GUI can automatically transition from the collapsed view to the expanded view of the analyte section associated with the selected alarm, and from the expanded view to the collapsed view of the analyte section not associated with the selected alarm. As such, the sensor results GUI 560 is updated so as to display the collapsed view of the respective analyte section not associated with the selected alarm from the previous alarm GUI 540 or expanded GUI 550, and the expanded view of the respective analyte section associated with the selected alarm from the previous alarm GUI 540 or expanded GUI 550. In this manner, the sensor results GUI 560 obviates to the user which information is specific to the alarm previously selected and triggered.

For example, FIGS. 5G and 5H depict different configurations of GUI 560. Specifically, FIG. 5G depicts a default view of GUI 560, which is initially displayed in response to a second received input (e.g., by selecting, tapping, or pressing a corresponding area of the touchscreen) associated with the elevated ketone alarm on GUI 540 or expanded GUI 550. More specifically, FIG. 5H depicts GUI 560 after it has been updated from the configuration depicted in FIG. 5G. Even more specifically, an animation is automatically demonstrated to the user so as to show the transition from GUI 560, as depicted in FIG 5G, to GUI 560 as depicted in FIG. 5H.

With reference to FIG. 5G, GUI 560 initially displays the first expanded view of the glucose section 5692 (e.g., the glucose card and the glucose graph portion). Further, and as shown from FIG. 5G to FIG. 5H, GUI 560 transitions from displaying the first expanded view to the first collapsed view of the glucose section 5692 (e.g., the glucose section only displays the glucose card). In FIG. 5H, GUI 560 depicts the first analyte section 5692 in the first collapsed view and the second analyte section 5695 in the second expanded view. In addition, GUI 560 of FIGS. 5G and 5H display the second expanded view of the ketone section 5695 (e.g., GUI 560 displays the ketone card and ketone graph portion). Those of skill in the art will recognize that sensor results GUI 560 depicted in FIGS. 5G and 5H is similar to the sensor results GUIs described in FIGS. 4B-1 to 4G-6, and are fully within the scope of the present disclosure.

Although FIGS. 5B through 5H depict GUIs having high glucose alarms or elevated ketone alarms, or features related thereto, those of skill in the art will recognize that similar GUIs can be utilized for alarms for a low glucose alarm condition, an urgent low glucose alarm condition, a high ketone alarm condition, a signal loss alarm condition, amongst other alarm conditions, and are fully within the scope of the present disclosure.

Further, FIGS. 5C, 5D, and 5F through 5H, depict GUIs having a glucose card reflecting data indicative of a high glucose level. However, those of skill in the art will recognize that similar GUIs can be utilized comprising a glucose card reflecting data indicative of a normal glucose condition, a low glucose condition or an urgent low glucose condition, and are fully within the scope of the present disclosure. Similarly, FIGS. 5C, 5D, and 5F through 5H depict GUIs having a ketone card reflecting data indicative of a normal ketone condition or an elevated ketone condition. However, those of skill in the art will recognize that similar GUIs can be utilized comprising a ketone card reflecting data indicative of a high ketone condition, and are fully within the scope of the present disclosure. Those of skill in the art will further recognize that various combinations of the foregoing can be utilized in similar GUIs, and are fully within the scope of the present disclosure.

Example embodiments of urgent low glucose alarms and high ketone alarms will now be described. In a general sense, urgent low glucose alarms and high ketone alarms for analyte monitoring systems share certain similarities to the alarms previously described with respect to FIGS. 5B, 5C, 5E, and 5F. According to one aspect of the embodiments, urgent low glucose alarms will present an alarm to the user when his or her glucose level has fallen below an urgent low glucose threshold (e.g., below 55 mg/dL). According to another aspect of the embodiments, high ketone alarms will present an alarm to the user when his or her ketone level has risen above a high ketone threshold (e.g., above 1.5 mmol/L). According to another aspect of the embodiments, because of the critical nature of the user's condition, urgent low glucose alarms and high ketone alarms will override other settings of reader device 120, including the reader device's operating system, such as a "Mute" or "Do Not Disturb" setting. Further, in many embodiments, the glucose threshold levels which are typically adjustable for other alarms, cannot be changed for urgent low glucose alarms. Similarly, in many embodiments, the ketone threshold levels which are typically adjustable for other alarms, cannot be changed for high ketone alarms.

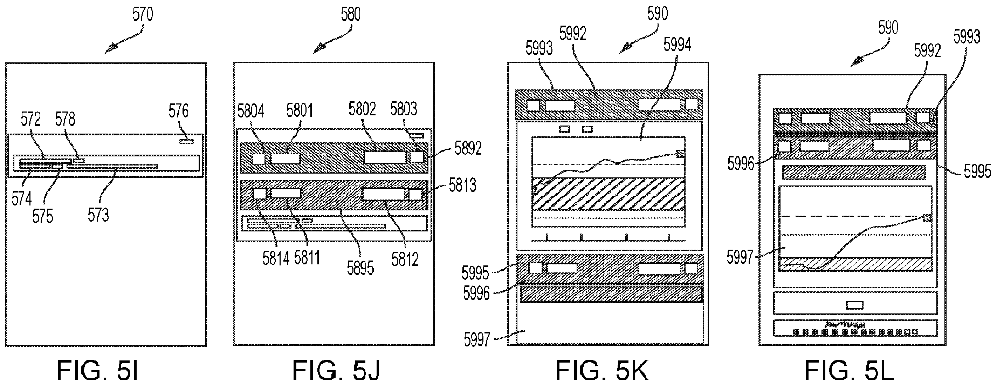

FIGS. 5I and 5J are example embodiments of GUIs comprising high ketone alarms for use in an analyte monitoring system. As stated above, these embodiments of GUIs have some similarities to the embodiments depicted in FIGS. 5B, 5C, 5E, and 5F. FIG. 5I, for example, has similarities to the GUI embodiments depicted in FIGS. 5B and 5E, and is a GUI 570 depicting high ketone alarm for an analyte monitoring system, wherein the alarm comprises a high ketone alarm condition text 572 having an alarm icon 578 adjacent thereto, an analyte alarm message 573 (e.g., "Your ketones are high and rising"), an analyte level measurement 574 (e.g., 2.1 mmol/L) associated with the alarm condition, and a trend indicator 575 (e.g., a trend arrow or directional arrow) associated with the alarm condition. Additionally, a time-of-alarm indicator 576 is also shown. In some embodiments, though not illustrated, GUI 570 can further comprise a condition tag, wherein the condition tag comprises a textual description indicating that the alarm reflected in GUI 570 is critical (e.g., the condition tag can recite "critical").

FIG. 5J is another GUI 580 depicting high ketone alarm for an analyte monitoring system. In this embodiment, the GUI is similar to embodiments previously described in FIGS. 5C and 5F. Specifically, GUI 580 is displayed in response to a first received input by the user (e.g., by swiping or long-pressing a corresponding area of the touchscreen). In some embodiments, GUI 570 expands into GUI 580 in response to a first predetermined input by the user, such as when the user drags, long-presses for a predetermined press time, or swipes with a finger, or by some other first predetermined gesture. Moreover, expanded GUI 580 comprises a high ketone alarm for a high ketone alarm condition, wherein the alarm depicts similarities to the alarm shown in FIG. 5I (e.g., an alarm condition text 582,"High Ketone Alarm"; an analyte alarm message 583, "Your Ketones are high and rising"; an analyte level measurement 584, a current ketone level of 2.1 mmol/L; a trend indicator 585 ; and a time-of-alarm indicator 586 associated with the alarm condition).

Expanded GUI 580 further depicts a glucose card section 5892 reflecting data indicative of a glucose level, wherein the data reflected on the glucose card section 5892 comprises a "High Glucose" text description 5801 having an alarm icon 5804, a current glucose level value 5802 of 320 mg/dL, and a glucose directional trend arrow 5803 indicating the glucose level is rising. Expanded GUI 580 further depicts a ketone card section 5895 reflecting data indicative of a ketone level, wherein the data reflected on the ketone card section 5895 comprises a "High Ketones" text description 5811 having an alarm icon 5814, a current ketone level 5812 of 2.1 mmol/L, and a ketone directional trend arrow 5813 indicating the ketone level is rising.

FIGS. 5K and 5L are additional example embodiments of sensor results GUI embodiments comprising data indicative of the first analyte level and data indicative of the second analyte level associated with the alarm GUIs depicted in FIGS. 5I and 5J. These embodiments of GUIs have some similarities to the embodiments depicted in FIGS. 5D, 5G, and 5H. Specifically, FIGS. 5K and 5L depict a GUI similar to the GUI embodiment depicted in FIGS. 5D, 5G, and 5H, except that FIGS. 5K and 5L depict a GUI 590 which is outputted in response to a received input by the user corresponding to the high ketone alarm depicted in FIGS. 5I or 5J.

Similar to GUI 530 or GUI 560, GUI 590 can be configured to display the expanded view of the respective analyte section associated with the selected alarm from previous GUI 570 or expanded GUI 580, and the collapsed view of the respective analyte section not associated with the selected alarm from previous GUI 570 or expanded GUI 580. Moreover, GUI 590 transitions between varying configurations of the sensor results GUI, similar to that which was previously described with respect to GUI 560.

For example, FIGS. 5K and 5L depict different configurations of GUI 590. Specifically, FIG. 5K depicts a default view of GUI 590, which is initially displayed in response to a second received input (e.g., by selecting, tapping, or pressing a corresponding area of the touchscreen) associated with the high ketone alarm on GUI 570 or expanded GUI 580. More specifically, FIG. 5L depicts GUI 590 after it has been transitioned from the configuration shown in FIG. 5G. Even more specifically, an animation is automatically demonstrated to the user so as to show the transition from GUI 590, as depicted in FIG 5K, to GUI 590 as depicted in FIG. 5L.

With reference to FIG. 5K, GUI 590 initially displays the first expanded view of the glucose section 5992 (e.g., the glucose card 5993 and the glucose graph portion 5994). Further, and as depicted through FIGS. 5K and 5L, GUI 590 transitions from displaying the first expanded view (FIG. 5K) to the first collapsed view (FIG. 5L) of the glucose section 5992 (e.g., the glucose section 5882 only displays the glucose card 5993). In addition, GUI 590 displays the second expanded view of ketone section 5995 (e.g., the ketone card 5996 and the ketone graph portion 5997) in both configurations depicted in FIGS. 5K and 5L. Those of skill in the art will recognize that the sensor results GUI depicted in FIGS. 5K and 5L are similar to the sensor results GUIs described in FIGS. 4B-1 to 4G-6, and are fully within the scope of the present disclosure.

Although FIGS. 5I through 5L depict GUIs having high ketone alarms, or features related thereto, those of skill in the art will recognize that similar GUIs can be utilized for non-configurable alarm settings for an urgent low glucose alarm, and are fully within the scope of the present disclosure.

According to an aspect of many embodiments, the GUIs comprising alarms for use with an analyte monitoring system, and described herein, can include two alarms. For example, in some embodiments, though not illustrated, the GUIs can comprise: (1) an urgent low glucose alarm for an urgent low glucose alarm condition, a low glucose alarm for a low glucose alarm condition, or a high glucose alarm for a high glucose alarm condition; and (2) an elevated ketone alarm for an elevated ketone condition or a high ketone alarm for a high ketone condition.

In some embodiments (not illustrated), if a first alarm condition has a higher priority than a second alarm condition and both occur simultaneously, the analyte monitoring system will display the first alarm associated with the first alarm condition. In some embodiments, the alarm GUIs described herein can further include an alarm priority setting, wherein the priority of one or more alarm conditions can be configurable and/or non-configurable. In some exemplary embodiments (not shown), multiple alarm conditions can be present. In these embodiments, if one alarm condition of the multiple alarm conditions is determined to have higher priority than the other alarm conditions, the first alarm associated with the first alarm condition is displayed on the alarm GUI and cannot be dismissed. Further, in some embodiments, a first alarm condition having greater priority than a second alarm condition will present a first alarm notification that will overlap over the second alarm notification so as to prevent the first alarm notification from being obscured until manually dismissed by the user. In some alarm GUI embodiments, once a second predetermined gesture is received, and a sensor results GUI corresponding to a second alarm condition is displayed, an animation is automatically demonstrated to the user so as to show the transition from the sensor results GUI corresponding to the second alarm condition to the sensor results GUI corresponding to the first alarm condition having or becoming a higher priority.

As previously described, a GUI comprising an alarm for an analyte monitoring system can expand into an expanded GUI comprising an alarm for an analyte monitoring system. In these embodiments, and as previously detailed, the expanded GUI includes an alarm, a first analyte reflecting data indicative of a first analyte level (e.g., a glucose card reflecting data indicative of a glucose level), and a second analyte card reflecting data indicative of a second analyte level (e.g., a ketone card reflecting data indicative of a ketone level). Though not illustrated, in some embodiments, when the alarm is associated with data indicative of the first analyte level, the expanded GUI can further include a trend line associated with the data indicative of the first analyte level. In like manner, in some embodiments, when the alarm is associated with data indicative of the second analyte level, the expanded GUI can further include a trend line associated with the data indicative of the second analyte level.

According to another aspect of these embodiments, the alarms described herein can include both alarms with configurable settings (e.g., low glucose alarm, high glucose alarm, signal loss alarm) and alarms with non-configurable settings (e.g., urgent low glucose alarm, high ketone alarm) operating on a single computing device within the same analyte monitoring system. In some embodiments, for example, an analyte monitoring system can include a reader device 120 comprising wireless communication circuitry configured to receive data indicative of an analyte level from a sensor control device 102, and one or more processors coupled with a memory, the memory storing instructions that, when executed by the one or more processors, cause the one or more processors to: (1) determine whether the data indicative of the analyte level meets one or more alarm conditions, wherein the one or more alarm conditions comprises a first alarm condition associated with a first set of alarms settings that are configurable by the user, and a second alarm condition associated with a second set of alarm settings that are not configurable by the user, and wherein the second alarm condition is an urgent low glucose alarm condition or a high ketone alarm condition; and (2) in response to a determination that at least one of the one or more alarm conditions is met, present an alarm associated with the at least one of the one or more alarm conditions.

FIGS. 6A to 6R are example embodiments of GUIs including alarm settings for alarms in an analyte monitoring system. FIG. 6A is a GUI 600 depicting an alarms settings interface comprising a plurality of selectable alarm options. Specifically, GUI 600 comprises three selectable glucose alarm options (an urgent low glucose alarm option 602, a low glucose alarm option 604, and a high glucose alarm option 606) and three selectable ketone alarm options (an elevated ketone alarm option 603, and a high ketone alarm option 605). In some embodiments, GUI 600 can further include one or more selectable other alarms options. For example, in the embodiment depicted in FIG. 6A, the alarm settings interface comprises one selectable other alarm options. Specifically, the alarm settings interface comprises a signal loss alarm option 607. Adjacent to each selectable alarm option is a textual indicator to indicate whether the alarm is on or off. Additionally, in some embodiments, a selectable "Learn More" option 608 for additional information is provided beneath the selectable alarms.

In some embodiments, though not illustrated in FIG. 6A, GUI 600 can further comprise a Silent Mode feature. In many embodiments, though not illustrated, the Silent Mode feature includes a switch that can be toggled between an "on" state and an "off" state. Upon toggling switch to the "on" state, a configuration modal (not shown) is displayed to the user. The user can specify the amount of time for Silent Mode to be enabled. In some embodiments, though not illustrated, configuration modal can include a "Save" button, which will save the user's selection in terms of the configurable duration of Silent Mode. Upon actuating "Save" button, the user can be prompted with another confirmation modal (not depicted), which indicates to the user that "all glucose and signal loss alarms will be silenced" for the selected amount of time. In many embodiments, the confirmation modal can include a "Turn On" option to proceed with enabling Silent Mode, and/or a "Cancel" option, in case the user does not want to proceed with Silent Mode.

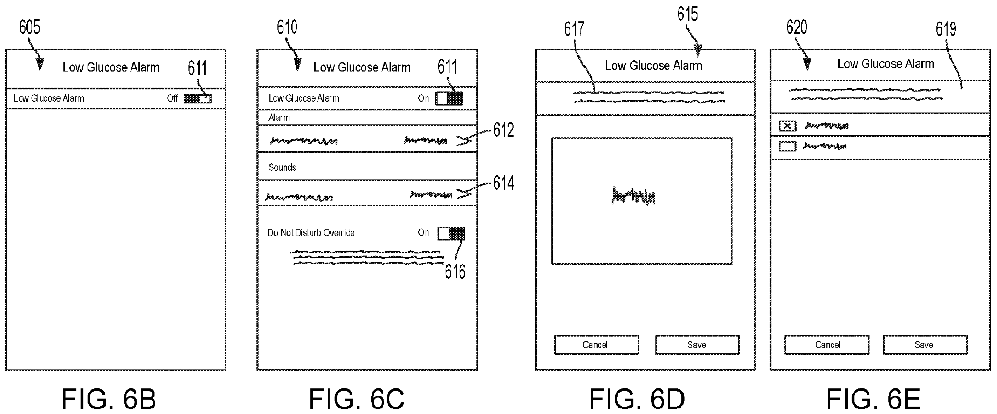

FIG. 6B is a GUI 605 depicting a low glucose alarm settings interface. According to one aspect of the embodiments, GUI 605 can be displayed when the user selects the low glucose alarm in previous GUI 600. According to another aspect of the embodiments, GUI 605 includes a textual label for the "Low Glucose Alarm" adjacent to a switch 611 configured to be toggled between an on position and an off position. Those of skill in the art will understand that, instead of a toggle, GUI 605 can include any one or more of: an on-off checkbox, on-off slider switch, on-off radio buttons, on-off buttons, and the like.

FIG. 6C is a GUI 610 depicting the low glucose alarm setting interface after the switch 611 has been toggled to the on position. After switch 611 has been toggled to the on position, as can be seen in FIG. 6C, GUI 610 can include a plurality of configurable settings, including (but not limited to): the textual label for the "Low Glucose Alarm" adjacent to the switch 611 that is configured to be toggled between an on position and an off position, a low glucose alarm threshold setting 612, a low glucose alarm tone setting 614, and a low glucose alarm override setting 616. According to one aspect of the embodiments, the low glucose alarm threshold setting 612 can be configured by the user such that the user can select a low glucose threshold (as shown in GUI 615 of FIG. 6D), where a low glucose alarm will be triggered when the user's glucose level falls below the selected low glucose threshold (e.g., below 70 mg/dL). In some embodiments, GUI 615 can include an informational section 617 that instructs the user that they will receive a low glucose alarm when the user's glucose is lower than the selected value. According to another aspect of the embodiments, the low glucose alarm tone setting 614 can be configurable by the user such that the user can select a standard alarm (e.g., adopting the operating system's alarm tone), as shown in GUI 620 of FIG. 6E, or a custom low glucose alarm (e.g., allowing the user to select a specific tone or output). GUI 620 can further include an informational section 619 that indicates that the user can choose the alarm sound, and that alarms are configured to follow the volume and vibrational settings of the user's phone. According to another aspect of the embodiments, the low glucose alarm override setting 616 can be configured by the user such that, when enabled, a low glucose alarm will always output a sound (or vibration), and display a visual notification on the display of reader device 120 (e.g., on a lock screen), even if reader device 120 is muted or configured in a "Do Not Disturb" mode.

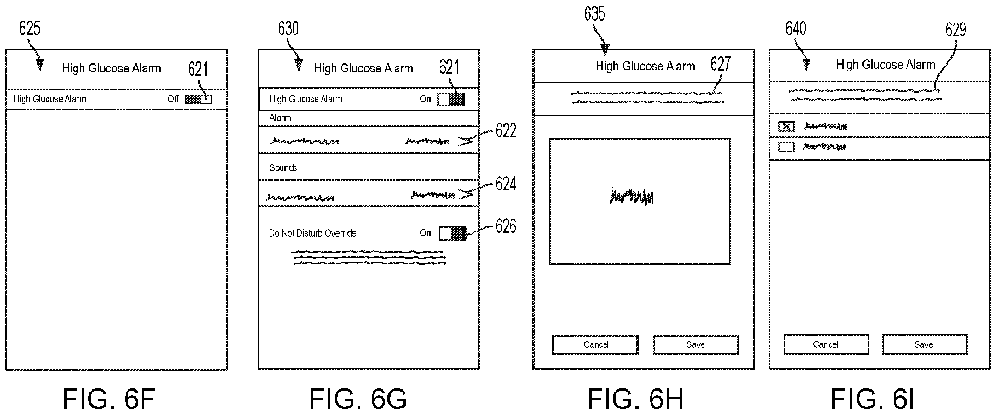

FIGS. 6F to 6I are additional example embodiments of GUIs including alarm settings for alarms in an analyte monitoring system. FIG. 6F is a GUI 625 depicting a high glucose alarm settings interface. According to one aspect of the embodiments, GUI 625 can be displayed when the user selects the high glucose alarm in previous GUI 600. According to another aspect of the embodiments, GUI 625 includes a textual label for the "High Glucose Alarm" adjacent to a switch 621 configured to be toggled between an on position and an off position. Those of skill in the art will understand that, instead of a toggle, GUI 625 can include any one or more of: an on-off checkbox, on-off slider switch, on-off radio buttons, on-off buttons, and the like.

FIG. 6G is a GUI 630 depicting a High Glucose Alarm settings interface after the switch 621 has been toggled to the on position. After switch 621 has been toggled to the on position, as can be seen in FIG. 6G, and like GUI 610 of FIG. 6C, GUI 630 can include a plurality of configurable settings, including (but not limited to): the textual label for "High Glucose Alarm" adjacent to a switch 621 that is configured to be toggled between an on position and an off position, a configurable high glucose alarm threshold setting 622, a configurable high glucose alarm tone setting 624, and a high glucose alarm override setting 626. According to one aspect of the embodiments, the high glucose alarm threshold setting 622 can be configured by the user such that the user can select a high glucose threshold (as shown in GUI 635 of FIG. 6H), where a high glucose alarm will be triggered when the user's glucose level rises above the selected high glucose threshold (e.g., above 240 mg/dL). In some embodiments, GUI 635 can include an informational section 627 that instructs the user that they will receive a low glucose alarm when the user's glucose is lower than the selected value. According to another aspect of the embodiments, the high glucose alarm tone setting 624 can be configurable by the user such that the user can select a standard alarm (e.g., adopting the operating system's alarm tone), as shown in GUI 640 of FIG. 6I, or a custom low glucose alarm (e.g., allowing the user to select a specific tone or output). GUI 640 can further include an informational section 629 that indicates that the user can choose the alarm sound, and that alarms are configured to follow the volume and vibrational settings of the user's phone.

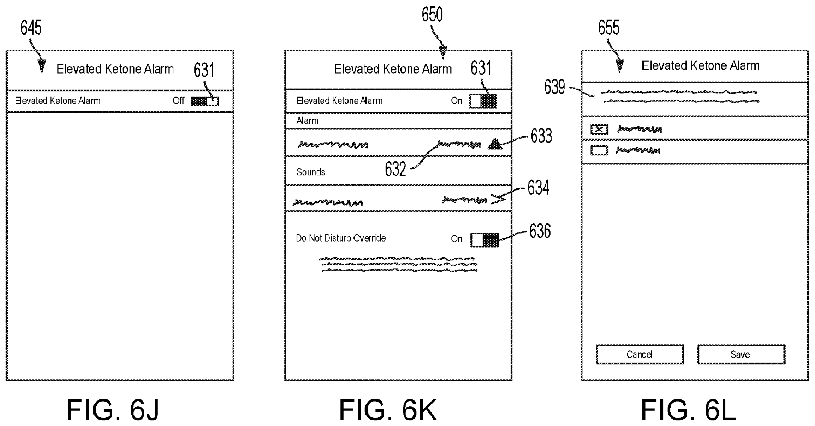

FIGS. 6J to 6L are additional example embodiments of GUIs including alarm settings for alarms in an analyte monitoring system. FIG. 6J is a GUI 645 depicting an elevated ketone alarm settings interface. According to one aspect of the embodiments, GUI 645 can be displayed when the user selects the elevated ketone alarm in previous GUI 600. According to another aspect of the embodiments, GUI 645 includes a textual label for the "Elevated Ketone Alarm" adjacent to a switch 63 1 configured to be toggled between an on position and an off position. Those of skill in the art will understand that, instead of a toggle, GUI 645 can include any one or more of: an on-off checkbox, on-off slider switch, on-off radio buttons, on-off buttons, and the like.

FIG. 6K is a GUI 650 depicting an Elevated Ketone Alarm settings interface after the switch 631 has been toggled to the on position. After switch 631 has been toggled to the on position, as can be seen in FIG. 6K, and similar to GUI 610 and GUI 630, GUI 630 can include a plurality of configurable settings, including (but not limited to): a textual label for "Elevated Ketone Glucose Alarm" adjacent to a switch 631 that is configured to be toggled between an on position and an off position, a configurable elevated ketone alarm tone setting 634, and an elevated ketone alarm override setting 636. According to another aspect of the embodiments, GUI 650 includes an elevated ketone alarm threshold setting 632. In some embodiments, the elevated ketone alarm threshold setting 632 includes a lock icon 633 indicating that the elevated ketone alarm threshold setting 632 is locked such that the user cannot select an elevated ketone threshold. Further, and according to one aspect of the embodiments, the elevated ketone alarm threshold setting 632 is locked such that it will be triggered when the user's ketone level goes above a particular ketone threshold (e.g., above 1.0 mmol/L). According to yet another aspect of the embodiments, the elevated ketone alarm tone setting 634 can be configurable by the user such that the user can select a standard alarm (e.g., adopting the operating system's alarm tone), as shown in GUI 655 of FIG. 6L, or a custom elevated ketone alarm (e.g., allowing the user to select a specific tone or output). In some embodiments, GUI 655 can further include an informational section 639 that indicates that the user can choose the alarm sound, and that alarms are configured to follow the volume and vibrational settings of the user's phone.

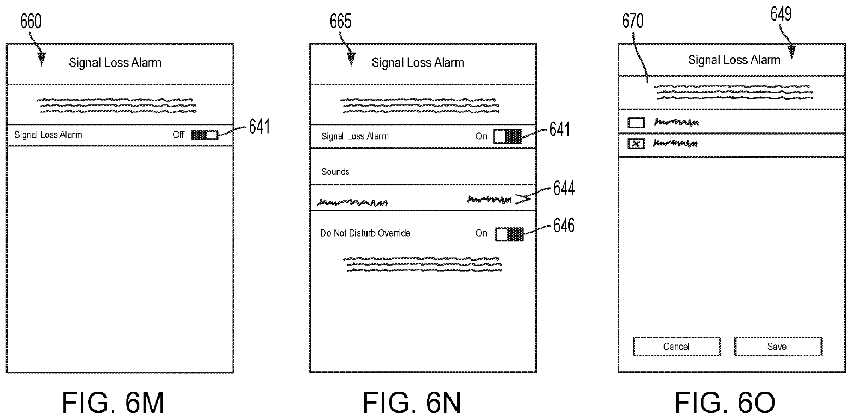

FIG. 6M to 60 depict additional example embodiments of GUIs including alarm settings for alarms in an analyte monitoring system. FIG. 6M is a GUI 660 depicting a signal loss alarm settings interface. According to one aspect of the embodiments, GUI 660 can be displayed when the user selects the signal loss alarm in previous GUI 600. According to another aspect of the embodiments, GUI 660 includes a textual label for the "Signal Loss Alarm" adjacent to a switch 641 configured to be toggled between an on position and an off position. Those of skill in the art will understand that, instead of a toggle, GUI 660 can include any one or more of: an on-off checkbox, on-off slider switch, on-off radio buttons, on-off buttons, and the like.

FIG. 6N is a GUI 665 depicting a signal loss alarm settings interface after the switch 641 has been toggled to the on position. In addition, like the GUIs depicts in FIGS. 6C, 6G, and 6K, GUI 665 can include (but is not limited to): the textual label for "Signal Loss Alarm" adjacent to a switch 641 that is configured to be toggled between an on position and an off position, a configurable signal loss alarm tone setting 644, and a signal loss alarm override setting 646. According to yet another aspect of the embodiments, the signal loss alarm tone setting 644 can be configurable by the user such that the user can select a standard alarm (e.g., adopting the operating system's alarm tone), as shown in GUI 670 of FIG. 6O, or a custom signal loss alarm (e.g., allowing the user to select a specific tone or output). According to another aspect of the embodiments, the GUIs depicted in FIGS. 6M to 60 can each include an informational section 649 that indicates that the signal loss alarm is received when the "glucose and ketones alarms are not available because the Sensor is not communicating with the app."

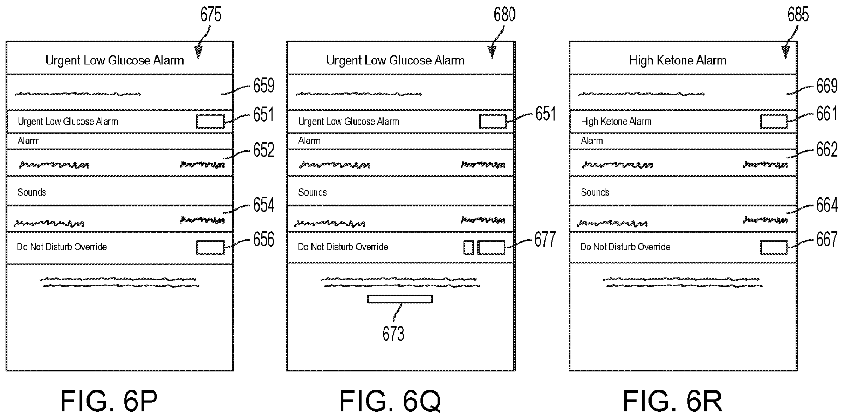

FIGS. 6P to 6R are additional example embodiments of GUIs including alarm settings for alarms in an analyte monitoring system. FIG. 6P is a GUI 675 depicting an urgent low glucose alarm settings interface. According to one aspect of the embodiments, GUI 675 can be displayed when the user selects the urgent low glucose alarm in previous GUI 600. According to another aspect of the embodiments, GUI 675 includes an informational section 659 that indicates that the Urgent Low Glucose Alarm "is ON and cannot be modified." In addition, like the GUIs depicted in FIGS. 6C, 6G, 6K, and 6N, GUI 675 further includes (but is not limited to): a textual label for "Urgent Low Glucose Alarm" adjacent to a switch 651, an urgent low glucose alarm threshold setting 652, an urgent low glucose alarm override setting 656, and an urgent low glucose alarm tone setting 654. As shown in FIG. 6P, the urgent low glucose alarm tone setting 654 has been configured to output a custom urgent low glucose alarm.

FIG. 6Q is a GUI 680 depicting an urgent low Glucose Alarm settings interface, similar to GUI 675 of FIG. 6P. As seen at the bottom third of the interface, the urgent low glucose alarm override setting 677 is shown in an "Off" state. In addition, a critical alert icon or badge is displayed adjacent to the "Off" state, indicating that corrective action is needed. According to one aspect of some embodiments, GUI 680 presents an active "Open Settings" link 673 near the bottom of the interface. Upon selection of link 673, a notification settings interface is configured to be displayed (not shown).

FIG. 6R is a GUI 685 depicting a high ketone alarm settings interface. According to one aspect of the embodiments, GUI 685 can be displayed when the user selects the high ketone alarm in previous GUI 600. According to another aspect of the embodiments, GUI 685 includes an informational section 669 that indicates that the High Ketone Alarm "is ON and cannot be modified." In addition, like the GUIs depicted in FIGS. 6C, 6G, 6K, 6N, and 6P, GUI 685, GUI 685 further includes: a textual label for "High Ketone Alarm" adjacent to a switch 661, a high ketone alarm threshold setting 662, a high ketone alarm override setting 667, and a high ketone alarm tone setting 664. As shown in FIG. 6R, the high ketone alarm tone setting 664 has been configured to output a custom high ketone alarm.

In many embodiments, the aforementioned settings are not configurable to the user, and displayed for informational purposes only. For example, unlike switches 611, 621, 631, and 641 in GUIs 610, 630, 650, and 665 respectively, (FIGS. 6C, 6G, 6K, and 6N), switches 651 and 661 in GUIs 670 and 685, respectively, (FIGS. 6P and 6R), cannot be toggled to an "Off" position or state. Similarly, according to another aspect of some embodiments, the urgent low glucose alarm threshold setting 652, the urgent low glucose alarm tone setting 654, and the urgent low glucose alarm override setting 656 cannot be modified or disabled. In like manner, according to another aspect of some embodiments, the high ketone alarm threshold setting 662, high ketone alarm tone setting 664, and the high ketone alarm override setting 667 cannot be modified or disabled.

In other embodiments, one or more of the aforementioned settings can be configurable by the user, while the rest of the settings are displayed for information purposes only. For example, in certain embodiments, the textual label and switch 651, alarm threshold setting 652, and alarm override setting 656 can be non-configurable, while the alarm tone setting 654 can be configurable so as to allow the user to select a specific tone or vibration. Similarly, in certain embodiments, the textual label and switch 661, alarm threshold setting 662, and alarm override setting 667 can be non-configurable, while the alarm tone setting 664 can be configurable so as to allow the user to select a specific tone or vibration. Other combinations of configurable and non-configurable settings are possible, and those of skill in the art will recognize that these combinations are fully within the scope of the present disclosure.

### Example Embodiments of Alarm Unavailability Features and GUIs

Example embodiments of methods, systems, and related GUIs for detecting the unavailability of alarms in an analyte monitoring system will now be described.

As previously described with respect to FIGS. 6C, 6G, 6K, and 6N, certain alarms can require that the user configure specific operating system features that can interfere with the alarms. For example, some operating systems for mobile computing devices include such features as "Do Not Disturb" or "Mute" that can interfere with the audible, visual, or vibratory alarm presentation of the aforementioned alarms. In addition, users can unknowingly take certain actions with their analyte monitoring system that can also interfere with alarms. Thus, needs exist for robust methods and system for detecting the unavailability of alarms in an analyte monitoring system.

FIG. 7A depicts an example embodiment of a method for determining an alarm unavailability condition is present in an analyte monitoring system. At Step 702, one or more alarms are enabled in an analyte monitoring system. In many embodiments, the one or more alarms can comprise a low glucose alarm, an urgent low glucose alarm, a high glucose alarm, an elevated ketone alarm, a high ketone alarm, and/or a signal loss alarm, amongst other examples, that can be enabled on one or more of: a reader device 120, a sensor control device 102, or any other computing device that is a part of, or in communication with, the analyte monitoring system.

At Step 704, a determination is made as to whether one or more alarm unavailability conditions are present. According to one aspect of the embodiments, alarm unavailability conditions can comprise any one or more of the following conditions: the wireless communication circuitry (e.g., Bluetooth or Bluetooth Low Energy) is disabled and/or not functioning, systemwide notifications are disabled, application-specific notifications are disabled, a mute or silent feature is enabled, the analyte monitoring application has been force-closed by the user or by the system (i.e., no longer running in the background or foreground), critical alerts are disabled, the "Override Do Not Disturb" feature is disabled, the "Do Not Disturb" channel is turned off; and/or alarm tone(s) are set to silent. In some embodiments, other alarm unavailability conditions can further include: no active sensor detected or sensor fault conditions (e.g., temperature too high, temperature too low, sensor not communicating with reader device 120 or application, no active sensor detected, and/or a signal loss condition existing and a sensor needs to be scanned). Those of skill in the art will recognize that these aforementioned alarm unavailability conditions are meant to be illustrative only, and do not represent an exhaustive list of all alarm unavailability conditions. Other conditions relating to either the sensor, sensor control device 102, or reader device 120 that can cause interference with either: (1) the determination of alarm conditions, or (2) the presentation of alarms in the analyte monitoring system are possible and are fully within the scope of the present disclosure.

Referring again to FIG. 7A, if no alarm unavailability conditions are detected, then method 700 returns to Step 702 and continues to monitor for alarm unavailability conditions (as long as at least one alarm is enabled). However, if one or more alarm unavailability conditions are detected, then, at Step 706, one or more notifications associated with the detected one or more alarms unavailability conditions are presented to the user.

FIG. 7B depicts an example embodiment of an alarms unavailability interface in an analyte monitoring system. FIG. 7B is a GUI 710 depicting an alarms setup interface providing instructions 715 to the user that a different sensor has been applied, and that no ketone alarms will be received because the sensor does not support ketone alarms. In some embodiments, GUI 710 further includes a confirmation "Got It" button 711 that can be pressed by the user.

According to another aspect of the embodiments, the one or more notifications associated with the detected one or more alarm unavailability conditions can comprise modal windows, as seen in the GUIs depicted in FIGS. 7C to 7L. In some embodiments, the modal can provide information regarding the specific cause of the alarm unavailability condition, along with a confirmation ("OK") button, as seen in GUI 720 depicted in FIG. 7C (sounds and vibration off). In some embodiments (not illustrated), the modal can provide a number of possible reasons for the alarm unavailability condition along with a confirmation ("OK") button.

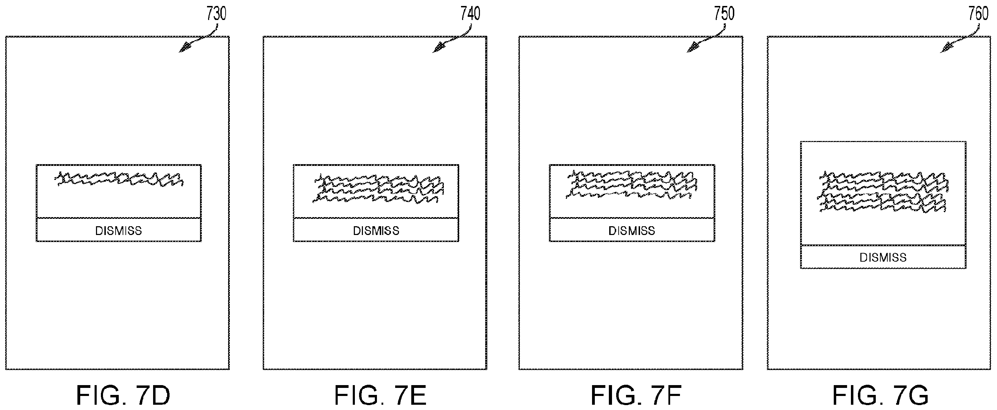

In other embodiments, the modal can provide information regarding the specific cause of the alarm unavailability condition, along with a "Dismiss" button, as depicted in GUI 730 (signal loss), GUI 740 (no active sensor), and GUI 750 (Bluetooth disabled), of FIGS. 7D, 7E, and 7F, respectively. In some embodiments, the modal can provide a number of possible reasons for the alarm unavailability condition along with a "Dismiss" button, as seen in FIG. 7G (GUI 760).

In yet other embodiments, the modal can provide information regarding the specific cause of the alarm unavailability condition, along with a "Settings" button that opens up the corresponding settings interface to allow the user to correct the condition, as depicted in GUI 770 (notifications disabled) and GUI 780 (critical alerts disabled, Do Not Disturb mode turned on) of FIGS. 7H and 7I, respectively. In some embodiments, the modal can provide a number of possible reasons for the alarm unavailability condition along with a "Settings" button that opens up the corresponding settings interface to allow the user to correct the condition, as depicted in GUI 790 (FIG. J). In other embodiments, the modal can provide information regarding the specific cause of the alarm unavailability, along with a "Dismiss" button, and a "Settings" button that opens up the corresponding settings interface to allow the user to correct the condition, as depicted in FIG. 7K (GUI 795). These examples are meant to be illustrative only, and those of skill in the art will recognize that other combinations and permutations of modals can be implemented and are fully within the scope of the present disclosure.

According to another aspect of the embodiments, the one or more notifications associated with the detected one or more alarm unavailability conditions can comprise an in-app notification within an analyte monitoring application, as seen in GUI 725, GUI 755, and GUI 775 (FIGS. 7L to 7N). In some embodiments, the notification associated with the alarm unavailability condition can be presented as a modal window, similar to the embodiments previously described herein. For example, in FIG. 7L, GUI 725 presents a modal providing information regarding the specific cause of the alarm unavailability condition, along with a "Dismiss" button. In this regard, GUI 725 is similar to the GUIs depicted FIGS. 7D to 7F, except that the modal in GUI 725 is presented as an in-app modal notification 727. Further, in the exemplary embodiment depicted in FIG. 7M, GUI 755 comprises an in-app modal notification 757 providing a number of possible reasons for the alarm unavailability condition along with a "Dismiss" button, and a "Settings" button that opens up the corresponding settings interface to allow the user to correct the condition. Moreover, in-app modal notification 727 and in-app modal notification 757 can be configured so as to partially obstruct or superimpose the underlying interface from view. In this regard, in-app modal notification 727 and in-app modal notification 757 impede the user's ability to fully view the underlying interfaces. Further, those of skill in the art will recognize that other combinations and permutations of modals can be implemented as in-app modal notifications, and are fully within the scope of the present disclosure.

In other embodiments, the notification associated with the alarm unavailability condition can be presented as an in-app banner notification 777 positioned on a same interface as an alarms settings interface, as seen in FIG. 7N. For example, the in-app banner notification 777 can be presented on GUI 600 comprising the plurality of selectable alarm options.

Although not shown, in some embodiments, the in-app banner notification 777 can persist through different interfaces within the analyte monitoring application (e.g., reports, logbook, etc.). In this regard, the in-app banner notification 777 allows for the user to continue to review recent and historical analyte data, as well as reports. Further, according to one aspect of the embodiments, the one or more notifications associated with the detected one or more alarms unavailability conditions can comprise banner notifications or pop-up windows displayed to the user outside of the analyte monitoring application (e.g., on a lock screen), as seen in GUI 705 (FIG. 70)

Although the above descriptions of the figures and embodiments refer to alarms and alarm interfaces for a reader device, those of skill in the art will appreciate that these alarms and alarm interfaces can also be implemented in a sensor control device, a local computing system, a trusted computing system, or any other computing device within, or in communication with, an analyte monitoring system. Moreover, as described earlier, any of the GUIs and features described herein can comprise instructions stored in memory of a reader device, sensor control device, or any other computing device that is part of, or in communication with, the analyte monitoring system.

### Example Embodiments of Onboarding GUIs and Features Related Thereto

Example embodiments of methods, systems, and related GUIs for onboarding in an analyte monitoring system will now be described. FIGS. 8A through 8I are block diagrams depicting example embodiments of onboarding interfaces, or features related thereto, any of which can be utilized with the embodiments described herein. According to one aspect of the embodiments, an onboarding GUI can be displayed when the user launches the analyte monitoring application. In some embodiments, an onboarding GUI is displayed during a sensor warm-up period (e.g., a 60-minute glucose-ketone sensor warm-up period). In some embodiments, an onboarding GUI is displayed when the user is using the glucose-ketone sensor for a first time or switching from a glucose sensor to a glucose-ketone sensor. In some embodiments, during the sensor warm-up period, onboarding GUIs can provide the user with a brief introduction to the analyte monitoring application and features related thereto.

For example, in some embodiments, an onboarding GUI can be displayed which indicates to the user that the glucose-ketone scanning process is complete. In some embodiments, an onboarding GUI can inform the user that the glucose-ketone sensor has been started and will be ready in a predetermined period of time (e.g., in 60 minutes). In some embodiments, an onboarding GUI explains to the user why monitoring ketones is important. For example, and as depicted in FIG. 8A, onboarding GUI 800 can explain to the user that tracking ketone levels is important to detect early signs of diabetic ketoacidosis, and that regular monitoring can help prevent complications. Onboarding GUI 800 can include a selectable back button 801 to return the user to the previously displayed onboarding GUI, and a next button 802 to output a subsequent onboarding GUI. Specifically, in some embodiments, onboarding GUI 805 (FIG. 8B) is displayed next to the user. Onboarding GUI 805 can comprise a selectable back button 806, a selectable next button 807, and a message 808 indicating to the user that in addition to providing glucose readings, the analyte monitoring application can also display ketone readings. Moreover, in some embodiments, the message 808 can further indicate that ketone levels can be normal, elevated, or high.

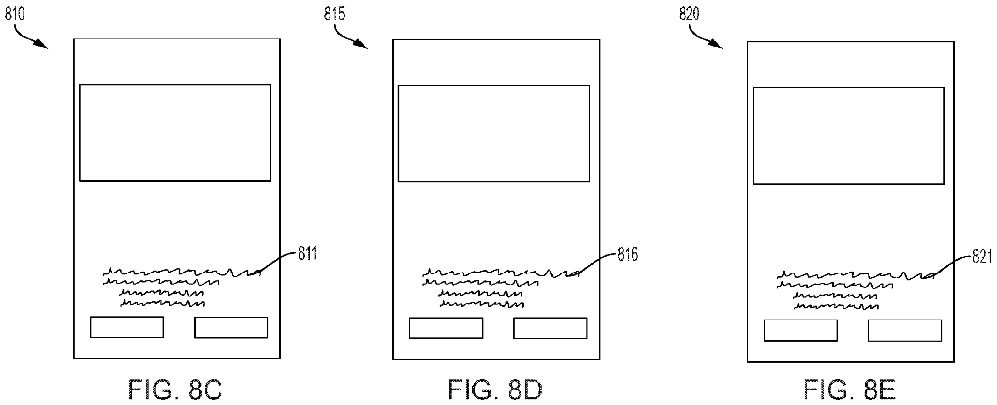

Next, and as depicted in FIG. 8C, an onboarding GUI 810 can be displayed to the user which comprises introductory information on normal ketones. Specifically, onboarding GUI 810 can comprise a selectable back button, a selectable next button, and a message 811 informing the user that when the user's current ketone reading is within a normal range, the user will see a green banner on the home screen or home GUI. Following this, an onboarding GUI 815 (FIG. 8D), which comprises introductory information on elevated ketones, can be displayed to the user. Specifically, onboarding GUI 815 can comprise a selectable back button, a selectable next button, and a message 816 notifying the user that when the user's current ketone reading is elevated, the ketone value will be displayed on a yellow banner. Additionally, the message 816 can indicate to the user that a graph will be shown. Next, and as depicted in FIG. 8E, an onboarding GUI 820 can be displayed, wherein onboarding GUI 820 comprises introductory information related to high ketones. Specifically, onboarding GUI 820 can comprise a selectable back button, a selectable next button, and a message 821 informing the user that when the user's current ketone reading is high, the ketone value will be displayed on a red banner. In some embodiments, the message 821 can further inform the user to seek medical attention when the user has high ketones. In some embodiments, the portion of the message 821 pertaining to seeking medical attention when the user has high ketones can comprise a bolded font so as to emphasize the importance of the information.

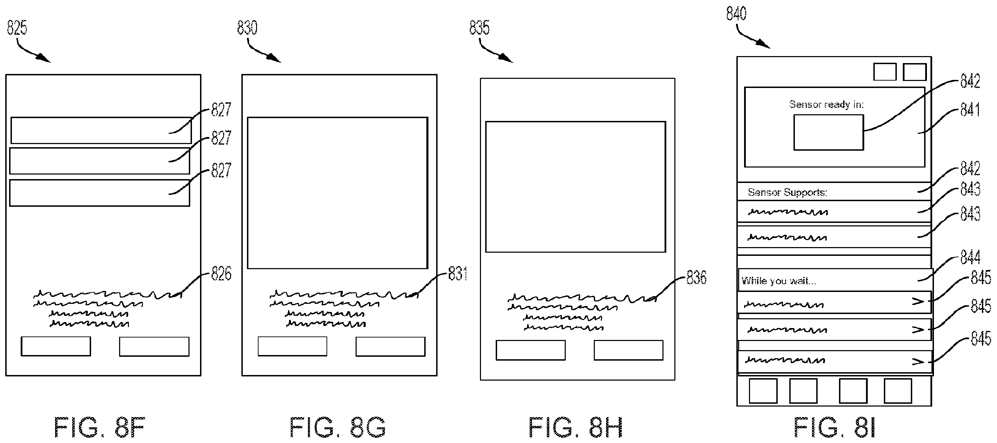

Further, in some embodiments, and as depicted in FIG. 8F, an onboarding GUI 825 can be displayed to the user, wherein onboarding GUI 825 provides introductory information on various directional trend arrows displayed in the analyte monitoring application. For example, onboarding GUI 825 can comprise a message 826 indicating that an arrow indicates the user's recent ketone trend, and that there are fewer trend arrows for ketones than there are for glucose trends. In some embodiments, onboarding GUI 825 further comprises one or more images 827 illustrating directional trend arrows related to ketone trends. For example, in some embodiments, the one or more images 827 illustrates (1) an upward angled directional trend arrow indicates that the ketone level is rising; (2) a horizontally-angled directional trend arrow indicates that the ketone level is changing slowly; and (3) a downward angled directional trend arrow indicates that the ketone level is falling. In some embodiments, onboarding GUI 825 can also include a selectable back button and a selectable next button.

Next, and as shown in FIG. 8G, an onboarding GUI 830 can be displayed to the user, wherein the onboarding GUI 830 comprises a selectable back button, a selectable next button, and a message 831 informing the user that the user can scroll up to view ketone information, and that a ketone graph will only appear if ketone levels are above a normal range. Following this, an onboarding GUI 835 (FIG. 8H) can be displayed, wherein onboarding GUI 835 comprises a selectable back button, a selectable next button, and a message 836 indicating that alarms will notify the user when the user's ketone levels are elevated or high.

Referring next to FIG. 8I, a sensor warm-up GUI 840 is displayed next, wherein sensor warm-up GUI 840 comprises a sensor warm-up card 841 with a timer or countdown indicator 842 configured to indicator to the user when the glucose-ketone sensor will be ready (e.g., "58 minutes"). In some embodiments, sensor warm-up GUI 840 further comprises a sensor supports section 842 with a plurality of selectable support options 843, wherein each of the plurality of selectable options 843, upon being selected, can output an interface which provides the user with additional information related to what the glucose-ketone supports. For example, in some embodiments, the sensor supports section 842 can comprise selectable support options 843 relating to (1) glucose and ketone alarms and/or (2) streaming glucose and ketone readings. Additionally, in some embodiments, the sensor warm-up GUI 840 can further comprise an informational section 844 with a plurality of selectable informational options 845, wherein each of the plurality of selectable informational options 845, upon being selected, can output an interface which provides the user with additional information related to the analyte monitoring application or features related thereto. Specifically, in some exemplary embodiments, the informational section 844 can include selectable informational options 845 relating to (1) learning more about the user's continuous glucose monitoring; (2) why ketones are important; and (3) learning how the user can customize alarms. Those of skill in the art will appreciate that the sensor supports section 842 and informational section 844 can provide various other types of support options 843 and informational options 845, respectively, which can output various other types of information to the user without departing from the scope of the present disclosure.

### Example Embodiments of Insights GUIs and Features Related Thereto

FIGS. 9A through 9F are block diagrams depicting example embodiments of insights interfaces, or features related thereto, any of which can be utilized with the embodiments described herein. Referring to FIGS. 9A through 9E, an example embodiment of a block diagram of an insights GUI 900 for an analyte monitoring application. As best shown in FIGS. 4H-1 to 4H-3, from sensor results GUI 41000, or any other sensor results interface described herein, the user can select an insights icon on the bottom bar navigation menu (e.g., the selectable insights icon 41052 on the bottom bar navigation menu 41050 depicted inFIGS. 4H-1 to 4H-3,), and the insights GUI 900 (FIGS. 9A through 9F) can be outputted.

With reference to FIGS. 9A to 9E, insights GUI 900 can provide daily updates and information related to the data indicative of the first analyte level and the data indicative of the second analyte level for a particular period of time (e.g., a particular day). Specifically, insights GUI 900 comprises a Daily Summary view 901a and a Reports view 901b, with a toggle, switch, or slidable element that allows the user to select or transition between the two views.

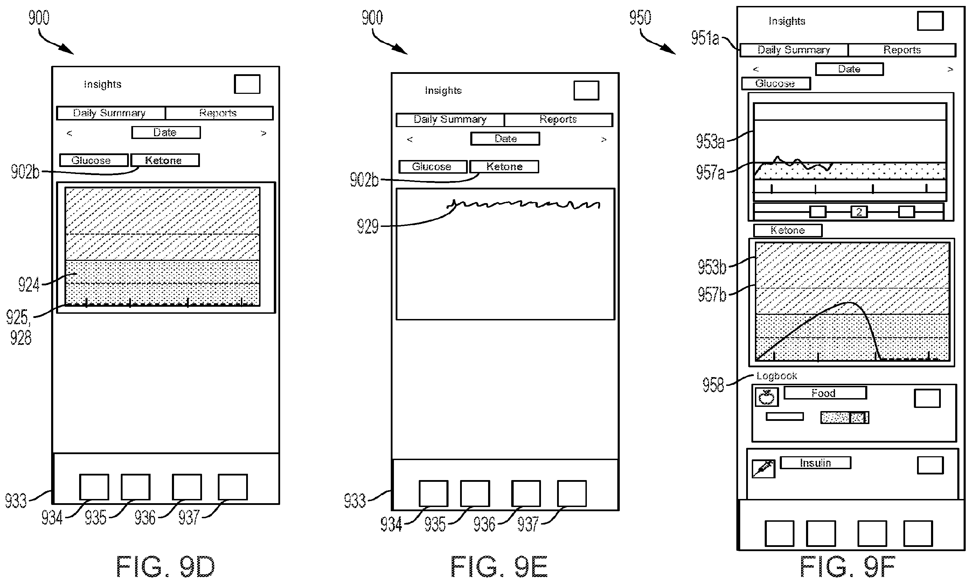

According to one aspect of the embodiments, the Daily Summary view 901a can comprise a selectable first analyte tab 902a or a first tab 902a (e.g., a glucose tab 902a) which outputs data indicative of the first analyte level (best shown in FIG. 9A), and a selectable second analyte tab 902b or a second tab 902b (a ketone tab 902b) which outputs data indicative of the second analyte level (best shown in FIGS. 9C and 9D). In this manner, the Daily Summary view 901a is configured to output only one of the data indicative of the first analyte level or data indicative of the second analyte level on a single screen. In some embodiments, the selectable second analyte tab 902b is only accessible if there is data indicative of the second analyte level for a past predetermined time period (e.g., the last 30 days, the last 60 days, or the last 90 days).

In some embodiments, the selectable first tab 902a comprises a bolded portion or colored portion to indicate the first tab 902 has been selected (e.g., FIG. 9A). In like manner, the selectable second tab comprises a bolded portion or colored portion to indicate the second tab 902b has been selected (e.g., FIGS. 9B-9E).

Further and with particular reference to FIG. 9A, the Daily Summary view 901a further comprises a date indicator 903 (e.g., "Thurs, Mar 10"), showing the relevant date associated with the displayed data indicative of the first analyte level and data indicative of the second analyte level. In some embodiments, a selectable left switch 905a is displayed adjacent to and to the left of the date indicator 903, and a selectable right switch 905b is displayed adjacent to and to the right of the date indicator 903. Specifically, the selectable left switch 905a, upon being selected, updates the relevant date associated with the daily updates and information outputted to insights GUI 900. More specifically the selectable left switch 905a changes the relevant date to a preceding day and the date indicator 903 is updated accordingly to reflect the relevant preceding day's date (e.g., upon the user selecting the left switch 905a, the date indicator 903 shows "Wed Mar 9" instead of "Thurs Mar 10"). Similarly, the selectable right switch 905b, upon being selected, updates the relevant date associated with the daily updates and information outputted to insights GUI 900. More specifically the selectable right switch 905b changes the relevant date to a subsequent day and the date indicator 903 is updated accordingly to reflect the relevant preceding day's date (e.g., upon the user selecting the right switch 905b, the date indicator 903 shows "Fri Mar 11" instead of "Thurs Mar 10'').

In some embodiments, the date indicator 903 is selectable. In this manner, and upon the user selecting the date indicator 903, a calendar half-sheet 904 (FIG. 9B) is outputted to insights GUI 900, wherein the calendar half-sheet 904 provides a chart with days for a particular month (e.g., a calendar half-sheet 904 for December 2022), wherein the user can select on a particular day in the chart so as to output an insights GUI 900 comprising daily updates and information pertaining to the selected day (e.g., the user can select on the 6^{th} day of December 2022 shown in the chart so as to output insights GUI 900 comprising daily updates and information pertaining to December 6^{th} 2022). In some embodiments, and as best shown in FIG. 9B, the calendar half-sheet 904 can include one or more switches 906 such that the user can change the month displayed on the calendar half-sheet 904. In this regard, the user can view an insights GUI 900 pertaining to data indicative of the first analyte level and the data indicative of the second analyte level in previous days.

Further, and turning back to FIG. 9A, the first analyte tab 902a of the Daily Summary view 901a is configured to output a first analyte graph summary section 907a (e.g., a glucose graph summary section 907a) and a logbook section 908. In some embodiments, the logbook section 908 is displayed directly adjacent to and below the first analyte graph summary section 907a. Specifically, the first analyte graph summary section 907a comprises a first analyte graph 910 reflecting data indicative of the first analyte level for a particular day associated with the insights GUI 900. More specifically, the first analyte graph 910 can comprise a first analyte trend line 911 to reflect the user's analyte level, based on the data indicative of the first analyte level, over the particular day associated with the insights GUI 900. In some embodiments, the first analyte graph 910 can include a shaded color area (e.g., a green colored portion) 912 to indicate the user's target analyte range (e.g., target glucose threshold) associated with the data indicative of the first analyte level.

In some embodiments, and still with reference to FIG. 9A, the first analyte graph summary section 907a can further comprise an event timeline 913. Specifically, in some embodiments, the event timeline 913 can be positioned below, above, or adjacent to the first analyte graph 910. More specifically, one or more icons 914 are positioned the event timeline 913, and are oriented such that each icon 914 aligns with one or more points along an x-axis of the first analyte graph 910. Each of the one or more icons 914 is configured to represent the occurrence of an event for the user. In this manner, the user can visually associate an event represented by each of the one or more icons 914 with data indicative of the first analyte level that is represented by a corresponding point on the first analyte trend line 911. In some embodiments, the one or more icons 914 can include: a food icon, a rapid-acting insulin icon, a long-acting insulin icon, and an exercise icon. In some embodiments, if one or more events occurred at a same time, then one icon 914 is used to represent the one or more events. Specifically, in some embodiments, the one icon 914 used to represent the one or more events can include a numerical number to represent the number of events represented by the one icon 914 (e.g., "2" to represent the occurrence of two contemporaneous events).

With reference to FIG. 9A, the logbook section 908 of the first analyte tab 902a can comprise one or more activity events related to the data indicative of the first analyte level. Specifically, the activity events can include one or more of the following: (1) one or more meal events 915 configured to indicate a time of a logged or detected meal, (2) one or more treatment events 916 configured to indicate a time in which the user administered a treatment; (3) and one or more exercise events configured to indicate a time of a logged or detected exercise (not illustrated). Specifically, in some embodiments, each of the one or more meal events 915 can include one or more of the following: (1) a timestamp 917 associated with a time the meal event 915 (e.g., "9:41am PST"); and (2) a first analyte level value 918 associated with the meal event 915 (e.g., "110 mg/dL") adjacent to a trend indicator 919 (e.g., a directional trend arrow indicating the glucose level is changing slowly).

In some embodiments, and as best shown in FIG. 9A, the first analyte level value 917 and trend indicator 919 are displayed in colored box 920, wherein the color of the colored box 920 is indicative of a condition associated with the first analyte level value associated with the meal event 915 (e.g., a green colored box 920 to indicate a normal glucose level associated with the meal event 915). In some embodiments, each meal event 915 further includes a macronutrient counter 921 (e.g., a carbohydrate counter 921), wherein the macronutrient counter indicates the amount of macronutrients associated with the meal event (e.g., "27g" to represent 27 grams of carbohydrates associated with the meal event 915). Those of skill in the art will appreciate that various other types of macronutrients can be represented by the macronutrient counter 921 without departing from the scope of the present disclosure.

Further, in some embodiments, and still with reference to FIG. 9A, each of the one or more treatment events 916 can be a rapid-acting insulin event 916 or a long-lasting insulin event 916. According to an aspect of the embodiments, each of the one or more treatment events 916 can comprise a textual description 922 which indicates the type of treatment event 916 (e.g., "rapid-acting insulin" or "long-lasting insulin") and a dosage counter 923. Specifically, the dosage counter is configured to indicate a dosage associated with the treatment event 916. For example, if the user had 10 units of rapid-acting insulin, then the corresponding dosage counter 923 can indicate "10 units" were utilized (e.g., "10U"). In some embodiments, though not illustrated in FIG. 9A, each treatment event 916 comprises a timestamp indicate a time associated with the treatment event 916. Those of skill in the art will appreciate that various other types of treatment events can be displayed on the first analyte tab 902a without departing from the scope of the present disclosure.

According to another aspect of the embodiments, though not illustrated, each of the one or more exercise events can comprise a timestamp associated with a time of the exercise event along with an exercise duration indicator configured to indicate a duration or amount of time spend on the exercise represented by the exercise event.

In some embodiments, the one or more meal events 915 one or more treatment events 916, and/or one or more exercise events are displayed in chronological order in the logbook section 908.

Further, and with particular reference to FIG. 9C, in some embodiments, the second analyte tab 902b of the Daily summary View 901a is configured to output a second analyte graph summary section 907b (e.g., a ketone graph summary section 907b) and an alarms section 909. Specifically, the second analyte graph summary section 907b comprises a second analyte graph 924 reflecting data indicative of the second analyte level for a particular day associated with the insights GUI 900. More specifically, the second analyte graph 924 can comprise second analyte trend line 925 to reflect the user's analyte level, based on the data indicative of the second analyte level, over the particular day associated with the insights GUI 900. In some embodiments, the second analyte graph 924 can include a shaded area 926 indicative of a high ketone threshold and a shaded area 927 indicative of an elevated ketone threshold. More specifically, in some embodiments, the shaded area 926 indicative of a high ketone threshold is a first color (e.g., red color) and the shaded area 927 indicative of an elevated ketone threshold is a second color (e.g., yellow color), wherein the second color is different from the first color. More specifically, in some embodiments, the shaded area 926 indicative of a high ketone threshold covers an area on the second analyte graph 924 corresponding to a high ketone level (e.g., 1.5 mmol/L and above), and the shaded area 927 indicative of an elevated ketone threshold covers an area on the second analyte graph 924 corresponding to an elevated ketone level (e.g., expanding across an area of the y-axis which covers between 0.5 mmol/L up to 1.5 mmol/L).

As best shown in FIGS. 9C-9D, the second analyte trend line 925 can comprise a colored portion 928 (e.g., green colored portion) configured to indicate a condition associated with the second current analyte data. For example, the colored portion 928 can reflect when the user's analyte level is in a predetermined normal analyte range (e.g., normal ketone range). Specifically, according to some embodiments, when the user's analyte level is in the predetermined normal ketone range, the colored portion 928 is displayed along the area of the second analyte trend line 925 which corresponds to the period of time in which the user's analyte level is in the predetermined normal ketone range. In some embodiments, when the user's analyte level is in a predetermined normal analyte range (e.g., normal ketone range), the second analyte trend line 925 comprises the colored portion 938 and is configured as a flat line for the period of time corresponding to when the user's analyte level is within the predetermined normal ketone range. For example, if the user's analyte level is in the predetermined normal ketone range between 6PM and 9PM, then the second analyte trend line 925 appears on the second analyte graph 924 with the colored portion (e.g., green colored portion) 928 and as a flat line across the x-axis from the time range in which the user's analyte level was in the predetermined normal ketone range (e.g., between 6PM to 9PM).

According to an aspect of the embodiment, and as best depicted in FIG. 9D, if an adverse state of condition is not detected in association with the second historical analyte data and/or second current analyte data for the particular day represented by insights GUI 900, then the entire second analyte trend line 925 appears as a flat line comprising the colored portion 928. Further, in some embodiments, if no ketone alarm condition or elevated ketone alarm condition is present for the particular day represented by insights GUI 900, then the second analyte tab 902b of the Daily summary View 901a does not display the alarms section (best shown in FIGS. 9D and 9E).

In some embodiments, and with reference to FIG. 9E, if data indicative of the second analyte level is not available or present, then the second analyte tab 902b of the Daily summary View 901a can comprise a message 929 indicating to the user that there is not enough sensor data. Specifically, in some embodiments, if data indicative of the second analyte level is not available or present, then the second analyte tab 902b can display the message 929 instead of the second analyte graph summary section (e.g., a ketone graph summary section).

Further, and turning back to FIG. 9C, the second analyte tab 902b of the Daily summary View 901a can further comprise the alarms section 909, wherein the alarms section 909 can include a list of alarm events 930 related to the data indicative of the second analyte level. For example, in some embodiments, the alarms section 909 can include a list of alarm events 930 relating to high ketone alarms for a high ketone alarm condition and/or elevated ketone alarms for an elevated ketone alarm condition. According to an aspect of the embodiments, each alarm event 930 can include a textual description 931 (e.g., "High Ketone Alarm" or "Elevated Ketone Alarm") indicating the type of alarm corresponding to the alarm event 930, and a timestamp 932, wherein the timestamp 932 indicates a time associated with the alarm event 930.

According to yet another aspect of the embodiments, the insights GUI 900 can further comprise a bottom bar navigation menu 933 with a plurality of selectable icons. In some embodiments, and as depicted in FIGS. 9A, and 9C-9E, the bottom bar navigation menu 933 comprises four selectable icons: (1) a selectable home icon 934 which, upon being selected, outputs a home or sensor results interface, as described herein (e.g., FIGS. 4B-1 to 4H-3); (2) a selectable insights icon 935 which, upon being selected, outputs interfaces comprising insights, reports, logbooks, daily summary data (e.g., FIGS. 9A-9F); (3) a selectable alarms icon 936 which, upon being selected, outputs interfaces relating to alarms (e.g., FIG. 6A); and (4) a selectable profile icon 937 which, upon being selected, outputs an interface related to the user's profile or account.

FIG. 9F is depicting a block diagram of an additional example embodiment of an insights GUI 950 or features related thereto, any of which can be utilized with the embodiments described herein. Insights GUI 950 is similar to insights GUI 900 (FIGS. 9A to 9E), except that a Daily Summary view 951a of insights GUI 950 comprises a first analyte portion 953a with a first analyte graph summary section 957a and a second analyte portion 953b with a second analyte graph summary section 957b on a single screen, wherein the first analyte portion 953a includes data indicative of the first analyte level, and the second analyte portion 953b includes data indicative of the second analyte level. Specifically, in some embodiments, the first analyte portion 953a is directly adjacent to and above the second analyte portion 953b on insights GUI 950. Further, in some embodiments, and as depicted in FIG. 9F, the Daily Summary view 951a can include a logbook section 958, wherein the logbook section 958 is displayed directly adjacent to and below the first analyte portion 953a and the second analyte portion 953b. In some embodiments, though not illustrated, the Daily Summary view 951a can also include an alarms section. In some embodiments, though not illustrated, the alarms section can be directly adjacent to and below the logbook section 958.

### Example Embodiments of Report GUIs and Features Related Thereto

FIGS. 10A through 10D are block diagrams depicting example embodiments of report GUIs, or features related thereto, for analyte monitoring systems, any of which can be utilized with the embodiments described herein.

FIG. 10A depicts an example embodiment of a report GUI 1000 for an analyte monitoring system, in which report GUI 1000 includes a plurality of interfaces 1100A and 1100B, each of which depicts a user's analyte levels (e.g., glucose levels and/or ketone levels) over a different predetermined time period in conjunction with a plurality of summary metrics.

As an initial matter, each interface of the plurality of interfaces 1100A and 1100B can display, in a single user-friendly format, (1) a first graph representing data indicative of the first analyte level (e.g., data indicative of the glucose level) over a predetermined time period, accompanied by a first plurality of useful summary metrics based on, or relating to, the data indicative of the first analyte level obtained from the analyte monitoring system; and (2) a second graph representing data indicative of the second analyte level (e.g., data indicative of the ketone level) over the predetermined time period, accompanied by a second plurality of useful summary metrics based on, or relating to, the data indicative of the second analyte level obtained from the analyte monitoring system. Further, the first and second plurality of summary metrics related to the data indicative of the first analyte level and the data indicative of the second analyte level, respectively, displayed in report GUI 1000 can be associated with the predetermined time period, e.g., a day, a week, or a month. For example, as shown in FIG. 10A, report GUI 1000 is configured to display a first graph of the data indicative of the first analyte level, a second graph of the data indicative of the second analyte level, and a first and second plurality of summary metrics for one day, as indicated by date 1001 (e.g., "SUN, Jun 03").

According to one aspect of the embodiments, report GUI 1000 can include a graph portion comprising the first graph, the second graph, an x-axis 1002 based on units of time, a first y-axis 1003 indicative of a first analyte concentration (e.g., glucose concentration) and a second y-axis 1004 indicative of a second analyte concentration (e.g., ketone concentration). For example, report GUI 1000 includes an x-axis 1002, labeled in two-hour increments across a twenty-four hour period, a first y-axis 1003, labeled in units of milligrams of glucose per deciliter (mg/dL), and a second y-axis 1003, labeled in units of millimoles per liter (mmol/L). Those of skill in the art will appreciate that the x-axis and/or the first and second y-axis can be labeled with other increments or units. For example, the x-axis can be labeled in thirty-minute increments, one-hour increments, or four-hour increments, etc.

According to another aspect of the embodiments, the graph portion of report GUI 1000 can further display a first high analyte threshold value ("180 mg/dL") and a first low analyte threshold value ("70 mg/dL") for a first target analyte range 1005 corresponding to the data indicative of the first analyte level, as indicated by the numeric labels on the right side. In like manner, the graph portion of report GUI 1000can further display a second high analyte threshold value ("1.5 mmol/L") for a second target analyte range 1006 corresponding to the data indicative of the second analyte level, as indicated by the numeric label on the right side. In some embodiments, the numeric values for the first target analyte range 1005 and the second target analyte range 1006 can be different colors from one another, and from the labels on the x-axis and/or y-axis, so as to make them more distinctive. Furthermore, in some embodiments, a first plurality of colored lines, each of which reflects a corresponding threshold value of the first target analyte range 1005, can extend across the graph portion to provide a visual reference with respect to the relationship at any point-in-time between the user's data indicative of the first analyte level and first target analyte range 1005. In some embodiments, a second plurality of colored lines, each of which reflects a corresponding threshold value of the second target analyte range 1006, can extend across the graph portion to provide a visual reference with respect to the relationship at any point-in-time between the user's data indicative of the second analyte level and second target analyte range 1006. In addition, according to some embodiments, the first target analyte range 1005 can be configured by the user, for example, by adjusting either or both of the first high analyte threshold value or the first low analyte threshold value.

Referring still to FIG. 10A, graph portion of report GUI 1000can include a first trend line 1007 showing the user's first analyte concentrations over the predetermined time period based on the data indicative of the first analyte level, and a second trend line 1008 showing the user's second analyte concentrations over the predetermined time period based on the data indicative of the second analyte level. According to one aspect of the embodiments, certain portions of the first trend line 1007 can be displayed in a first color (e.g., green) to indicate that, at an indicated time, the user's data indicative of the first analyte level is within first target analyte range 1005. Further, certain portions of the second trend line 1008 can be displayed in the first color (e.g., green) to indicate that, at an indicated time, the user's data indicative of the second analyte level is within second target analyte range 1006. In some embodiments, the second trend line 1008 is configured as a flat line along the x-axis so as to indicate that the user's data indicative of the second analyte level is within second target analyte range 1006. Though not illustrated, other portions of the first trend line 1007 can be displayed in a second color (e.g., orange) to indicate that, at a different indicated time, the user's data indicative of the first analyte level is above the first target analyte range 1005. Similarly, other portions of the first trend line 1007 can be displayed in a third color (e.g., red) to indicate that, at a different indicated time, the user's data indicative of the first analyte level is below the first target analyte range 1007.

Further, though not illustrated, other portions of the second trend line 1008 can be displayed in the second color (e.g., orange) to indicate that, at a different indicated time, the user's data indicative of the second analyte level is above the second target analyte range 1006, in a first condition (e.g., elevated ketone condition). Similarly, other portions of the second trend line 1008 can be displayed in the third color (e.g., red) to indicate that, at a different indicated time, the user's data indicative of the second analyte level is above the second target analyte range 1006, in a second condition (e.g., high ketone condition).

In some embodiments, the respective areas under (or above) the first trend line 1007 can be shaded or filled using the second color if the user's data indicative of the first analyte level is above the first target analyte range, shaded or filled using the third color if the user's data indicative of the first analyte level is below the first target analyte range. In some embodiments, the respective areas under (or above) the second trend line 1008 can be shaded or filled using the second color if the user's data indicative of the second analyte level is above the second target analyte range, in the first condition, and shaded or filled using the third color if the user's data indicative of the second analyte level is above the second target analyte range, in the second condition. According to some embodiments, the colored areas can extend from the line indicating the exceeded analyte threshold value to the first trend line 1007 and/or second trend line 1008. In this regard, these colored areas can graphically represent to the user the severity and/or duration of an analyte excursion. By contrast, if the user stays within the first target analyte range 1005 or the second target analyte range 1006 during the predetermined time period, the first trend line 1007 and/or second trend line 1008 remains colored according to the first color (e.g., green).

According to some embodiments, the respective areas under (or above) the first trend line 1007 and/or the second trend line 1008 can remain uncolored. In still other embodiments, the first trend line 1007 and/or the second trend line 1008 can each be a single color regardless of whether the analyte levels are above, below, or within first target analyte range 1005 or the second target analyte range 1006, respectively.

According to some embodiments, certain information can also be superimposed on the first graph proximate to the first trend line 1007. For example, in certain analyte monitoring systems, user-initiated analyte checks 1010 can be graphically represented as one or more discrete points on the first graph to indicate the time(s) at which said checks occurred. A user-initiated analyte check can be, for example, a finger stick blood glucose test, a scan of the sensor control device, a download of analyte data from a trusted computer system, a view of the analyte monitoring program on the reader device, and/or a rendering of a specific GUI (e.g., home screen, sensor results screen) of the analyte monitoring program or analyte monitoring application on the reader device. Those of skill in the art will appreciate that other types of user-initiated analyte checks are possible and within the scope of the present disclosure. According to another aspect of some embodiments, exercise events can be graphically represented as an exercise icon 1011 on the first graph to indicate the time(s) at which said exercise events occurred.

According to another aspect of some embodiments, report GUI 1000 can comprise one or more summary metrics. For example, in some embodiments, report GUI 1000 can include a "Time in Range" metric 1030 to indicate the percentage of time (e.g., "79%") that a user's data indicative of a first analyte level was within a first target analyte range for the predetermined time period. In other embodiments, the "Time in Range" metric 1030 can be displayed as a graphical representation (e.g., a pie chart, a plurality of bar portions, a partially filled ring), or, in the alternative, a numeric amount of time. Other types of graphical representations for "Time in Range" metrics are described in U.S. Publ. Nos. 2021/0282673, 2022/0248988, 2021/037860, 2022/0000399, 2021/0030323, 2022/0092019, and 2022/0110551, all of which are incorporated by reference in their entireties for all purposes.

Referring still to FIG. 10A, the summary metrics can also include total amounts of food or medication 1012 taken during the predetermined time period. In some embodiments, for example, the summary metrics can include the amount of carbohydrates (in units of grams) that was ingested by the user during the predetermined time period, and/or the type (e.g., rapid-acting or long-acting) and the number of units of insulin injected by the user during the predetermined time period. According to another aspect of the embodiments, the specific food and/or medication events 1013 can be displayed next to the first graph (e.g., above the first trend line 1007, below the first trend line 1007, or between the first trend line 1007 and the second trend line 1008), such that each food and/or medication event 1013 is aligned with an indicated time reflected along the x-axis. According to one aspect of some embodiments, each type of summary metric can be shown in a different color associated with the associated event(s) shown in a graph section of report GUI 1000, as described below.

In some embodiments a carbohydrate icon 1021 or a medication icon 1022 can be displayed in a first icon area to indicate, respectively, a meal event or an insulin event. In addition, according to some embodiments, the specific amount of carbohydrates (in units of grams) and/or the type (e.g., rapid-acting or long-acting) and the number of units of insulin injected by the user can also be displayed next to the carbohydrate icon 1021 or medication icon 1022. Accordingly, a user will be able to visually ascertain, at a glance, whether certain events (e.g., meal or insulin), and specific details regarding those events (e.g., grams of carbohydrates or units of rapid-acting insulin), has had an effect on the first trend line 1007. In some embodiments, an alarm icon can also be displayed in the first icon area to indicate the detection of an alarm condition associated with the data indicative of the first analyte level. Further, the position of alarm icon along the x-axis can indicate to the user the specific time at which the alarm condition was detected.

In addition, although the icons are shown in the first icon area that is separate from the first graph of report GUI 1000, those of skill in the art will appreciate that, in alternative embodiments, the icons can be displayed directly on the first graph adjacent to the first trend line 1007. Likewise, details of the alarm condition, grams of carbohydrates, and/or units of insulin can also be displayed directly on the first graph adjacent to the first trend line 1007.

Still with reference to FIG. 10A, the summary metrics can also include a first minimum analyte level 1031 and a first maximum analyte level 1032 for the predetermined time period (e.g., "301") relating to the data indicative of the first analyte level. Likewise, first maximum analyte levels 1016 and first minimum analyte levels 1017 for each time increment (e.g., every hour) can be displayed along the x-axis and adjacent to the graph portion. In addition, as can be seen in FIG. 10A, if either of the first minimum or the maximum analyte levels are above the first target analyte range, then the numeric value can be displayed in a first color (e.g., orange). Similarly, if either of the first minimum or the maximum analyte levels are below the first target analyte range, then the numeric value can be displayed in a second color (e.g., red). By contrast, if either of the first minimum or the maximum analyte levels are within the first target analyte range, then the numeric value can be displayed without any coloring. Accordingly, report GUI 1000 draws the user's attention to those portions of the first trend line 1007, and the corresponding first minimum or maximum analyte level numeric values, that are outside the first target analyte range to allow the user to ascertain the underlying reasons.

Furthermore, and still referring to FIG. 10A, the summary metrics can also include alarm metrics. Specifically, the summary metrics can include the total number of alarms related to the data indicative of the second analyte level for the predetermined time period. In some embodiments, for example, the summary metrics can include high ketone alarm metric 1014 which indicates a number of instances in which a high ketone alarm condition was present or triggered (e.g., "1") for the predetermined time period, and an elevated ketone alarm metric 1015 which indicates a number of instances in which an elevate ketone alarm condition was present or triggered (e.g. "1") for the predetermined time period. According to another aspect of the embodiments, a second maximum analyte level value metric 1018 for each time increment (e.g., every hour) can be displayed along the x-axis and adjacent to the second graph, wherein each second maximum analyte level value metric 1018 indicates a maximum second analyte level value for each time increment. In addition, as can be seen in FIG. 10A, if the second maximum analyte level value metric 1018 is associated with an elevated ketone alarm condition, then the second analyte level value metric 1018 can be displayed in the first color (e.g., orange or yellow). Similarly, if the second analyte level value metric 1018 is associated with a high ketone alarm condition, then the second analyte level value metric 1018 can be displayed in the second color (e.g., red). Accordingly, report GUI 1000 draws the user's attention to those portions of the second trend line 1008, and the corresponding second analyte level value metric 1018, that are outside the second target analyte range to allow the user to ascertain the underlying reasons.

In some embodiments, the summary metrics can also include an alarm condition metric 1019 which indicates the maximum second analyte level value associated with a high ketone alarm condition or elevated ketone alarm condition for the predetermined time period (e.g., "2.1"). In some embodiments, an alarm icon 1020 can be displayed in a second icon area to indicate the detection of an alarm condition. Further, the position of alarm icon 1020 along the x-axis can indicate to the user the specific time at which the alarm condition was detected. In addition, although the alarm icons 1020 are shown in the second icon area that is separate from the second graph of report GUI 1000, those of skill in the art will appreciate that, in alternative embodiments, the alarm icons 1020 can be displayed directly on the second graph adjacent to the second trend line 1008.

In some embodiments, when the data indicative of the second analyte level is within a second target analyte range for the entire predetermined time period, then the second graph portion is displayed in a collapsed view. Specifically, in some embodiments, and as best seen in interface 1100B, when the data indicative of the second analyte level is within a second target analyte range for the entire predetermined time period, the report GUI 1000 is configured such that the second trend line is replaced with a message 1027 indicating to the user that that there the user is within the second target analyte range (e.g., "Normal ketones, below or equal to 0.5 mmol/L.").

Further, according to another aspect of the embodiments, and with reference to FIG. 10A, report GUI 1000 can have a specific arrangement that enhances a user's ability to recognize patterns across multiple time periods. For example, the x-axis of each of interfaces 1100A and 1100B can be aligned with each other such that the specific time increments for each interface are also aligned. In this regard, a user is able to quickly recognize if her analyte levels are exceeding a first or second analyte threshold at a particular time of the day (e.g., after lunch).

According to another aspect of the embodiments, report GUI 1000 can include the name of the user 1024, a label indicating a selected reporting period 1023, and a "Time CGM Active" statistic 1025 to indicate the amount of analyte data available during the selected reporting period 1023. According to some embodiments, the selected reporting period 1023 can be configurable by the user. For example, in some embodiments, the selected reporting period 1023 can be configured by a selected start date and a selected end date. In other embodiments, the selected reporting period 1023 can be configured to a specific period of time (e.g., one week, two weeks, one month, etc.) Furthermore, in some embodiments, the "Time CGM Active" statistic can comprise a percentage of time 1035 during the selected reporting period 1023 for which analyte data was obtained. In other embodiments, the "Time CGM Active" statistic 1025 can comprise an actual amount of time (e.g., "12 Days, 11 Hours, 32 Minutes"). Report GUI 1000 can also include a legend 1026 providing textual descriptions of one or more icons that are displayed in any one or more of the interfaces 1100A and 1100B.

According to another aspect of the embodiments, though not illustrated, a GUI of a snapshot report is displayed, wherein the snapshot report covers a predetermined time period and comprises a plurality of report portions on a single report GUI, including: a glucose trend interface, which can include an glucose trend graph, a low glucose events graph, and other related glucose metrics (e.g., Glucose Management Indicator); a health information interface, which can include information logged by the user about the user's average daily carbohydrate intake and medication dosages (e.g., insulin dosages); and a comments interface, which can include additional information about the user's analyte and medication patterns presented in a narrative format. In some embodiments, the comments interface provides a summary of high ketone events (e.g., "There were 2 high ketone events observed and the highest ketone value was 2.2 mmol/L."). Variants of GUIs comprising a snapshot report that are suitable for use with the system, device, and method embodiments set forth herein, are described in U.S. Patent Publication No. 2021/0378601, all of which is incorporated by reference in its entirety for all purposes.

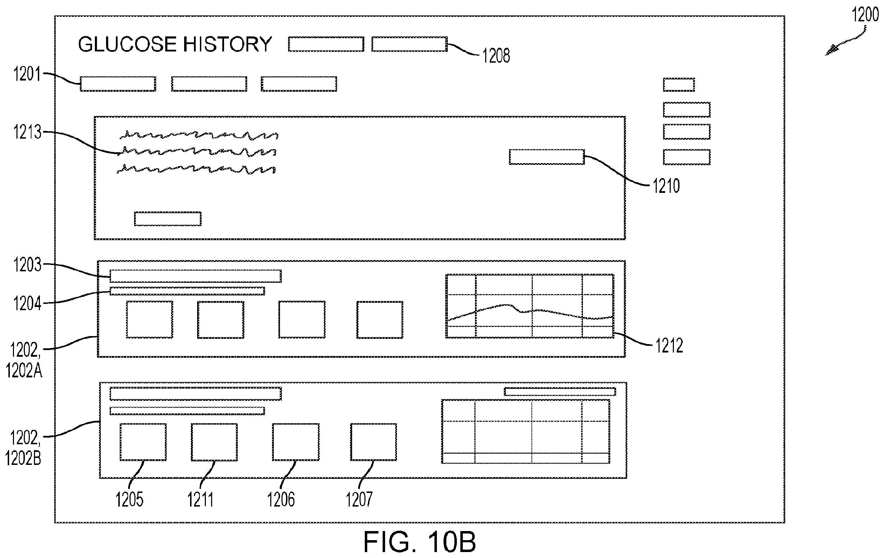

FIG. 10B depicts an example embodiment of a GUI associated with a patient dashboard 1200. The dashboard 1200 can allow a user to display merged analyte data (e.g., glucose data and/or ketone data) from different sources, e.g., FreeStyle Libre and FreeStyle Libre Pro. Specifically, the dashboard 1200 comprises one or more selectable tabs 1201, wherein each tab is configured to output to the dashboard 1200 information associated with one of the different sources. In some embodiments, one of the one or more selectable tabs 1201, upon being selected, outputs information related to all collected data associated with all the different sources.

According to an aspect of the embodiments, the dashboard 1200 comprises a selectable Glucose Report button 1210 which, upon being selected, allows the user to customize the dashboard 1200 to display different sets of data/information provided on report cards 1202. For example, report card 1202A and 1202B are displayed on dashboard 1200 shown in FIG. 10B.

In some embodiments, dashboard 1200 includes a message 1213 informing the user that they can view glucose history on the dashboard or click Glucose Report button 1210 to create customized reports that the user can view now or print/save in Portable Document Format (PDF). The message 1213 can further inform the user that healthcare professionals should use information in the software in conjunction with other clinical information available to them.

Specifically, in some embodiments, the user can utilize a filter to customize the dashboard 1200. The filter can include selections to display specific data over a selected time-range, compare specific data over a selected time-range, and/or choose the selected time-range for which each report card 1202 provides data/information related thereto. In some embodiments, upon the user selecting a filter, a corresponding tag 1208 is displayed on the dashboard to indicate to the user which filters are being utilized (e.g., "2 weeks" or "Compare"). Further, in some embodiments, the tag(s) 1208 comprises a toggle which allows the user to update the tag 1208. In some embodiments, a timeline is provided on a right-side panel of the dashboard 1200 so as to allow the user to select a particular timeframe for the selected time-range (e.g. October 2022, September 2022, August 2022).

According to some embodiments, the dashboard 1200 includes a plurality of the report cards 1202, wherein each of the report cards 1202 is configured to provide a summary report for a selected time-range (e.g., one-week period, two-week period, one-month period). Specifically, each report card 1202 can comprise: (1) a date-range indicator 1203 (e.g., "September 29, 2022-October 12, 2022") which indicates the period of time corresponding to the selected time-range associated with the respective report card 1202; (2) a source indicator 1204 to indicate the type of device and/or software used to obtain the data indicative of the first analyte level and/or the data indicative of the second analyte level for the respective report card 1202; (3) an average glucose indicator 1205 (e.g., "96 mg/dL); (4) a hypo-event indicator 1206 configured to indicate a number of hypoglycemic events for the period of time corresponding to the selected time-range associated with the respective report card 1202 (e.g., "4 Hypo Events"), (5) a ketone-event indicator 1207 (also herein referred to as a ketone-alarm indicator 1207) configured to indicate a number of instances in which a high ketone condition or alarm was presented for the period of time corresponding to the selected time-range associated with the respective report card 1202 (e.g., "2 High Ketone events" or "2 High Ketone alarms"); (6) data-percentage indicator 1211 configured to indicate a percentage of days within a total number of day for the period of time for which analyte data was provided (e.g. "100% Days of Data"); and (7) an analyte graph 1212 of specific data over a portion of or all of the selected time-range.

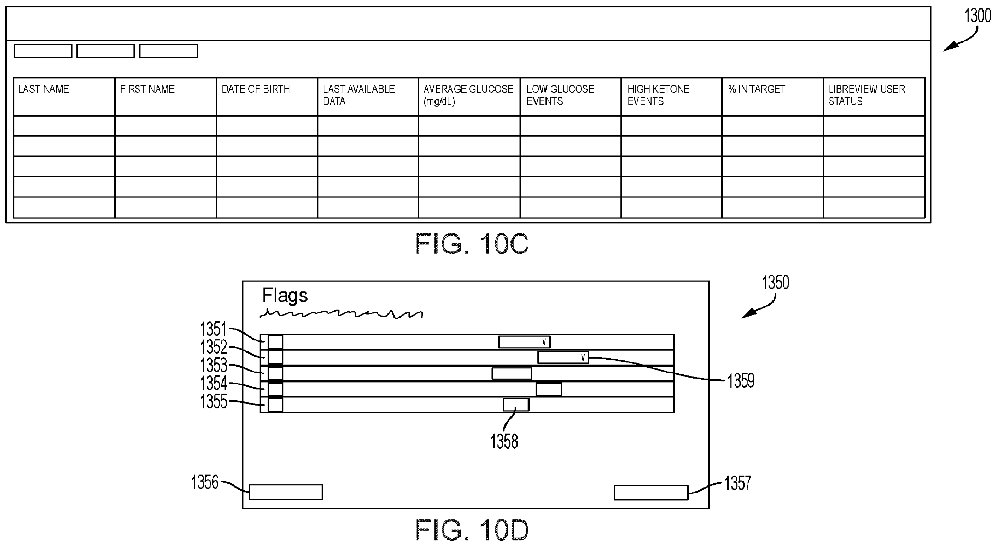

Turning to FIG. 10C, an additional example embodiment of a GUI associated with a patient dashboard 1300 is depicted. Specifically, the user can select different columns to include in the patient dashboard display, including but not limited to: (1) a last name; (2) a first name; (3) a date of birth; (4) date associated with last available data (e.g., data indicative of the first analyte level or data indicative of the second analyte level); (5) average glucose (mg/dL); (6) a number of incidences related to low glucose events; (7) a number of incidences related to high ketone events; (8) a percentage in targe; and (9) a user status indicator.

In some embodiments, the user can create conditional flags to highlight patients in the patient dashboard 1300. Specifically, the user can, through a flag modal 1350 (FIG. 10D), select from a plurality of selectable flagging options. For example, the user can select different flag options so as to filter the patients and data related thereto that is displayed in the patient dashboard 1300. In some exemplary embodiments, the flag modal can comprise a plurality of selectable flagging options, such as: (1) a usage flagging option 1351 which, upon being selected, highlights patients in the dashboard 1300 who have not uploaded data in a particular elapsed period of time (e.g. in the last day, last week, last two weeks, last four weeks, last 90 days, last 180 days, etc.); (2) a ketone alarm flagging option 1352 which, upon being selected, highlights patients in the dashboard 1300 who have at least one ketone event in a particular elapse period of time (e.g. in the last day, last week, last two weeks, last four weeks, last 90 days, last 180 days, etc.); (3) an average scans/views per day flagging option 1353 which, upon being selected, highlights patients in the dashboard 1300 who have fewer than a predetermined number of scans/views per day (e.g., "3" scans/views per day); (4) an average glucose flagging option 1354 which, upon being selected, highlights patients in the dashboard 1300 who have an average glucose value above a predetermined threshold (e.g., 150 mg/dL); and (5) a low glucose events flagging option 1355 which, upon being selected, highlights patients in the dashboard 1300 with more than a predetermined number of low glucose events according to their low glucose threshold setting (e.g. one low glucose event).

In some embodiment, the user can select or update the particular elapsed period of time for the usage flagging option 1351 and ketone alarm flagging option 1352 through a selectable field 1359 which is configured to output a dropdown menu (not illustrated), wherein the dropdown menu provides the user with a plurality of selectable options, such as a plurality of selectable periods of time to choose from for the particular elapsed period of time. In some embodiments, a text-entry field 1358 is provided to allow the user to enter a particular elapsed period of time, a predetermined number of scans/views per day, predetermined threshold, and/or predetermined number of low glucose events. Further, in some embodiments, the flag modal 1350 includes a selectable cancel button 1356 and a selectable save button 1357.

Various aspects of the present subject matter are set forth below, in review of, and/or in supplementation to, the embodiments described thus far, with the emphasis here being on the interrelation and interchangeability of the following embodiments. In other words, an emphasis is on the fact that each feature of the embodiments can be combined with each and every other feature unless explicitly stated otherwise or logically implausible. The embodiments described herein are restated and expanded upon in the following paragraphs without explicit reference to the figures.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

To the extent the embodiments disclosed herein include or operate in association with memory, storage, and/or computer readable media, then that memory, storage, and/or computer readable media are non-transitory. Accordingly, to the extent that memory, storage, and/or computer readable media are covered by one or more claims, then that memory, storage, and/or computer readable media is only non-transitory.

In many instances, entities are described herein as being coupled to other entities. It should be understood that the terms "coupled" and "connected" (or any of their forms) are used interchangeably herein and, in both cases, are generic to the direct coupling of two entities (without any non-negligible (e.g., parasitic) intervening entities) and the indirect coupling of two entities (with one or more non-negligible intervening entities). Where entities are shown as being directly coupled together, or described as coupled together without description of any intervening entity, it should be understood that those entities can be indirectly coupled together as well unless the context clearly dictates otherwise.

The subject matter described herein and in the accompanying figures is done so with sufficient detail and clarity to permit the inclusion of claims, at any time, in means-plus-function format pursuant to 35 U.S.C. section 112, part (f) However, a claim is to be interpreted as invoking this means-plus-function format only if the phrase "means for" is explicitly recited in that claim.

Aspects of the invention are set out in the independent claims and preferred features are set out in the dependent claims. The preferred features of the dependent claims may be provided in combination in a single embodiment and preferred features of one aspect may be provided in conjunction with other aspects.

As used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

While the embodiments are susceptible to various modifications and alternative forms, specific examples thereof have been shown in the drawings and are herein described in detail. These embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the scope of the claims by features, functions, steps, or elements that are not within that scope.

### Clauses

Exemplary embodiments are set forth in the following numbered clauses.
1. A system for monitoring a plurality of analytes in a user, the system comprising:
   a sensor control device comprising a sensor, wherein at least a portion of the sensor is configured to be in fluid contact with a bodily fluid of the user, and wherein the sensor control device is configured to transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte;
   a reader device comprising:
      wireless communication circuitry configured to receive the data indicative of the plurality of analyte levels of the user,
      one or more processors coupled with a memory, the memory storing an analyte monitoring application that, when executed by the one or more processors, causes the one or more processors to:
         output, based on the data indicative of the first analyte level and the data indicative of the second analyte level, a sensor results graphical user interface (GUI) comprising a first analyte section and a second analyte section,
         wherein the first analyte section comprises a first analyte card and a first analyte graph portion reflecting the data indicative of the first analyte level, and
         wherein the second analyte section comprises a second analyte card and a second analyte graph portion reflecting the data indicative of the second analyte level.
2. The system of clause 1, wherein the reader device is a smart phone.
3. The system of clause 1 or 2, wherein the data indicative of the first analyte level comprises data indicative of a glucose level.
4. The system of clause 1, 2 or 3, wherein the data indicative of the second analyte level comprises data indicative of a ketone level.
5. The system of any preceding clause, wherein the sensor is a glucose-ketone sensor.
6. The system of any preceding clause, wherein the first analyte card comprises a text description, a first current analyte level value, and a first trend indicator associated with the data indicative of the first analyte level of the user.
7. The system of clause 6, wherein the first trend indicator is a first directional trend arrow.
8. The system of clause 7, wherein the first directional trend arrow indicates a glucose trend.
9. The system of clause 6, 7 or 8, wherein the text description includes information on a condition associated with the data indicative of the first analyte level of the user.
10. The system of any one of clauses 6 to 9, wherein the text description indicates whether the first current analyte level value is within a predetermined analyte range.
11. The system of any one of clauses 6 to 10, wherein the first analyte card further comprises an alarm icon, wherein the alarm icon is adjacent to the text description.
12. The system of any one of clauses 6 to 11, wherein the first analyte card further comprises a calibration icon, wherein the calibration icon is adjacent to the first current analyte level value.
13. The system of any one of clauses 6 to 12, wherein the first analyte card further comprises a background color indicative of a condition associated with the data indicative of the first analyte level of the user.
14. The system of any of clauses 6 to 13, wherein the first current analyte level value comprises a numerical value and a first unit of measure.
15. The system of clause 14, wherein the first current analyte level value is a current glucose level value, and wherein the first unit of measure is a value in mg/dL.
16. The system of any of clauses 6 to 15, wherein the data indicative of the first analyte level value comprises an out-of-range condition, and wherein the first analyte card further comprises an out-of-range text indicator associated with the out-of-range condition.
17. The system of clause 16, wherein the first current analyte level value and the first trend indicator are not displayed on the first analyte card when the out-of-range condition is present.
18. The system of any one of clauses 1 to 16, wherein the second analyte card comprises a text description, a second current analyte level value, and a second trend indicator associated with the data indicative of the second analyte level of the user.
19. The system of clause 18, wherein the second trend indicator is a second directional trend arrow.
20. The system of clause 19, wherein the second directional trend arrow indicates a ketone trend.
21. The system of any of clauses 18 to 20, wherein the text description includes information on a condition associated with the data indicative of the second analyte level of the user.
22. The system of any one of clauses 18 to 21, wherein the text description indicates whether the second current analyte level value is within a predetermined analyte range.
23. The system of any one of clauses 18 to 22, wherein the second analyte card further comprises an alarm icon, wherein the alarm icon is adjacent to the text description.
24. The system of any one of clauses 18 to 23, wherein the second analyte card further comprises a calibration icon, wherein the calibration icon is adjacent to the second current analyte level value.
25. The system of any one of clauses 18 to 24, wherein the second analyte card further comprises a background color indicative of a condition associated with the data indicative of the second analyte level of the user.
26. The system of any one of clauses 18 to 25, wherein the second current analyte level value comprises a numerical value and a second unit of measure.
27. The system of any one of clauses 18 to 26, wherein the second current analyte level value is a current ketone level value, and wherein the second unit of measure is a value in mmol/L.
28. The system of any one of clauses 18 to 27, wherein the second current analyte level value is within a predefined analyte range, and wherein the second current analyte level value and the second trend indicator are only displayed on the second analyte card when the second current analyte level exceeds the predefined analyte range.
29. The system of clause 28, wherein only the text description of the second analyte card is displayed when the second current analyte level value is within the predefined analyte range, and wherein the text description comprises information relating to the predefined analyte range.
30. The system of any preceding clause, wherein the first analyte section is configured to transition between a first collapsed view and a first expanded view, wherein the sensor results GUI is further configured to display the first analyte section in the first expanded view by default.
31. The system of any preceding clause, wherein the first analyte section is configured to transition between a first collapsed view and a first expanded view, wherein the first collapsed view is displayed on the sensor results GUI, wherein the reader device further comprises a touchscreen, wherein the analyte monitoring application, when executed by the one or more processors, further causes the one or more processors to:
   receive input from the touchscreen corresponding to a gesture, preferably a scroll gesture, a tap gesture, or a pulldown gesture, and
   display the first expanded view of the first analyte section in response to the received input
32. The system of any preceding clause, wherein the first analyte section is configured to transition between a first collapsed view and a first expanded view, wherein the first collapsed view is displayed on the sensor results GUI, wherein the reader device further comprises a touchscreen, wherein the analyte monitoring application, when executed by the one or more processors, further causes the one or more processors to:
   receive input from the touchscreen corresponding to a drag gesture on the sensor results GUI,
      and
   display the first expanded view of the first analyte section in response to the received input.
33. The system of any of clauses 32, wherein the second analyte section is configured to transition between a second collapsed view and a second expanded view, wherein the analyte monitoring application, when executed by the one or more processors, further causes the one or more processors to:
   display the second collapsed view in response to the received input, wherein the second collapsed view of the second analyte section is configured to display only the second analyte card.
34. The system of any preceding clause, wherein the first analyte section is configured to transition between a first expanded view and a first collapsed view, wherein the first expanded view is displayed on the sensor results GUI, wherein the reader device further comprises a touchscreen, wherein the analyte monitoring application, when executed by the one or more processors, further causes the one or more processors to:
   receive input from the touchscreen corresponding to a scroll gesture, a tap gesture, or a pullup gesture, and
   display the first collapsed view of the first analyte section in response to the received input.
35. The system of clause 34, wherein the second analyte section is configured to transition between a second collapsed view and a second expanded view, wherein the analyte monitoring application, when executed by the one or more processors, further causes the one or more processors to:
   display the second expanded view in response to the received input from the touchscreen,
   wherein in the second expanded view, the second analyte section displays the second analyte card and the second analyte graph portion.
36. The system of any of clauses 30 to 35, wherein in the first expanded view, the first analyte section displays the first analyte card and the first analyte graph portion.
37. The system of any of clauses 30 to 36, wherein in the first collapsed view, the first analyte section displays only the first analyte card.
38. The system of any of clauses 30 to 37, wherein the first analyte graph portion is only displayed on the sensor results GUI when the first analyte section is in the first expanded view.
39. The system of any of clauses 30 to 38, wherein the first analyte card is displayed on the sensor results GUI when the first analyte section is in the first expanded view or in the first collapsed view.
40. The system of any preceding clause, wherein the second analyte section is configured to transition between a second collapsed view and a second expanded view in response to the data indicative of the second analyte level reaching a predetermined threshold.
41. The system of any preceding clause, wherein the second analyte section is configured to transition between a second collapsed view and a second expanded view, wherein the second collapsed view is displayed on the sensor results GUI, wherein the reader device further comprises a touchscreen, wherein the analyte monitoring application, when executed by the one or more processors, further causes the one or more processors to:
   receive input from the touchscreen corresponding to a scroll gesture, a tap gesture, or a pullup gesture, and
   display the second expanded view of the second analyte section in response to the received input.
42. The system of clause 41, wherein the first analyte section is configured to transition between a first collapsed view and a first expanded view, wherein the analyte monitoring application, when executed by the one or more processors, further causes the one or more processors to:
   display the first collapsed view of the first analyte section in response to the received input from the touchscreen, wherein in the first collapsed view, the first analyte section displays only the first analyte card.
43. The system of any preceding clause, wherein the second analyte section is configured to transition between a second collapsed view and a second expanded view, wherein the second expanded view is displayed on the sensor results GUI, wherein the reader device further comprises a touchscreen, wherein the analyte monitoring application, when executed by the one or more processors, further causes the one or more processors to:
   receive input from the touchscreen corresponding to a scroll gesture, a tap gesture, or a pulldown gesture, and
   display the second collapsed view of the second analyte section in response to the received input.
44. The system of clause 43, wherein the first analyte section is configured to transition between a first collapsed view and a first expanded view, wherein the analyte monitoring application, when executed by the one or more processors, further causes the one or more processors to:
   display the first expanded view in response to the received input from the touchscreen, wherein in the first expanded view, the first analyte section displays the first analyte card and the first analyte graph portion.
45. The system of any of clauses 40 to 44, wherein in the second expanded view, the second analyte section displays the second analyte card and the second analyte graph portion.
46. The system of any of clauses 40 to 45, wherein in the second collapsed view, the second analyte section displays only the second analyte card.
47. The system of any of clauses 40 to 46, wherein the second analyte graph portion is only displayed on the sensor results GUI when the second analyte section is in the second expanded view.
48. The system of any of clauses 40 to 47, wherein the second analyte card is displayed on the sensor results GUI when the second analyte section is in the second expanded view or in the second collapsed view.
49. The system of any preceding clause, wherein the first analyte section is configured to transition between a first collapsed view and a first expanded view, and wherein the second analyte section is configured to transition between a second collapsed view and a second expanded view.
50. The system of clause 49, wherein the first analyte section is in the first expanded view and the second analyte section is in the second expanded view, and wherein the first analyte card, the first analyte graph portion, the second analyte card, and the second analyte graph portion are displayed on the sensor results GUI at a same time
51. The system of clause 49 or 50, wherein the first analyte section is in the first expanded view and the second analyte section is in the second collapsed view, and wherein the first analyte card, the first analyte graph portion, and the second analyte card are displayed on the sensor results GUI at a same time.
52. The system of clause 49, 50 or 51, wherein the first analyte section is in the first collapsed view and the second analyte section is in the second expanded view, and wherein the first analyte card, the second analyte card, and the second analyte graph portion are displayed on the sensor results GUI at a same time.
53. The system of any of clauses 49 to 52, wherein the first analyte section is in the collapsed view and the second analyte section is in the collapsed view, and wherein the first analyte card and the second analyte card are displayed on the sensor results GUI at a same time.
54. The system of any of clauses 49 to 53, wherein the first analyte section is a glucose section, wherein the first analyte card is a glucose card, wherein the first analyte graph portion is a glucose graph portion, and wherein the data indicative of a first analyte level is a data indicative of a glucose level.
55. The system of clause 54, wherein the second analyte section is a ketone section, wherein the second analyte card is a ketone card, wherein the second analyte graph portion is a ketone graph portion, and wherein the data indicative of a second analyte level is a data indicative of a ketone level.
56. The system of clause 55, wherein the data indicative of the glucose level indicates a target glucose threshold range, a high glucose threshold range, or a low glucose threshold range, and wherein the sensor results GUI is configured to display the glucose section in the first expanded view.
57. The system of clause 55 or 56, wherein in the first expanded view, the glucose section displays the glucose card and the glucose graph portion, wherein the glucose card comprises a text description, a current glucose level value, and a glucose trend indicator associated with the data indicative of the glucose level, and wherein the glucose graph portion comprises a glucose trend line associated with the data indicative of the glucose level
58. The system of any of clauses 55 to 57, wherein the data indicative of the ketone level indicates a normal ketone threshold range, and wherein the sensor results GUI is configured to display the ketone section in the second collapsed view.
59. The system of any of clauses 55 to 58, wherein in the second collapsed view, the ketone section only displays the ketone card, and wherein the ketone card comprises a text description indicating the normal ketone threshold range.
60. The system of any of clauses 55 to 59, wherein the glucose card, the glucose graph portion, and the ketone card are displayed on the sensor results GUI at a same time.
61. The system of any of clauses 55 to 60, wherein the reader device further comprises a touchscreen, wherein the analyte monitoring application, when executed by the one or more processors, further causes the one or more processors to:
   receive input from the touchscreen corresponding to a selected point on the ketone section, and
   based on the received input, transition the ketone section from the second collapsed view to the second expanded view, wherein in the second expanded view, the ketone section is configured to display the ketone card and a message relating to the normal ketone threshold range.
62. The system of clause 61, wherein, based on the received input, the glucose section is configured to transition from the first expanded view to the first collapsed view, wherein in the first collapsed view, the glucose section displays only the glucose card.
63. The system of any of clauses 55 to 62, wherein the data indicative of the ketone level indicates an elevated ketone threshold range or a high ketone threshold range, and wherein the sensor results GUI is configured to display the ketone section in the second expanded view.
64. The system of any of clauses 55 to 63, wherein in the second expanded view, the ketone section is configured to display the ketone card and the ketone graph portion, wherein the ketone card comprises a text description, a current ketone level value, and a ketone trend indicator associated with the data indicative of the ketone level, and wherein the ketone graph portion comprises a ketone trend line associated with the data indicative of the ketone level.
65. The system of any of clauses 55 to 64, wherein the glucose card, the glucose graph portion, and the ketone card, and the ketone graph portion are displayed on the sensor results GUI at a same time.
66. The system of any of clauses 55 to 65, wherein the reader device further comprises a touchscreen, wherein the analyte monitoring application, when executed by the one or more processors, further causes the one or more processors to:
   receive input from the touchscreen corresponding to a selected point on ketone section, and
   based on the received input, transition the glucose section from the first expanded view to the first collapsed view,
   wherein in the first collapsed view, the glucose section displays only the glucose card, and
   wherein the ketone section continues to display on the sensor results GUI in the second expanded view.
67. The system of any of clauses 55 to 66, wherein the reader device further comprises a touchscreen, wherein the analyte monitoring application, when executed by the one or more processors, further causes the one or more processors to:
   receive input from the touchscreen corresponding to a selected point on glucose section, and
   based on the received input, transition the ketone section from the second expanded view to the second collapsed view,
   wherein in the second collapsed view, the ketone section displays only the ketone card, and
   wherein the glucose section continues to display on the sensor results GUI in the first expanded view.
68. The system of any preceding clause, wherein the data indicative of the first analyte level of the user includes a first historical analyte data and a first current analyte data, and wherein the first analyte graph portion comprises the first historical analyte data and the first current analyte data.
69. The system of clause 68, wherein, upon a first use of the sensor, the first analyte graph portion does not display the first historical analyte data, and wherein the first analyte graph portion displays the first current analyte data.
70. The system of any preceding clause, wherein the first analyte graph portion comprises a first analyte trend line based on the data indicative of the first analyte level of the user.
71. The system of clause 70, wherein the first analyte trend line comprises an x-axis which includes a unit of time and a y-axis which includes a unit of measure associated with the data indicative of the first analyte level of the user.
72. The system of clause 70, wherein the first analyte trend line comprises a colored circle, wherein the colored circle is indicative of a condition associated with the data indicative of the first analyte level of the user.
73. The system of clause 70, wherein the first analyte trend line comprises a colored portion on an area of the first analyte trend line, wherein the colored portion indicates a condition corresponding to the data indicative of the first analyte level associated with the area of the first analyte trend line.
74. The system of any preceding clause, wherein the data indicative of the second analyte level of the user includes a second historical analyte data and a second current analyte data, and wherein the second analyte graph portion comprises the second historical analyte data and the second current analyte data.
75. The system of clause 74, wherein, upon a first use of the sensor, the second analyte graph portion does not display the second historical analyte data, and wherein the second analyte graph portion displays the second current analyte data.
76. The system of any preceding clause, wherein the second analyte graph portion comprises a second analyte trend line based on the data indicative of the second analyte level of the user.
77. The system of clause 76, wherein the second analyte trend line comprises an x-axis which includes a unit of time and a y-axis which includes a unit of measure associated with the data indicative of the second analyte level of the user.
78. The system of clause 76, wherein the second analyte trend line comprises a colored circle, wherein the colored circle is indicative of a condition associated with the data indicative of the second analyte level of the user.
79. The system of any preceding clause, wherein the first analyte card and the first analyte graph portion are displayed on the sensor results GUI, wherein the reader device further comprises a touchscreen, wherein the analyte monitoring application, when executed by the one or more processors, further causes the one or more processors to:
   receive input from the touchscreen corresponding to a selected point on the first analyte graph portion, and
   update the first analyte section based on the received input from the touchscreen.
80. The system of clause 79, wherein the data indicative of the first analyte level of the user includes a first historical analyte data and a first current analyte data, wherein the first analyte graph portion comprises the first historical analyte data and the first current analyte data, and wherein, based on the received input, the first analyte graph portion is configured to display the first historical analyte data associated with the selected point.
81. The system of clause 79 or 80, wherein the first analyte graph portion comprises a first analyte trend line based on the data indicative of the first analyte level of the user, wherein the first analyte trend line comprises a first colored circle indicating the first current analyte data,
   wherein the analyte monitoring application, when executed by the one or more processors,
   further causes the one or more processors to:
      update the first analyte section based on the received input, wherein, once updated, the first analyte section further comprises a second colored circle corresponding to the selected point, and wherein a color of the second colored circle indicates a condition corresponding to the first historical analyte data associated with the selected point.
82. The system of any of clauses 79 to 81, wherein the first analyte graph portion is further configured to display a timestamp corresponding to the first historical analyte data associated with the selected point.
83. The system of any preceding clause, wherein the second analyte card and the second analyte graph portion are displayed on the sensor results GUI wherein the reader device further comprises a touchscreen, wherein the analyte monitoring application, when executed by the one or more processors, further causes the one or more processors to:
   receive input from the touchscreen corresponding to a selected point on the second analyte graph portion, and
   update the second analyte section based on the received input from the touchscreen.
84. The system of clause 83, wherein the data indicative of the second analyte level of the user includes a second historical analyte data and a second current analyte data, wherein the second analyte graph portion comprises the second historical analyte data and the second current analyte data, and wherein, based on the received input, the second analyte graph portion is configured to display the second historical analyte data associated with the selected point
85. The system of clause 83 or 84, wherein the second analyte graph portion comprises a second analyte trend line based on the data indicative of the second analyte level of the user, wherein the second analyte trend line comprises a first colored circle indicating the second current analyte data, wherein the analyte monitoring application, when executed by the one or more processors, further causes the one or more processors to:
   update the second analyte section based on the received input, wherein, once updated, the second analyte section further comprises a second colored circle corresponding to the selected point, wherein a color of the second colored circle indicates a condition corresponding to the second historical analyte data associated with the selected point.
86. The system of clause 83, 84 or 85, wherein the second analyte graph portion is further configured to display a timestamp corresponding to the second historical analyte data associated with the selected point.
87. The system of any preceding clause, wherein the first analyte graph portion comprises one or more lines indicative of a low glucose threshold or a high glucose threshold.
88. The system of clause 87, wherein the one or more lines comprise a color indicative of the low glucose threshold or the high glucose threshold.
89. The system of clause 87 or 88, wherein the first analyte graph portion further comprises a shaded area indicative of a target glucose threshold.
90. The system of any preceding clause, wherein the second analyte graph portion comprises one or more lines indicative of an elevated ketone threshold or a high ketone threshold.
91. The system of clause 90, wherein the one or more lines comprise a color indicative of the elevated ketone threshold or the high ketone threshold.
92. The system of clause 90 or 91, wherein the second analyte graph portion further comprises a shaded area indicative of a normal ketone threshold.
93. The system of any preceding clause, wherein the first analyte graph portion comprises one or more icons, wherein the one or more icons are displayed on one or more points on the first analyte graph portion, and wherein the one or more icons are associated with the data indicative of the first analyte level corresponding to the one or more points.
94. The system of any preceding clause, wherein the second analyte graph portion comprises one or more icons, wherein the one or more icons are displayed on one or more points on the second analyte graph portion, and wherein the one or more icons are associated with the data indicative of the second analyte level corresponding to the one or more points.
95. The system of clause 93 or 94, wherein the one or more icons comprise a food icon, a rapid-acting insulin icon, a long-acting insulin icon, or an exercise icon.
96. The system of clause 93, 94 or 95, wherein the one or more icons are selectable, wherein the reader device further comprises a touchscreen, and wherein the analyte monitoring application, when executed by the one or more processors, further causes the one or more processors to:
   receive input from the touchscreen corresponding to a selected one or more icons, and
   display a note card section corresponding to the selected one or more icons, wherein the note card section includes a text description associated with the selected one or more icons.
97. The system of any preceding clause, wherein the first analyte section is displayed proximal and directly adjacent to the second analyte section on the sensor results GUI.
98. The system of any preceding clause, wherein the first analyte card and the second analyte card are displayed on the sensor results GUI at all times.
99. The system of any preceding clause, wherein the second analyte card and the second analyte graph portion are displayed on the sensor results GUI, wherein the analyte monitoring application, when executed by the one or more processors, further causes the one or more processors to:
   display, on the second analyte graph portion, a banner notification comprising instructions, wherein the instructions relate to a high ketone level condition or an elevated ketone level condition, wherein the banner notification further comprises a background color indicative of the high ketone level condition or the elevated ketone level condition.
100. The system of any preceding clause, wherein data indicative of the first analyte level of the user is provided periodically, and wherein data indicative of the second analyte level of the user is provided periodically.
101. The system of any preceding clause, wherein data indicative of the first analyte level of the user is provided every minute, and wherein data indicative of the second analyte level of the user is provided every minute.
102. The system of any preceding clause, wherein the data indicative of the first analyte level of the user and the data indicative of the second analyte level of the user are displayed on the analyte monitoring application at a same time.
103. The system of any preceding clause, wherein the first analyte section is automatically updated at a predetermined frequency based on the data indicative of the first analyte level of the user.
104. The system of any preceding clause, wherein the second analyte section is automatically updated at a predetermined frequency based on the data indicative of the second analyte level of the user.
105. The system of any preceding clause, wherein the analyte monitoring application, when executed by the one or more processors, further causes the one or more processors to: determine a status of the sensor, and
   display, on the sensor results GUI, the status of the sensor, wherein the status of the sensor comprises an indication of a remaining lifetime of the sensor, wherein the indication of the remaining lifetime of the sensor comprises a graphical indication, wherein the graphical indication comprises a progress indicator that visually illustrates the remaining lifetime of the sensor.
106. The system of clause 105, wherein the graphical indication is a plurality of circles and the progress indicator is each box of the plurality of circles that comprises a colored portion, wherein the ratio of the each circle of the plurality of circles that comprises the colored portion and a total plurality of circles is proportional to a ratio of the remaining lifetime of the sensor and a total lifetime of the sensor
107. The system of clause 106, wherein the total lifetime of the sensor is about 15 days.
108. The system of any of clauses 105 to 107, wherein if the remaining lifetime of the sensor is greater than about 1 day, the graphical indication is a first plurality of circles and the progress indicator is each circle of the first plurality of circles that comprises a first colored portion, and wherein if the remaining lifetime of the sensor is less than about 1 day, the graphical indication is a second plurality of circles and the progress indicator is each circles of the second plurality of circles that comprises a second colored portion.
109. The system of clause 108, wherein if the remaining lifetime of the sensor is greater than about 1 day, the graphical indication is a first plurality of circles and the progress indicator is each circle of the first plurality of circles that comprises a first colored portion, wherein if the remaining lifetime of the sensor is less than about 1 day, the graphical indication is a second plurality of circles and the progress indicator is each circles of the second plurality of circles that comprises a second colored portion, and wherein if the remaining lifetime of the sensor is less than about 1 hour, the graphical indication is a graphical bar and the progress indicator a portion of the graphical bar comprising a third colored portion.
110. The system of clause 109, wherein the ratio of each circle of the second plurality of circles that comprises the second colored portion and a total second plurality of circles is proportional to a ratio of the remaining lifetime of the sensor relative to the less than about 1 day of remaining lifetime of the sensor.
111. The system of clause 109 or 110, wherein the ratio of the portion of the graphical bar that comprises the third colored portion and a total of the graphical bar is proportional to a ratio of the remaining lifetime of the sensor relative to the less than about 1 hour of remaining lifetime of the sensor.
112. The system of any of clauses 105 to 111, wherein the indication of the remaining lifetime of the sensor further comprises a text description comprising a numerical value.
113. The system of clause 112, wherein if the remaining lifetime of the sensor is greater than about 1 day, the numerical value of the text description comprises a number of days of the remaining lifetime of the sensor
114. The system of clause 112 or 113, wherein if the remaining lifetime of the sensor is less than about 1 day but greater than about 1 hour, the numerical value of the text description comprises a number of hours of the remaining lifetime of the sensor.
115. The system of clause 112, 113 or 114, wherein if the remaining lifetime of the sensor is less than about 1 hour, the numerical value of the text description comprises a number of minutes of the remaining lifetime of the sensor.
116. The system of any preceding clause, further comprising the trusted computer system, wherein the trusted computer system comprises a cloud-based server.
117. The system of any preceding clause, wherein the sensor control device is configured to wirelessly transmit the data indicative of the analyte level of the user to the first reader device according to a Bluetooth or Bluetooth Low Energy communication protocol.
118. The system of any preceding clause, wherein the sensor control device is configured to wirelessly transmit the data indicative of the first analyte level of the user and the data indicative of the second analyte level of the user to the reader device according to a Near Field Communication protocol.
119. The system of any preceding clause, wherein reader device is configured to wirelessly communicate with the sensor control device according to a first wireless communication protocol, and wherein the reader device is further configured to wirelessly communicate with the trusted computer system according to a second wireless communication protocol that is different from the first wireless communication protocol.
120. The system of any preceding clause, wherein the sensor results GUI further comprises a selectable add note link, wherein the analyte monitoring application, when executed by the one or more processors, further causes the one or more processors to:
   in response to a selection of the add note link, output a note interface.
121. A system for monitoring a plurality of analytes in a user, the system comprising:
   a sensor control device comprising an analyte sensor, wherein at least a portion of the analyte sensor is configured to be in fluid contact with a bodily fluid of the user, and wherein the sensor control device is configured to transmit data indicative of a plurality of analyte levels of the user, wherein the data indicative of the plurality of analyte levels comprises a data indicative of a first analyte level and a data indicative of a second analyte level, wherein the first analyte level is indicative of a first analyte, and wherein the second analyte level is indicative of a second analyte different from the first analyte;
   a reader device comprising:
   wireless communication circuitry configured to receive the data indicative of the plurality of analyte levels of the user, and
   one or more processors coupled with a memory, the memory storing an analyte monitoring application that, when executed by the one or more processors, causes the one or more processors to:
      determine whether the data indicative of the first analyte level or the data indicative of the second analyte level meets one or more alarm conditions, wherein the one or more alarm conditions comprise a first alarm condition associated with a first set of alarm settings that are configurable by the user and a second alarm condition associated with a second set of alarm settings that are not configurable by the user,
      in response to a determination that at least one of the one or more alarm conditions is met, display an alarm notification user interface (GUI) comprising an alarm associated with the at least one of the one or more alarm conditions.
122. The system of clause 121, wherein the reader device is a smart phone.
123. The system of clause 121 or 122, wherein the reader device further comprises a touchscreen, and wherein the analyte monitoring application, when executed by the one or more processors, further causes the one or more processors to:
   receive input from the touchscreen corresponding to a drag gesture, a long-press gesture, or a swipe gesture, and
   expand the alarm notification GUI in response to the received input, wherein when expanded, the alarm notification GUI comprises the alarm associated with the at least one of the one or more alarm conditions, wherein the alarm notification GUI further comprises a first analyte card and a second analyte card, wherein the first analyte card reflects data indicative of the first analyte level, and wherein the second analyte card reflects data indicative of the second analyte level.
124. The system of any of clauses 121 to 123, wherein the data indicative of the first analyte level of the user comprises data indicative of a glucose level, and wherein the data indicative of the second analyte level of the user comprises data indicative of a ketone level.
125. The system of any of clauses 121 to 124, wherein the alarm comprises an alarm condition text, an analyte alarm message, an analyte level measurement, and a trend indicator associated with the at least one of the one or more alarm conditions.
126. The system of clause 125, wherein the alarm notification GUI further comprises a time-of-alarm indicator.
127. The system of clause 126, wherein the time-of-alarm indicator is configured to indicate an amount of time elapsed since the at least one of the one or more alarm conditions was triggered.
128. The system of clause 125, 126 or 127, wherein the alarm further comprises an alarm icon, wherein the alarm icon is adjacent to the alarm condition text.
129. The system of any of clauses 121 to 128, wherein the first alarm condition comprises a low glucose alarm condition.
130. The system any of clauses 121 to 128, wherein the first alarm condition comprises a high glucose alarm condition.
131. The system of any of clauses 121 to 128, wherein the first alarm condition comprises an elevated ketone alarm condition.
132. The system of any of clauses 121 to 128, wherein the first alarm condition comprises a signal loss alarm condition.
133. The system of any of clauses 121 to 132, wherein the second alarm condition comprises an urgent low glucose alarm condition.
134. The system of clause 133, wherein the urgent low glucose alarm condition comprises an urgent low glucose threshold of below 55 mg/dL.
135. The system of any of clauses 121 to 132, wherein the second alarm condition comprises a high ketone alarm condition.
136. The system of clause 135, wherein the high ketone alarm condition comprises a high ketone threshold of above 1.5 mmol/L.
137. The system of any of clauses 121 to 128, wherein the first alarm condition comprises a low glucose alarm condition, and wherein the low glucose alarm condition comprises a low glucose threshold of below 70 mg/dL.
138. The system of any of clauses 121 to 128, wherein the first alarm condition comprises a high glucose alarm condition, and wherein the high glucose alarm condition comprises a high glucose threshold of above 240 mg/dL.
139. The system of any of clauses 121 to 128, wherein the first alarm condition comprises a signal loss alarm condition, and wherein the signal loss alarm condition comprises a predetermined time elapsed since a current sensor reading was received.
140. The system of any of clauses 121 to 128, wherein the first alarm condition comprises an elevated ketone alarm condition, and wherein the elevated ketone alarm condition comprises an elevated ketone threshold of above 1.0 mmol/L.
141. The system of any of clauses 123 to 140, wherein the first analyte card comprises a text description of a first analyte condition, a first current analyte level value, and a first directional trend arrow based on the data indicative of the first analyte level.
142. The system of clause 141, wherein the first analyte card further comprises an alarm icon, wherein the alarm icon is adjacent to the text description of the first analyte condition.
143. The system of any of clauses 123 to 142, wherein the second analyte card comprises a text description of a second analyte condition, a second current analyte level value, and a second directional trend arrow based on the data indicative of the second analyte level of the user.
144. The system of clause 143, wherein the second analyte card further comprises an alarm icon, wherein the alarm icon is adjacent to the text description of the second analyte condition.
145. The system of clause 143 or 144, wherein the second current analyte level value and the second directional trend arrow are only displayed on the second analyte card when the data indicative of the second analyte level exceeds a predefined analyte threshold range.
146. The system of any of clauses 123 to 145, wherein the first analyte card comprises a background color indicative of a condition associated with the data indicative of the first analyte level of the user.
147. The system of any of clauses 123 to 146, wherein the second analyte card comprises a second background color indicative of a condition associated with the data indicative of the second analyte level of the user.
148. The system of any of clauses 123 to 147, wherein the first analyte card, the second analyte card, and the alarm are displayed on the alarm notification GUI at a same time.
149. The system of any of clauses 123 to 148, wherein the first analyte card comprises a glucose card, wherein the glucose card displays data indicative of a glucose level, and wherein the second analyte card comprises a ketone card, wherein the ketone card displays data indicative of a ketone level.
150. The system of clause 149, wherein the glucose card displays data indicative of the glucose level reflecting a low glucose condition, wherein the ketone card displays data indicative of the ketone level reflecting a normal ketone condition, an elevated ketone condition, or a high ketone condition, and wherein the first alarm condition comprises a low glucose alarm condition.
151. The system of clause 149, wherein the glucose card displays data indicative of the glucose level reflecting a low glucose condition, wherein the ketone card displays data indicative of the ketone level reflecting an elevated ketone condition, and wherein the first alarm condition comprises an elevated ketone alarm condition.
152. The system of clause 149, wherein the glucose card displays data indicative of the glucose level reflecting a low glucose condition, wherein the ketone card displays data indicative of the ketone level reflecting a high ketone condition, and wherein the second alarm condition comprises a high ketone alarm condition.
153. The system of clause 149, wherein the glucose card displays data indicative of the glucose level reflecting a low glucose condition, wherein the ketone card displays data indicative of the ketone level reflecting an elevated ketone condition, wherein the first alarm condition comprises a low glucose alarm condition, wherein the first alarm condition further comprises an elevated ketone alarm condition, and wherein the alarm notification GUI comprises two alarms, wherein a first alarm is associated with the low glucose alarm condition and a second alarm is associated with the elevated ketone alarm condition.
154. The system of clause 149, wherein the glucose card displays data indicative of the glucose level reflecting a low glucose condition, wherein the ketone card displays data indicative of the ketone level reflecting an elevated ketone condition, wherein the first alarm condition comprises a low glucose alarm condition, wherein the second alarm condition comprises a high ketone alarm condition, and wherein the alarm notification GUI comprises two alarms, wherein a first alarm is associated with the low glucose alarm condition and a second alarm is associated with the high ketone alarm condition.
155. The system of clause 149, wherein the glucose card displays data indicative of the glucose level reflecting a high glucose condition, wherein the ketone card displays data indicative of the ketone level reflecting a normal ketone condition, and wherein the first alarm condition comprises a high glucose alarm condition.
156. The system of clause 149, wherein the glucose card displays data indicative of the glucose level reflecting a high glucose condition, wherein the ketone card displays data indicative of the ketone level reflecting an elevated ketone condition, and wherein the first alarm condition comprises an elevated ketone alarm condition.
157. The system of clause 149, wherein the glucose card displays data indicative of the glucose level reflecting a high glucose condition, wherein the ketone card displays data indicative of the ketone level reflecting a high ketone condition, and wherein the second alarm condition comprises a high ketone alarm condition.
158. The system of clause 149, wherein the glucose card displays data indicative of the glucose level reflecting a high glucose condition, wherein the ketone card displays data indicative of the ketone level reflecting an elevated ketone condition, wherein the first alarm condition comprises a high glucose alarm condition, wherein the first alarm condition further comprises an elevated ketone alarm condition, and wherein the alarm notification GUI comprises two alarms, wherein a first alarm is associated with the high glucose alarm condition and a second alarm is associated with the elevated ketone alarm condition.
159. The system of clause 149, wherein the glucose card displays data indicative of the glucose level reflecting a high glucose condition, wherein the ketone card displays data indicative of the ketone level reflecting a high ketone condition, wherein the first alarm condition comprises a high glucose alarm condition, wherein the second alarm condition comprises a high ketone alarm condition, and wherein the alarm notification GUI comprises two alarms, wherein a first alarm is associated with the high glucose alarm condition and a second alarm is associated with the high ketone alarm condition.
160. The system of clause 149, wherein the glucose card displays data indicative of the glucose level reflecting a normal glucose condition, wherein the ketone card displays data indicative of the ketone level reflecting an elevated ketone condition, and wherein the first alarm condition comprises an elevated ketone alarm condition.
161. The system of clause 149, wherein the glucose card displays data indicative of the glucose level reflecting a normal glucose condition, wherein the ketone card displays data indicative of the ketone level reflecting a high ketone condition, and wherein the second alarm condition comprises a high ketone alarm condition.
162. The system of clause 149, wherein the glucose card displays data indicative of the glucose level reflecting an urgent low glucose condition, wherein the ketone card displays data indicative of the ketone level reflecting a normal ketone condition, and wherein the second alarm condition comprises an urgent low glucose alarm condition.
163. The system of clause 149, wherein the glucose card displays data indicative of the glucose level reflecting an urgent low glucose condition, wherein the ketone card displays data indicative of the ketone level reflecting a high ketone condition, and wherein the second alarm condition comprises a high ketone alarm condition.
164. The system of clause 149, wherein the glucose card displays data indicative of the glucose level reflecting an urgent low glucose condition, wherein the ketone card displays data indicative of the ketone level reflecting an elevated ketone condition, and wherein the first alarm condition comprises an elevated ketone alarm condition.
165. The system of clause 149, wherein the glucose card displays data indicative of the glucose level reflecting an urgent low glucose condition, wherein the ketone card displays data indicative of the ketone level reflecting a high ketone condition, wherein the second alarm condition comprises an urgent low glucose alarm condition, wherein the second alarm condition further comprises a high ketone alarm condition, and wherein the alarm notification GUI comprises two alarms, wherein a first alarm is associated with the urgent low glucose alarm condition and a second alarm is associated with the high ketone alarm condition.
166. The system of clause 149, wherein the glucose card displays data indicative of the glucose level reflecting an urgent low glucose condition, wherein the ketone card displays data indicative of the ketone level reflecting an elevated ketone condition, wherein the first alarm condition comprises an elevated ketone alarm condition, wherein the second alarm condition comprises an urgent low glucose alarm condition, and wherein the alarm notification GUI comprises two alarms, wherein a first alarm is associated with the elevated ketone alarm condition and a second alarm is associated with the urgent low glucose alarm condition.
167. The system of any of clauses 121 to 166, wherein the reader device further comprises a touchscreen, and wherein the analyte monitoring application, when executed by the one or more processors, further causes the one or more processors to:
   receive input from the touchscreen corresponding to a drag gesture, a long-press gesture, or a swipe gesture, and
   expand the alarm notification GUI in response to the received input, wherein when expanded, the alarm notification GUI comprises the alarm associated with the at least one of the one or more alarm conditions, wherein the alarm notification GUI further comprises a first analyte card, a second analyte card, and a trend line, wherein the first analyte card reflects data indicative of the first analyte level, wherein the second analyte card reflects data indicative of the second analyte level, and wherein the trend line is associated with the at least one of the one or more alarm conditions.
168. The system of any of clauses 121 to 167, wherein the reader device further comprises a touchscreen, wherein the analyte monitoring application, when executed by the one or more processors, further causes the one or more processors to:
   receive input from the touchscreen corresponding to the alarm associated with at least one of the one or more alarm conditions, and
   output a sensor results GUI, wherein the sensor results GUI comprises a first analyte section and a second analyte section,
   wherein the first analyte section comprises the first analyte card and a first analyte graph portion reflecting the data indicative of the first analyte level, and
   wherein the second analyte section comprises the second analyte card and a second analyte graph portion reflecting the data indicative of the second analyte level.
169. The system of clause 168, wherein the first analyte section is configured to transition between a first collapsed view and a first expanded view, and wherein the second analyte section is configured to transition between a second collapsed view and a second expanded view.
170. The system of clause 169, wherein the data indicative of the first analyte level meets the one or more alarm conditions, wherein the analyte monitoring application, when executed by the one or more processors, further causes the one or more processors to:
   in response to a selection of the alarm associated with the at least one or more alarm conditions associated with the data indicative of the first analyte level, and
   display the first expanded view of the first analyte section on the sensor results GUI, wherein in the first expanded view, the first analyte card and the first analyte graph portion are displayed on the sensor results GUI.
171. The system of clause 169 or 170, wherein the analyte monitoring application, when executed by the one or more processors, further causes the one or more processors to:
   in response to a selection of the alarm associated with the at least one or more alarm conditions associated with the data indicative of the first analyte level, display the second collapsed view of the second analyte section on the sensor results GUI,
   wherein in the second collapsed view, only the second analyte card is displayed on the sensor results GUI.
172. The system of clause 169, 170 or 171, wherein the analyte monitoring application, when executed by the one or more processors, further causes the one or more processors to:
   in response to a selection of the alarm associated with the at least one or more alarm conditions associated with the data indicative of the first analyte level, display an animation on the sensor results GUI, wherein the animation is configured to demonstrate the first analyte section transitioning from the first collapsed view to the first expanded view.
173. The system of any of clauses 169 to 172, wherein the analyte monitoring application, when executed by the one or more processors, further causes the one or more processors to:
   in response to a selection of the alarm associated with the at least one or more alarm conditions associated with the data indicative of the first analyte level, display an animation on the sensor results GUI, wherein the animation is configured to demonstrate the second analyte section transitioning from the second expanded view to the second collapsed view.
174. The system of any of clauses 169 to 173, wherein the data indicative of the second analyte level meets the one or more alarm conditions, wherein the analyte monitoring application, when executed by the one or more processors, further causes the one or more processors to:
   in response to a selection of the alarm associated with the at least one or more alarm conditions associated with the data indicative of the second analyte level, and
   display the second expanded view of the second analyte section on the sensor results GUI, wherein in the second expanded view, the second analyte card and the second analyte graph portion are displayed on the sensor results GUI.
175. The system of any of clauses 169 to 174, wherein the analyte monitoring application, when executed by the one or more processors, further causes the one or more processors to:
   in response to a selection of the alarm associated with the at least one or more alarm conditions associated with the data indicative of the second analyte level, display the first collapsed view of the first analyte section on the sensor results GUI,
   wherein in the first collapsed view, only the first analyte card is displayed on the sensor results GUI.
176. The system of any of clauses 169 to 175, wherein the analyte monitoring application, when executed by the one or more processors, further causes the one or more processors to:
   in response to a selection of the alarm associated with the at least one or more alarm conditions associated with the data indicative of the second analyte level, display an animation on the sensor results GUI, wherein the animation is configured to demonstrate the second analyte section transitioning from the second collapsed view to the second expanded view.
177. The system of any of clauses 169 to 177, wherein the analyte monitoring application, when executed by the one or more processors, further causes the one or more processors to:
   in response to a selection of the alarm associated with the at least one or more alarm conditions associated with the data indicative of the second analyte level, display an animation on the sensor results GUI, wherein the animation is configured to demonstrate the first analyte section transitioning from the first expanded view to the first collapsed view.
178. The system of any of clauses 121 to 177, wherein the analyte monitoring application, when executed by the one or more processors, further causes the one or more processors to:
   output an alarm settings GUI, wherein the alarm settings GUI comprises a plurality of selectable glucose alarms options, a plurality of selectable ketones alarms options, and one or more selectable other options.
179. The system of clause 178, wherein the plurality of selectable glucose alarms options comprises an urgent low glucose alarm option, a low glucose alarm option, and a high glucose alarm option.
180. The system of clause 179, wherein the analyte monitoring application, when executed by the one or more processors, further causes the one or more processors to:
   in response to a selection of the urgent low glucose alarm option, output a first alarm GUI comprising the second set of alarm settings that are not configurable by the user.
181. The system of clause 180, wherein the second set of alarm settings that are not configurable by the user comprises a non-configurable on-off setting.
182. The system of clause 180, wherein the second set of alarm settings that are not configurable by the user comprises a non-configurable urgent low glucose threshold setting.
183. The system of clause 180, wherein the second set of alarm settings that are not configurable by the user comprises a non-configurable alarm tone setting.
184. The system of clause 180, wherein the second set of alarm settings that are not configurable by the user comprises a non-configurable setting to override a do not disturb feature or a mute feature.
185. The system of any of clauses 180 to 184, wherein the analyte monitoring application, when executed by the one or more processors, further cause the one or more processors to: display, on the first alarm GUI, a banner comprising a message indicating that the second set of alarm settings cannot be modified.
186. The system of any of clauses 180 to 185, wherein the analyte monitoring application, when executed by the one or more processors, further cause the one or more processors to: display, on the first alarm GUI, a message indicating that the second set of alarm settings will override a do not disturb feature or a muting feature.
187. The system of any of clauses 179 to 186, wherein the analyte monitoring application, when executed by the one or more processors, further causes the one or more processors to:
   in response to a selection of the low glucose alarm option, output a second alarm GUI comprising the first set of alarm settings that are configurable by the user.
188. The system of clause 187, wherein the first set of alarm settings that are configurable by the user comprises a configurable on-off switch.
189. The system of clause 187 or 188, wherein the first set of alarm settings that are configurable by the user comprises a configurable low glucose threshold setting.
190. The system of clause 187, 188 or 189, wherein the first set of alarm settings that are configurable by the user comprises a configurable alarm tone setting.
191. The system of any of clauses 187 to 190, wherein the first set of alarm settings that are configurable by the user comprises a configurable switch to override a do not disturb feature or a mute feature.
192. The system of any of clauses 187 to 191, wherein the analyte monitoring application, when executed by the one or more processors, further cause the one or more processors to:
   display, on the second alarm GUI, instructions relating to a configurable switch to override a do not disturb feature or a mute feature.
193. The system of any of clauses 179 to 192, wherein the analyte monitoring application, when executed by the one or more processors, further causes the one or more processors to:
   in response to a selection of the high glucose alarm option, output a third alarm GUI comprising the first set of alarm settings that are configurable by the user.
194. The system of clause 193, wherein the first set of alarm settings that are configurable by the user comprises a configurable on-off switch.
195. The system of clause 193 or 194, wherein the first set of alarm settings that are configurable by the user comprises a configurable high glucose threshold setting.
196. The system of clause 193, 194 or 195, wherein the first set of alarm settings that are configurable by the user comprises a configurable alarm tone setting.
197. The system of any of clauses 193 to 196, wherein the first set of alarm settings that are configurable by the user comprises a configurable switch to override a do not disturb feature.
198. The system of any of clauses 193 to 197, wherein the analyte monitoring application, when executed by the one or more processors, further cause the one or more processors to:
   display, on the third alarm GUI, instructions relating to a configurable switch to override a do not disturb feature or a mute feature.
199. The system of any of clauses 178 to 180, wherein the plurality of selectable ketone alarm options comprises an elevated ketone alarm option and a high ketone alarm option.
200. The system of clause 199, wherein the analyte monitoring application, when executed by the one or more processors, further causes the one or more processors to:
   in response to a selection of the elevated ketone alarm option, output a fourth alarm GUI comprising the first set of alarm settings that are configurable by the user.
201. The system of clause 200, wherein the first set of alarm settings that are configurable by the user comprises a configurable on-off switch.
202. The system of clause 200 or 201, wherein the analyte monitoring application, when executed by the one or more processors, further cause the one or more processors to:
   display, on the fourth alarm GUI, an elevated ketone threshold setting, wherein the elevated ketone threshold setting comprises a lock icon indicating that the elevated ketone threshold setting is locked.
203. The system of any of clauses 200 to 202, wherein the analyte monitoring application, when executed by the one or more processors, further cause the one or more processors to:
   display, on the fourth alarm GUI, an elevated ketone threshold setting, wherein the elevated ketone threshold setting is non-configurable.
204. The system of any of clauses 200 to 203, wherein the first set of alarm settings that are configurable by the user comprises a configurable alarm tone setting.
205. The system of any of clauses 200 to 204, wherein the first set of alarm settings that are configurable by the user comprises a configurable switch to override a do not disturb feature or a mute feature.
206. The system of any of clauses 200 to 205, wherein the analyte monitoring application, when executed by the one or more processors, further cause the one or more processors to:
   display, on the fourth alarm GUI, instructions relating to a configurable switch to override a do not disturb feature or a mute feature.
207. The system of any of clauses 199 to 206, wherein the analyte monitoring application, when executed by the one or more processors, further causes the one or more processors to:
   in response to a selection of the high ketone alarm option, output a fifth alarm GUI comprising the second set of alarm settings that are not configurable by the user.
208. The system of clause 207, wherein the second set of alarm settings that are not configurable by the user comprises a non-configurable on-off setting.
209. The system of clause 207 or 208, wherein the second set of alarm settings that are not configurable by the user comprises a non-configurable high ketone threshold setting.
210. The system of clause 207, 208 or 209, wherein the second set of alarm settings that are not configurable by the user comprises a non-configurable alarm tone setting.
211. The system of any of clauses 207 to 210, wherein the second set of alarm settings that are not configurable by the user comprises a non-configurable setting to override a do not disturb feature or a mute feature.
212. The system of any of clauses 207 to 211, wherein the analyte monitoring application, when executed by the one or more processors, further cause the one or more processors to:
   display, on the fifth alarm GUI, a banner comprising a message indicating that the second set of alarm settings cannot be modified.
213. The system of any of clauses 207 to 212, wherein the analyte monitoring application, when executed by the one or more processors, further cause the one or more processors to:
   display, on the fifth alarm GUI, a message indicating that the second set of alarm settings will override a do not disturb feature or a muting feature.
214. The system of any of clauses 178 to 213, wherein the one or more selectable other options comprises a signal loss alarm option.
215. The system of clause 214, wherein the analyte monitoring application, when executed by the one or more processors, further causes the one or more processors to:
   in response to a selection of the signal loss alarm option, output a sixth alarm GUI comprising the first set of alarm settings that are configurable by the user.
216. The system of clause 215, wherein the first set of alarm settings that are configurable by the user comprises a configurable on-off switch.
217. The system of clause 215 or 216, wherein the first set of alarm settings that are configurable by the user comprises a configurable alarm tone setting.
218. The system of clause 215, 216 or 217, wherein the first set of alarm settings that are configurable by the user comprises a configurable switch to override a do not disturb feature or a mute feature.
219. The system of any of clauses 215 to 218, wherein the analyte monitoring application, when executed by the one or more processors, further cause the one or more processors to:
   display, on the sixth alarm GUI, instructions relating to a configurable switch to override a do not disturb feature or a mute feature.
220. The system of any of clauses 215 to 219, wherein the analyte monitoring application, when executed by the one or more processors, further cause the one or more processors to:
   display, on the sixth alarm GUI, a banner comprising a message indicating that the user will receive a signal loss alarm when the analyte sensor is not communicating with the analyte monitoring application.
221. The system of any of clauses 121 to 220, wherein the alarm settings GUI further comprises a selectable learn more link, wherein the analyte monitoring application, when executed by the one or more processors, further cause the one or more processors to:
   in response to a selection of the learn more link, output a learning interface.
222. A system for monitoring one or more analytes in a user, comprising:
   a sensor control device comprising an analyte sensor, wherein at least a portion of the analyte sensor is configured to be in fluid contact with a bodily fluid of the user, and wherein the sensor control device is configured to transmit data indicative of one or more analyte levels of the user, wherein the data indicative of the one or more analyte levels comprises a data indicative of a first analyte level and a data indicative of a second analyte level, wherein the first analyte level is indicative of a first analyte, and wherein the second analyte level is indicative of a second analyte different from the first analyte; and
   a reader device, comprising:
   wireless communication circuitry configured to receive the data indicative of the analyte level from the sensor control device; and
   one or more processors coupled with a memory, the memory storing instructions that, when executed by the one or more processors, cause the one or more processors to: detect one or more alarm unavailability conditions while at least one alarm of the analyte monitoring system is enabled; and
      present a notification associated with the detected one or more alarm unavailability conditions.
223. The system of clause 222, wherein the at least one enabled alarm comprises one or more of a low glucose alarm, an urgent low glucose alarm, a high glucose alarm, an elevated ketone alarm, a high ketone alarm, and a signal loss alarm.
224. The system of clause 222 or 223, wherein the one or more alarm unavailability conditions comprise a wireless communication circuitry being disabled or malfunctioning.
225. The system of clause 224, wherein the wireless communication circuitry comprises a Bluetooth or Bluetooth Low Energy communication circuitry.
226. The system of any of clauses 222 to 225, wherein the one or more alarm unavailability conditions comprise one or more systemwide notifications being disabled.
227. The system of any of clauses 222 to 226, wherein the one or more alarm unavailability conditions comprise one or more application-specific notifications being disabled.
228. The system of any of clauses 222 to 227, wherein the one or more alarm unavailability conditions comprise a sensor fault condition, wherein the sensor fault condition comprises a high sensor temperature or a low sensor temperature.
229. The system of any of clauses 222 to 228, wherein the one or more alarm unavailability conditions comprise critical alerts being disabled.
230. The system of any of clauses 222 to 229, wherein the one or more alarm unavailability conditions comprise a signal loss condition.
231. The system of any of clauses 222 to 230, wherein the one or more alarm unavailability conditions comprise an override do-not-disturb feature being disabled.
232. The system of any of clauses 222 to 231, wherein the one or more alarm unavailability conditions comprise one or more alarm tones being set to silent.
233. The system of any of clauses 222 to 232, wherein the one or more alarm unavailability conditions comprise a detection of no active sensor.
234. The system of any of clauses 222 to 233, wherein the one or more alarm unavailability conditions comprise a sensor fault condition.
235. The system of clause 234, wherein the sensor fault condition comprises the sensor control device not communicating with an analyte monitoring application.
236. The system of any of clauses 222 to 235, wherein the notification associated with the detected one or more alarm unavailability conditions comprises a banner notification or a pop-up window displayed on a display of the reader device.
237. The system of any one of clauses 222 to 235, wherein the notification associated with the detected one or more alarm unavailability conditions comprises a modal displayed within an analyte monitoring application.
238. The system of clause 237, wherein the modal includes text indicating one or more reasons for the one or more alarm unavailability conditions.
239. The system of clause 237 or 238, wherein the modal includes a button configured to open an operating system settings interface.
240. The system of clause 237, 238 or 239, wherein the modal includes a button configured to dismiss the notification associated with the detected one or more alarm unavailability conditions.
241. The system of any of clauses 222 to 240, wherein the data indicative of the first analyte level of the user comprises data indicative of a glucose level, and wherein the data indicative of the second analyte level of the user comprises data indicative of a ketone level,
   wherein the first tab is further configured to output a first analyte graph summary section and a logbook section, wherein the logbook section comprises information on one or more activity events related to the data indicative of the first analyte level, and
   wherein the second tab is further configured to output a second analyte graph section and an alarms section, wherein the alarms section comprises a list of one or more alarm events related to the data indicative of the second analyte level.

The claims of the parent application are reproduced below. These clauses define preferable combinations of features. The applicant reserves the right to pursue protection for these combinations of features, and/or any other subject-matter contained in the parent application as filed, either in the present divisional application or in a further application divided from the present divisional application. The claims of the parent application are not the claims of the current application which are contained in a separate section headed "claims".
1. A system for monitoring a plurality of analytes in a user, the system comprising:
   a sensor control device comprising an analyte sensor, wherein at least a portion of the analyte sensor is configured to be in fluid contact with a bodily fluid of the user, and wherein the sensor control device is configured to transmit data indicative of a plurality of analyte levels of the user, wherein the data indicative of the plurality of analyte levels comprises a data indicative of a first analyte level and a data indicative of a second analyte level, wherein the first analyte level is indicative of a first analyte, and wherein the second analyte level is indicative of a second analyte different from the first analyte;
   a reader device comprising:
      wireless communication circuitry configured to receive the data indicative of the plurality of analyte levels of the user, and
      one or more processors coupled with a memory, the memory storing an analyte monitoring application that, when executed by the one or more processors, causes the one or more processors to:
         determine whether the data indicative of the first analyte level or the data indicative of the second analyte level meets one or more alarm conditions, wherein the one or more alarm conditions comprise a first alarm condition associated with a first set of alarm settings that are configurable by the user and a second alarm condition associated with a second set of alarm settings that are not configurable by the user,
         in response to a determination that at least one of the one or more alarm conditions is met, display an alarm notification user interface (GUI) comprising an alarm associated with the at least one of the one or more alarm conditions.
2. The system of claim 1, wherein the data indicative of the first analyte level of the user comprises data indicative of a glucose level, and wherein the data indicative of the second analyte level of the user comprises data indicative of a ketone level.
3. The system of claim 1, wherein the alarm comprises an alarm condition text, an analyte alarm message, an analyte level measurement, and a trend indicator associated with the at least one of the one or more alarm conditions.
4. The system of claim 1, wherein the first alarm condition comprises a low glucose alarm condition.
5. The system of claim 1, wherein the first alarm condition comprises a high glucose alarm condition.
6. The system of claim 1, wherein the first alarm condition comprises an elevated ketone alarm condition.
7. The system of claim 1, wherein the first alarm condition comprises a signal loss alarm condition.
8. The system of claim 1, wherein the second alarm condition comprises an urgent low glucose alarm condition.
9. The system of claim 1, wherein the second alarm condition comprises a high ketone alarm condition.
10. The system of claim 1, wherein the reader device further comprises a touchscreen, and wherein the analyte monitoring application, when executed by the one or more processors, further causes the one or more processors to:
   receive input from the touchscreen corresponding to a drag gesture, a long-press gesture, or a swipe gesture, and
   expand the alarm notification GUI in response to the received input, wherein when expanded, the alarm notification GUI comprises the alarm associated with the at least one of the one or more alarm conditions, wherein the alarm notification GUI further comprises a first analyte card and a second analyte card, wherein the first analyte card reflects data indicative of the first analyte level, and wherein the second analyte card reflects data indicative of the second analyte level.
11. The system of claim 10, wherein the first analyte card comprises a text description of a first analyte condition, a first current analyte level value, and a first directional trend arrow based on the data indicative of the first analyte level.
12. The system of claim 10, wherein the second analyte card comprises a text description of a second analyte condition, a second current analyte level value, and a second directional trend arrow based on the data indicative of the second analyte level of the user.
13. The system of claim 10, wherein the first analyte card comprises a glucose card, wherein the glucose card displays data indicative of a glucose level, and wherein the second analyte card comprises a ketone card, wherein the ketone card displays data indicative of a ketone level.
14. The system of claim 13, wherein the glucose card displays data indicative of the glucose level reflecting a low glucose condition, wherein the ketone card displays data indicative of the ketone level reflecting a normal ketone condition, an elevated ketone condition, or a high ketone condition, and wherein the first alarm condition comprises a low glucose alarm condition.
15. The system of claim 13, wherein the glucose card displays data indicative of the glucose level reflecting a low glucose condition, wherein the ketone card displays data indicative of the ketone level reflecting an elevated ketone condition, and wherein the first alarm condition comprises an elevated ketone alarm condition.
16. The system of claim 13, wherein the glucose card displays data indicative of the glucose level reflecting a low glucose condition, wherein the ketone card displays data indicative of the ketone level reflecting a high ketone condition, and wherein the second alarm condition comprises a high ketone alarm condition.
17. The system of claim 13, wherein the glucose card displays data indicative of the glucose level reflecting a low glucose condition, wherein the ketone card displays data indicative of the ketone level reflecting an elevated ketone condition, wherein the first alarm condition comprises a low glucose alarm condition, wherein the first alarm condition further comprises an elevated ketone alarm condition, and wherein the alarm notification GUI comprises two alarms, wherein a first alarm is associated with the low glucose alarm condition and a second alarm is associated with the elevated ketone alarm condition.
18. The system of claim 13, wherein the glucose card displays data indicative of the glucose level reflecting a low glucose condition, wherein the ketone card displays data indicative of the ketone level reflecting an elevated ketone condition, wherein the first alarm condition comprises a low glucose alarm condition, wherein the second alarm condition comprises a high ketone alarm condition, and wherein the alarm notification GUI comprises two alarms, wherein a first alarm is associated with the low glucose alarm condition and a second alarm is associated with the high ketone alarm condition.
19. The system of claim 13, wherein the glucose card displays data indicative of the glucose level reflecting a high glucose condition, wherein the ketone card displays data indicative of the ketone level reflecting a normal ketone condition, and wherein the first alarm condition comprises a high glucose alarm condition.
20. The system of claim 13, wherein the glucose card displays data indicative of the glucose level reflecting a high glucose condition, wherein the ketone card displays data indicative of the ketone level reflecting an elevated ketone condition, and wherein the first alarm condition comprises an elevated ketone alarm condition.
21. The system of claim 13, wherein the glucose card displays data indicative of the glucose level reflecting a high glucose condition, wherein the ketone card displays data indicative of the ketone level reflecting a high ketone condition, and wherein the second alarm condition comprises a high ketone alarm condition.
22. The system of claim 13, wherein the glucose card displays data indicative of the glucose level reflecting a high glucose condition, wherein the ketone card displays data indicative of the ketone level reflecting an elevated ketone condition, wherein the first alarm condition comprises a high glucose alarm condition, wherein the first alarm condition further comprises an elevated ketone alarm condition, and wherein the alarm notification GUI comprises two alarms, wherein a first alarm is associated with the high glucose alarm condition and a second alarm is associated with the elevated ketone alarm condition.
23. The system of claim 13, wherein the glucose card displays data indicative of the glucose level reflecting a high glucose condition, wherein the ketone card displays data indicative of the ketone level reflecting a high ketone condition, wherein the first alarm condition comprises a high glucose alarm condition, wherein the second alarm condition comprises a high ketone alarm condition, and wherein the alarm notification GUI comprises two alarms, wherein a first alarm is associated with the high glucose alarm condition and a second alarm is associated with the high ketone alarm condition.
24. The system of claim 13, wherein the glucose card displays data indicative of the glucose level reflecting a normal glucose condition, wherein the ketone card displays data indicative of the ketone level reflecting an elevated ketone condition, and wherein the first alarm condition comprises an elevated ketone alarm condition.
25. The system of claim 13, wherein the glucose card displays data indicative of the glucose level reflecting a normal glucose condition, wherein the ketone card displays data indicative of the ketone level reflecting a high ketone condition, and wherein the second alarm condition comprises a high ketone alarm condition.
26. The system of claim 13, wherein the glucose card displays data indicative of the glucose level reflecting an urgent low glucose condition, wherein the ketone card displays data indicative of the ketone level reflecting a normal ketone condition, and wherein the second alarm condition comprises an urgent low glucose alarm condition.
27. The system of claim 13, wherein the glucose card displays data indicative of the glucose level reflecting an urgent low glucose condition, wherein the ketone card displays data indicative of the ketone level reflecting a high ketone condition, and wherein the second alarm condition comprises a high ketone alarm condition.
28. The system of claim 13, wherein the glucose card displays data indicative of the glucose level reflecting an urgent low glucose condition, wherein the ketone card displays data indicative of the ketone level reflecting an elevated ketone condition, and wherein the first alarm condition comprises an elevated ketone alarm condition.
29. The system of claim 13, wherein the glucose card displays data indicative of the glucose level reflecting an urgent low glucose condition, wherein the ketone card displays data indicative of the ketone level reflecting a high ketone condition, wherein the second alarm condition comprises an urgent low glucose alarm condition, wherein the second alarm condition further comprises a high ketone alarm condition, and wherein the alarm notification GUI comprises two alarms, wherein a first alarm is associated with the urgent low glucose alarm condition and a second alarm is associated with the high ketone alarm condition.
30. The system of claim 13, wherein the glucose card displays data indicative of the glucose level reflecting an urgent low glucose condition, wherein the ketone card displays data indicative of the ketone level reflecting an elevated ketone condition, wherein the first alarm condition comprises an elevated ketone alarm condition, wherein the second alarm condition comprises an urgent low glucose alarm condition, and wherein the alarm notification GUI comprises two alarms, wherein a first alarm is associated with the elevated ketone alarm condition and a second alarm is associated with the urgent low glucose alarm condition.
31. The system of claim 1, wherein the reader device further comprises a touchscreen, and wherein the analyte monitoring application, when executed by the one or more processors, further causes the one or more processors to:
   receive input from the touchscreen corresponding to a drag gesture, a long-press gesture, or a swipe gesture, and
   expand the alarm notification GUI in response to the received input, wherein when expanded, the alarm notification GUI comprises the alarm associated with the at least one of the one or more alarm conditions, wherein the alarm notification GUI further comprises a first analyte card, a second analyte card, and a trend line, wherein the first analyte card reflects data indicative of the first analyte level, wherein the second analyte card reflects data indicative of the second analyte level, and wherein the trend line is associated with the at least one of the one or more alarm conditions.
32. The system of claim 1, wherein the reader device further comprises a touchscreen, wherein the analyte monitoring application, when executed by the one or more processors, further causes the one or more processors to:
   receive input from the touchscreen corresponding to the alarm associated with at least one of the one or more alarm conditions, and
   output a sensor results GUI, wherein the sensor results GUI comprises a first analyte section and a second analyte section,
   wherein the first analyte section comprises the first analyte card and a first analyte graph portion reflecting the data indicative of the first analyte level, and
   wherein the second analyte section comprises the second analyte card and a second analyte graph portion reflecting the data indicative of the second analyte level.
33. The system of claim 32, wherein the first analyte section is configured to transition between a first collapsed view and a first expanded view, and wherein the second analyte section is configured to transition between a second collapsed view and a second expanded view.
34. The system of claim 33, wherein the data indicative of the first analyte level meets the one or more alarm conditions, wherein the analyte monitoring application, when executed by the one or more processors, further causes the one or more processors to:
   in response to a selection of the alarm associated with the at least one or more alarm conditions associated with the data indicative of the first analyte level, and
   display the first expanded view of the first analyte section on the sensor results GUI, wherein in the first expanded view, the first analyte card and the first analyte graph portion are displayed on the sensor results GUI.
35. The system of claim 33, wherein the data indicative of the second analyte level meets the one or more alarm conditions, wherein the analyte monitoring application, when executed by the one or more processors, further causes the one or more processors to:
   in response to a selection of the alarm associated with the at least one or more alarm conditions associated with the data indicative of the second analyte level, and
   display the second expanded view of the second analyte section on the sensor results GUI, wherein in the second expanded view, the second analyte card and the second analyte graph portion are displayed on the sensor results GUI.
36. The system of claim 1, wherein the analyte monitoring application, when executed by the one or more processors, further causes the one or more processors to:
   output an alarm settings GUI, wherein the alarm settings GUI comprises a plurality of selectable glucose alarms options, a plurality of selectable ketones alarms options, and one or more selectable other options.
37. The system of claim 36, wherein the plurality of selectable ketone alarm options comprises an elevated ketone alarm option and a high ketone alarm option.
38. The system of claim 37, wherein the analyte monitoring application, when executed by the one or more processors, further causes the one or more processors to:
   in response to a selection of the elevated ketone alarm option, output a first alarm GUI comprising the first set of alarm settings that are configurable by the user.
39. The system of claim 38, wherein the analyte monitoring application, when executed by the one or more processors, further cause the one or more processors to:
   display, on the first alarm GUI, an elevated ketone threshold setting, wherein the elevated ketone threshold setting is non-configurable.
40. The system of claim 38, wherein the first set of alarm settings that are configurable by the user comprises a configurable alarm tone setting.
41. The system of claim 38, wherein the analyte monitoring application, when executed by the one or more processors, further cause the one or more processors to:
   display, on the first alarm GUI, instructions relating to a configurable switch to override a do not disturb feature or a mute feature.
42. The system of claim 37, wherein the analyte monitoring application, when executed by the one or more processors, further causes the one or more processors to:
   in response to a selection of the high ketone alarm option, output a second alarm GUI comprising the second set of alarm settings that are not configurable by the user.
43. The system of claim 42, wherein the second set of alarm settings that are not configurable by the user comprises a non-configurable on-off setting.
44. The system of claim 42, wherein the second set of alarm settings that are not configurable by the user comprises a non-configurable high ketone threshold setting.
45. The system of claim 42, wherein the second set of alarm settings that are not configurable by the user comprises a non-configurable alarm tone setting.
46. A system for monitoring one or more analytes in a user, comprising:
   a sensor control device comprising an analyte sensor, wherein at least a portion of the analyte sensor is configured to be in fluid contact with a bodily fluid of the user, and wherein the sensor control device is configured to transmit data indicative of one or more analyte levels of the user, wherein the data indicative of the one or more analyte levels comprises a data indicative of a first analyte level and a data indicative of a second analyte level, wherein the first analyte level is indicative of a first analyte, and wherein the second analyte level is indicative of a second analyte different from the first analyte; and
   a reader device, comprising:
      wireless communication circuitry configured to receive the data indicative of the analyte level from the sensor control device; and
      one or more processors coupled with a memory, the memory storing instructions that, when executed by the one or more processors, cause the one or more processors to:
         detect one or more alarm unavailability conditions while at least one alarm of the analyte monitoring system is enabled; and
         present a notification associated with the detected one or more alarm unavailability conditions.
47. The system of claim 46, wherein the at least one enabled alarm comprises one or more of a low glucose alarm, an urgent low glucose alarm, a high glucose alarm, an elevated ketone alarm, a high ketone alarm, and a signal loss alarm.
48. The analyte monitoring system of 46, wherein the data indicative of the first analyte level of the user comprises data indicative of a glucose level, and wherein the data indicative of the second analyte level of the user comprises data indicative of a ketone level.
49. An analyte monitoring system, comprising:
   a sensor control device comprising an analyte sensor, wherein the sensor control device is configured to be worn on a user's body and transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte; and
   a reader device comprising:
      a display;
      wireless communication circuitry configured to receive the data indicative of the one or more analyte levels of the user;
      one or more processors coupled with a memory, the memory storing an analyte monitoring application that, when executed by the one or more processors, causes the one or more processors to output to the display a graphical user interface (GUI) comprising:
         a graph portion comprising a first graph comprising a first trend line representing data indicative of the first analyte level over a predetermined time period and a second graph comprising a second trend line representing data indicative of the second analyte level over the predetermined time period;
         a first plurality of summary metrics associated with the data indicative of the first analyte level for the predetermined time period, wherein the first plurality of summary metrics comprises a plurality of first minimum analyte levels and first maximum analyte levels associated with a plurality of time increments within the predetermined time period, and
         a second plurality of summary metrics associated with the data indicative of the second analyte level for the predetermined time period, wherein the second plurality of summary metrics comprises one or more alarm metrics indicative of an alarm condition associated with the data indicative of the second analyte level, and
         wherein the graph portion further comprises an x-axis comprising units of time, and wherein the one or more alarm metrics, the first plurality of first minimum analyte levels and first maximum analyte levels are aligned with the x-axis of the graph portion.
50. An analyte monitoring system, comprising:
   a sensor control device comprising an analyte sensor, wherein the sensor control device is configured to be worn on a user's body and transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte; and
   a reader device comprising:
      a display;
      wireless communication circuitry configured to receive the data indicative of the plurality of analyte levels of the user;
      one or more processors coupled with a memory, the memory storing an analyte monitoring application that, when executed by the one or more processors, causes the one or more processors to output to the display a graphical user interface (GUI) comprising:
         a first view and a second view, wherein the first view comprises a first tab configured to output data indicative of the first analyte level for a particular period of time, and wherein the first view further comprises a second tab configured to output data indicative of the second analyte level for the particular period of time,
         wherein the first tab is further configured to output a first analyte graph summary section and a logbook section, wherein the logbook section comprises information on one or more activity events related to the data indicative of the first analyte level, and
         wherein the second tab is further configured to output a second analyte graph section and an alarms section, wherein the alarms section comprises a list of one or more alarm events related to the data indicative of the second analyte level.
51. A system for monitoring a plurality of analytes in a user, the system comprising:
   a sensor control device comprising a sensor, wherein at least a portion of the sensor is configured to be in fluid contact with a bodily fluid of the user, and wherein the sensor control device is configured to transmit data indicative of a plurality of analyte levels of the user, wherein the data indicative of the plurality of analyte levels includes data indicative of a first analyte level and data indicative of a second analyte level of the user, wherein the first analyte level is indicative of a first analyte, and wherein the second analyte level is indicative of a second analyte different from the first analyte;
   a reader device comprising:
      wireless communication circuitry configured to receive the data indicative of the plurality of analyte levels of the user,
      one or more processors coupled with a memory, the memory storing an analyte monitoring application that, when executed by the one or more processors, causes the one or more processors to:
         output, based on the data indicative of the first analyte level and the data indicative of the second analyte level, a sensor results graphical user interface (GUI) comprising a first analyte section and a second analyte section,
         wherein the first analyte section comprises a first analyte card and a first analyte graph portion reflecting the data indicative of the first analyte level, and
         wherein the second analyte section comprises a second analyte card and a second analyte graph portion reflecting the data indicative of the second analyte level.
52. The system of claim 51, wherein the data indicative of the first analyte level comprises data indicative of a glucose level.
53. The system of claim 52, wherein the data indicative of the second analyte level comprises data indicative of a ketone level.
54. The system of claim 51, wherein the sensor is a glucose-ketone sensor.
55. The system of claim 51, wherein the first analyte card comprises a text description, a first current analyte level value, and a first trend indicator associated with the data indicative of the first analyte level of the user.
56. The system of claim 55, wherein the first analyte card further comprises a background color indicative of a condition associated with the data indicative of the first analyte level of the user.
57. The system of claim 51, wherein the second analyte card comprises a text description, a second current analyte level value, and a second trend indicator associated with the data indicative of the second analyte level of the user.
58. The system of claim 57, wherein the second analyte card further comprises a background color indicative of a condition associated with the data indicative of the second analyte level of the user.
59. The system of claim 51, wherein the first analyte section is configured to transition between a first collapsed view and a first expanded view, wherein the sensor results GUI is further configured to display the first analyte section in the first expanded view by default.
60. The system of claim 59, wherein in the first expanded view, the first analyte section displays the first analyte card and the first analyte graph portion.
61. The system of claim 59, wherein in the first collapsed view, the first analyte section displays only the first analyte card.
62. The system of claim 59, wherein the first analyte graph portion is only displayed on the sensor results GUI when the first analyte section is in the first expanded view.
63. The system of claim 51, wherein the second analyte section is configured to transition between a second collapsed view and a second expanded view in response to the data indicative of the second analyte level reaching a predetermined threshold.
64. The system of claim 63, wherein in the second expanded view, the second analyte section displays the second analyte card and the second analyte graph portion.
65. The system of claim 63, wherein in the second collapsed view, the second analyte section displays only the second analyte card.
66. The system of claim 63, wherein the second analyte graph portion is only displayed on the sensor results GUI when the second analyte section is in the second expanded view.
67. The system of claim 51, wherein the first analyte section is configured to transition between a first collapsed view and a first expanded view, and wherein the second analyte section is configured to transition between a second collapsed view and a second expanded view.
68. The system of claim 67, wherein the first analyte section is in the first expanded view and the second analyte section is in the second expanded view, and wherein the first analyte card, the first analyte graph portion, the second analyte card, and the second analyte graph portion are displayed on the sensor results GUI at a same time.
70. The system of claim 67, wherein the first analyte section is in the first expanded view and the second analyte section is in the second collapsed view, and wherein the first analyte card, the first analyte graph portion, and the second analyte card are displayed on the sensor results GUI at a same time.
71. The system of claim 67, wherein the first analyte section is in the first collapsed view and the second analyte section is in the second expanded view, and wherein the first analyte card, the second analyte card, and the second analyte graph portion are displayed on the sensor results GUI at a same time.
72. The system of claim 67, wherein the first analyte section is in the collapsed view and the second analyte section is in the collapsed view, and wherein the first analyte card and the second analyte card are displayed on the sensor results GUI at a same time.
73. The system of claim 67, wherein the first analyte section is a glucose section, wherein the first analyte card is a glucose card, wherein the first analyte graph portion is a glucose graph portion, and wherein the data indicative of a first analyte level is a data indicative of a glucose level.
74. The system of claim 73, wherein the second analyte section is a ketone section, wherein the second analyte card is a ketone card, wherein the second analyte graph portion is a ketone graph portion, and wherein the data indicative of a second analyte level is a data indicative of a ketone level.
75. The system of claim 74, wherein the data indicative of the glucose level indicates a target glucose threshold range, a high glucose threshold range, or a low glucose threshold range, and wherein the sensor results GUI is configured to display the glucose section in the first expanded view.
76. The system of claim 75, wherein in the first expanded view, the glucose section displays the glucose card and the glucose graph portion, wherein the glucose card comprises a text description, a current glucose level value, and a glucose trend indicator associated with the data indicative of the glucose level, and wherein the glucose graph portion comprises a glucose trend line associated with the data indicative of the glucose level.
77. The system of claim 76, wherein the data indicative of the ketone level indicates a normal ketone threshold range, and wherein the sensor results GUI is configured to display the ketone section in the second collapsed view.
78. The system of claim 51, wherein the data indicative of the first analyte level of the user includes a first historical analyte data and a first current analyte data, and wherein the first analyte graph portion comprises the first historical analyte data and the first current analyte data.
79. The system of claim 51, wherein the first analyte graph portion comprises a first analyte trend line based on the data indicative of the first analyte level of the user.
80. The system of claim 51, wherein the second analyte graph portion comprises a second analyte trend line based on the data indicative of the second analyte level of the user.
81. The system of claim 51, wherein the first analyte card and the first analyte graph portion are displayed on the sensor results GUI, wherein the reader device further comprises a touchscreen, wherein the analyte monitoring application, when executed by the one or more processors, further causes the one or more processors to:
   receive input from the touchscreen corresponding to a selected point on the first analyte graph portion, and
   update the first analyte section based on the received input from the touchscreen.
82. The system of claim 81, wherein the data indicative of the first analyte level of the user includes a first historical analyte data and a first current analyte data, wherein the first analyte graph portion comprises the first historical analyte data and the first current analyte data, and wherein, based on the received input, the first analyte graph portion is configured to display the first historical analyte data associated with the selected point.
83. The system of claim 82, wherein the first analyte graph portion comprises a first analyte trend line based on the data indicative of the first analyte level of the user, wherein the first analyte trend line comprises a first colored circle indicating the first current analyte data, wherein the analyte monitoring application, when executed by the one or more processors, further causes the one or more processors to:
   update the first analyte section based on the received input, wherein, once updated, the first analyte section further comprises a second colored circle corresponding to the selected point, and wherein a color of the second colored circle indicates a condition corresponding to the first historical analyte data associated with the selected point.
84. The system of claim 51, wherein the second analyte card and the second analyte graph portion are displayed on the sensor results GUI wherein the reader device further comprises a touchscreen, wherein the analyte monitoring application, when executed by the one or more processors, further causes the one or more processors to:
   receive input from the touchscreen corresponding to a selected point on the second analyte graph portion, and
   update the second analyte section based on the received input from the touchscreen.
85. The system of claim 84, wherein the data indicative of the second analyte level of the user includes a second historical analyte data and a second current analyte data, wherein the second analyte graph portion comprises the second historical analyte data and the second current analyte data, and wherein, based on the received input, the second analyte graph portion is configured to display the second historical analyte data associated with the selected point
86. The system of claim 85, wherein the second analyte graph portion comprises a second analyte trend line based on the data indicative of the second analyte level of the user, wherein the second analyte trend line comprises a first colored circle indicating the second current analyte data, wherein the analyte monitoring application, when executed by the one or more processors, further causes the one or more processors to:
   update the second analyte section based on the received input, wherein, once updated, the second analyte section further comprises a second colored circle corresponding to the selected point, wherein a color of the second colored circle indicates a condition corresponding to the second historical analyte data associated with the selected point.
87. The system of claim 51, wherein the second analyte graph portion comprises one or more lines indicative of an elevated ketone threshold or a high ketone threshold.
88. The system of claim 51, wherein the second analyte graph portion comprises one or more icons, wherein the one or more icons are displayed on one or more points on the second analyte graph portion, and wherein the one or more icons are associated with the data indicative of the second analyte level corresponding to the one or more points.
89. The system of claim 88, wherein the one or more icons are selectable, wherein the reader device further comprises a touchscreen, and wherein the analyte monitoring application, when executed by the one or more processors, further causes the one or more processors to:
   receive input from the touchscreen corresponding to a selected one or more icons, and
   display a note card section corresponding to the selected one or more icons, wherein the note card section includes a text description associated with the selected one or more icons.
90. The system of claim 51, wherein the second analyte card and the second analyte graph portion are displayed on the sensor results GUI, wherein the analyte monitoring application, when executed by the one or more processors, further causes the one or more processors to:
   display, on the second analyte graph portion, a banner notification comprising instructions, wherein the instructions relate to a high ketone level condition or an elevated ketone level condition, wherein the banner notification further comprises a background color indicative of the high ketone level condition or the elevated ketone level condition.

## Claims

1. A system for monitoring a plurality of analytes in a user, the system comprising:
a sensor control device comprising a sensor, wherein at least a portion of the sensor is configured to be in fluid contact with a bodily fluid of the user, and wherein the sensor control device is configured to transmit data indicative of a plurality of analyte levels of the user, wherein the data indicative of the plurality of analyte levels includes data indicative of a glucose level and data indicative of a ketone level of the user;
a reader device comprising:
wireless communication circuitry configured to receive the data indicative of the plurality of analyte levels of the user,
one or more processors coupled with a memory, the memory storing an analyte monitoring application that, when executed by the one or more processors, causes the one or more processors to:
output, based on the data indicative of the glucose level and the data indicative of the ketone level, a sensor results graphical user interface (GUI) comprising a glucose section and a ketone section,
wherein the ketone section comprises a ketone graph portion comprising a ketone trend line reflecting the data indicative of the ketone level,
the ketone trend line is displayed as a flat line for a period of time corresponding to when the user's ketone level is within a predetermined normal ketone range.

2. The system of claim 1, wherein the flat line is displayed only if an adverse state of condition is detected in association with historical ketone data for a predefined period of time.

3. The system of claim 1 or 2, wherein the flat line is displayed only if the user's ketone level has exceeded a predetermined normal ketone range within a predefined period of time.

4. The system of any preceding claim, wherein, if the user's ketone level exceeds the predetermined normal ketone range within a predefined period of time, but is within the normal ketone range for an entire range of time over which the user's ketone data is shown on the ketone graph portion, the ketone trend line appears as a flat line for the entire range of time shown by the ketone graph portion.

5. The system of any preceding claim, wherein, if the user's ketone level exceeds the predetermined normal ketone range within a predefined period of time and within a range of time over which the user's ketone data is shown on the ketone graph portion, the ketone trend line appears as a flat line for only the part of the range of time shown by the ketone graph portion corresponding to when the user's ketone level is within the predetermined normal ketone range.

6. The system of claim 4 or 5, wherein the range of time shown by the ketone graph portion is three, six or twelve hours.

7. The system of any of claims 4 to 6, wherein the range of time shown by the ketone graph portion is selectable by the user using a selectable icon.

8. The system of any preceding claim, wherein, if an adverse state of condition is not detected in association with the historical ketone data and/or current ketone data for a predefined period of time, then the ketone section is displayed with a ketone card and without the ketone graph portion.

9. The system of any of claims 2 to 8, wherein the predefined period of time is the last 24 hours.

10. The system of any preceding claim, wherein the ketone trend line comprises a colored portion configured to indicate a condition associated with the current ketone data.

11. The system of claim 10, wherein the colored portion is displayed for a period of time corresponding to when the user's analyte level is within the predetermined normal ketone range.

12. The system of any preceding claim, wherein the ketone section further comprises a ketone card reflecting data indicative of the user's ketone level.

13. The system of any preceding claim, wherein the glucose section comprises a glucose card and a glucose graph portion reflecting the data indicative of the glucose level.

14. An analyte monitoring application that, when executed by one or more processors, causes the one or more processors to:
output, based on data indicative of a glucose level and data indicative of a ketone level of a user, a sensor results graphical user interface (GUI) comprising a glucose section and a ketone section, wherein the data indicative of the glucose level and data indicative of the ketone level of the user is obtained from a sensor, wherein at least a portion of the sensor is configured to be in fluid contact with a bodily fluid of the user;
wherein the ketone section comprises a ketone graph portion comprising a ketone trend line reflecting the data indicative of the ketone level, and
the ketone trend line is displayed as a flat line for a period of time corresponding to when the user's ketone level is within a predetermined normal ketone range.
